# EUROPÄISCHE PATENTSCHRIFT

(11) **EP 1 322 608 B1**
(45) Veröffentlichungstag und Bekanntmachung des Hinweises auf die Patenterteilung: **02.05.2007**
(21) Anmeldenummer: 01972035.8
(22) Anmeldetag: 10.09.2001
(51) Int. Cl.: C07D 207/22, C07D 207/26, C07D 409/14, C07D 401/14, C07D 405/14, C07D 417/14, A01N 43/36

(54) **DELTA1-PYRROLINE ALS PESTIZIDE**
DELTA1-PYRROLINES USED AS PESTICIDES
DELTA1-PYRROLINES UTILISES EN TANT QUE PESTICIDES

(30) Priorität: 22.09.2000 DE 10047116
(43) Veröffentlichungstag der Anmeldung: 02.07.2003
(73) Patentinhaber: Bayer CropScience AG, 40789 Monheim (DE)
(72) Erfinder: JANSEN, Johannes-Rudolf, 40789 Monheim (DE); PLANT, Andrew, Winnersh, Berkshire RG 41/5PD (GB); ALIG, Bernd, 53639 Königswinter (DE); KRAATZ, Udo, 51375 Leverkusen (DE); THIELKING, Gerhard, 51399 Burscheid (DE); ERDELEN, Christoph, 42799 Leichlingen (DE); TURBERG, Andreas, 42781 Haan (DE); HANSEN, Olaf, 42799 Lechlingen (DE)
(86) Internationale Anmeldenummer: PCT/EP2001/010422
(87) Internationale Veröffentlichungsnummer: WO 2002/024646

(56) Entgegenhaltungen:
- EP-A- 0 183 217
- WO-A-98/22438
- WO-A-99/59967
- WO-A-99/59968

## Beschreibung

Die vorliegende Erfindung betrifft neue Δ¹-Pyrroline, mehrere Verfahren zu ihrer Herstellung und ihre Verwendung als Schädlingsbekämpfungsmittel.

Bisher sind nur wenige 4-substituierte-2,5-bis-Aryl-Δ¹-Pyrroline bekannt geworden:

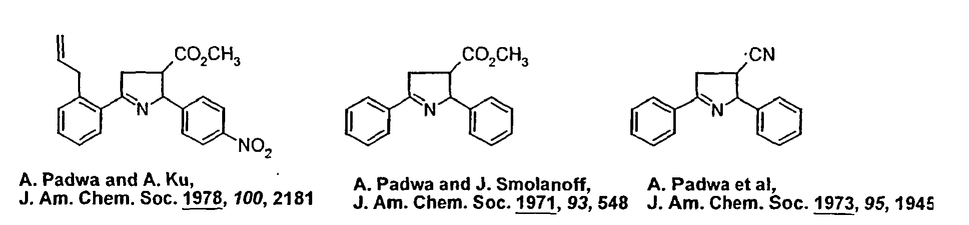

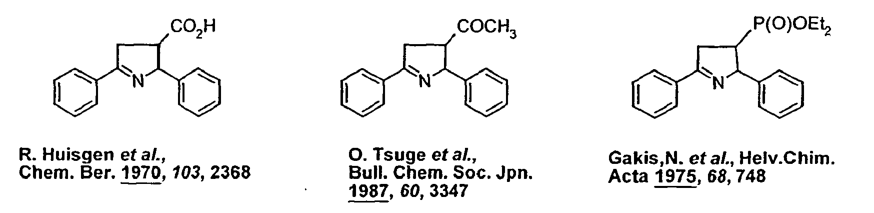

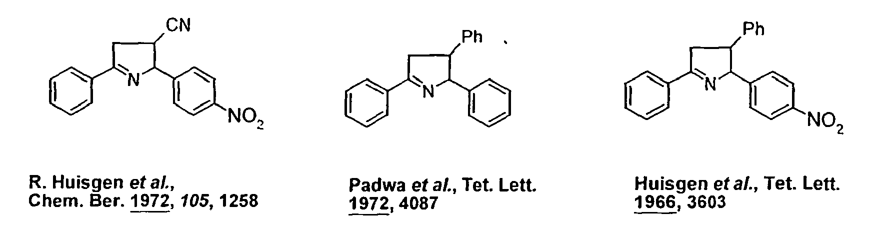

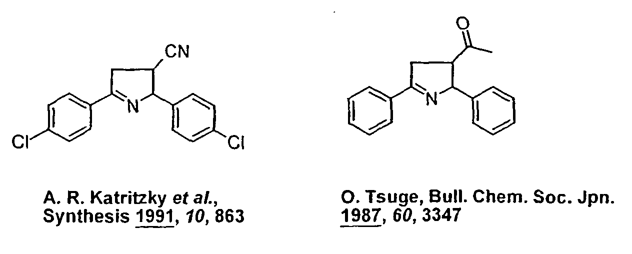

Über deren Eignung zur Verwendung als Schädlingsbekämpfungsmittel ist nichts bekannt.

In WO 00/21958, WO 99/59968, WO 99/59967 und WO 98/22438 wurden andere Δ¹-Pyrroline und deren Eignung als Schädlingsbekämpfungsmittel beschrieben.

Die Wirkungshöhe und/oder Wirkungsdauer dieser vorbekannten Verbindungen ist jedoch, insbesondere gegen bestimmte Organismen und/oder bei niedrigen Anwendungskonzentrationen, nicht in allen Anwendungsgebieten völlig zufriedenstellend.

Wegen der vielfältigen Anforderungen an moderne Schädlingsbekämpfungsmittel, beispielsweise bezüglich Wirkhöhe, Wirkdauer, Wirkspektrum, Anwendungsspektrum, Toxizität, Kombination mit anderen Wirkstoffen, Kombination mit Formulierungshilfsmitteln oder die Synthese, und wegen des möglichen Auftretens von Resistenzen kann die Entwicklung solcher Stoffe jedoch nie als abgeschlossen betrachtet werden, und es besteht beständig ein hoher Bedarf an neuen Verbindungen, die zumindest in Teilaspekten Vorteile gegenüber den bekannten Verbindungen besitzen.

Es wurden nun neue Δ¹-Pyrroline der Formel (I) 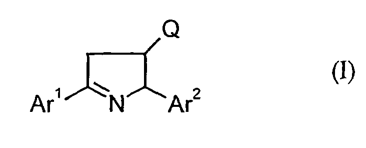 gefunden, in welcher
- Ar¹: für den Rest 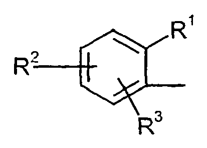 steht und
- Ar²: für den Rest 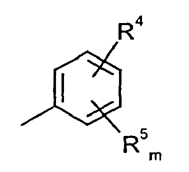 steht, in denen
- m: für 0, 1, 2, 3 oder 4 steht,
- R¹: für Halogen, Alkyl, Alkoxy, Halogenalkyl, Halogenalkoxy, Alkoxyalkyl, -S(O)ₒR⁶, -NR⁷R⁸ steht,
- R² und R³: unabhängig voneinander für Wasserstoff, Halogen, Cyano, Nitro, Alkyl, Alkoxy, Halogenalkyl, Halogenalkoxy, Alkoxyalkyl, -S(O)ₒR⁶, -NR⁷R⁸ stehen,
- R⁴: für Halogen oder eine der folgenden Gruppierungen
(l) -X-A
(m) -B-Z-D
(n) -Y-E
steht,
- R⁵: für Halogen, Hydroxy, Cyano, -CONH₂, -CSNH₂, Nitro, Alkyl, Alkylcarbonyl, Alkoxy, Halogenalkyl, Halogenalkoxy, Halogenalkylsulfonyloxy, Trialkylsilyl, Alkoxycarbonyl, -CONR⁷R⁸, -OSO₂NR⁷R⁸, -S(O)ₒR⁶, -NR⁷R⁸, -NHCO₂R⁶, Halogenalkylaminosulfonyl, Bisalkoxyboran, -B(OH)₂ steht,
- X: für eine direkte Bindung, Sauerstoff, -S(O)ₒ, -NR⁶, Carbonyl, Carbonyloxy, Oxycarbonyl, Oxysulfonyl (OSO₂), Alkylen, Alkenylen, Alkinylen, Alkylenoxy, Oxyalkylen, Oxyalkylenoxy, Thioalkylen, Cyclopropylen, Oxiranylen steht,
- A: für jeweils gegebenenfalls einfach oder mehrfach durch Reste aus der Liste W¹ substituiertes Phenyl, Naphthyl oder Tetrahydronaphthyl oder für gegebenenfalls einfach oder mehrfach durch Reste aus der Liste W² substituiertes 5-bis 10-gliedriges, gesättigtes oder ungesättigtes Heterocyclyl mit einem oder mehreren Heteroatomen aus der Reihe Stickstoff, Sauerstoff und Schwefel steht,
- B: für gegebenenfalls einfach oder zweifach durch Reste aus der Liste W¹ substituiertes p-Phenylen steht,
- Z: für -(CH₂)ₙ-, Sauerstoff oder -S(O)ₒ- steht,
- D: für Wasserstoff, Alkyl, Alkenyl, Alkinyl, Halogenalkyl, Halogenalkenyl, Halogenalkylsulfonyl, Dialkylaminosulfonyl steht,
- Y: für eine direkte Bindung, Sauerstoff, Schwefel, -SO₂-, Carbonyl, Carbonyloxy, Oxycarbonyl, Alkylen, Alkenylen, Alkinylen, Alkylenoxy, Oxyalkylen, Oxyalkylenoxy, Thioalkylen, Halogenalkylen, Halogenalkenylen steht,
- E: für Wasserstoff, Alkyl, Alkenyl, Alkinyl, Halogenalkyl, Halogenalkenyl, Halogenalkylsulfonyl, Dialkylaminosulfonyl steht,
- W¹: für Cyano, Halogen, Formyl, Nitro, Alkyl, Trialkylsilyl, Alkoxy, Halogenalkyl, Halogenalkoxy, Halogenalkenyloxy, Halogenalkylsulfonyloxy, Alkylcarbonyl, Alkoxycarbonyl, Pentafluorthio, -S(O)ₒR⁶, -SO₂NR⁷R⁸, -OSO₂NR⁷R⁸, -OSO₂N(R⁶)CO₂R⁶, -OSO₂R¹², -C(R⁶)=N-O(R⁶) steht,
- W²: für Cyano, Halogen, Formyl, Nitro, Alkyl, Trialkylsilyl, Alkoxy, Halogenalkyl, Halogenalkoxy, Halogenalkenyloxy, Halogenalkylsulfonyloxy, Alkylcarbonyl, Alkoxycarbonyl, -S(O)ₒR⁶, -SO₂NR⁷R⁸, -OSO₂NR⁷R⁸, -C(R6)=N-O(R⁶) steht,
- n: für 0, 1, 2, 3 oder 4 steht,
- Q: für -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, einen 5- bis 7-gliedrigen gesättigten oder ungesättigten Heterocyclus mit 2 bis 4 Heteroatomen aus der Reihe Stickstoff, Sauerstoff und Schwefel steht,
- Q: außerdem für -CO₂R¹³ oder -CONR¹⁴R¹⁵ steht,
- o: für 0, 1 oder 2 steht,
- R⁶: für Wasserstoff, Alkyl oder Halogenalkyl steht,
- R⁷ und R⁸: unabhängig voneinander für Wasserstoff, Alkyl, Cycloalkyl, Halogenalkyl, oder gemeinsam für Alkylen stehen,
- R⁹: für Wasserstoff, Alkyl, Cycloalkyl, Halogenalkyl, Aralkyl, Phenyl steht,
- R¹⁰: für Alkyl, Halogenalkyl, Aralkyl steht,
- R¹¹: für Alkyl, Aryl steht,
- R¹²: für Alkyl, Halogenalkyl, Aralkyl oder Aryl steht,
- R¹³: für Wasserstoff; für einfach oder mehrfach substituiertes Alkyl; für gegebenenfalls substituiertes Aminocarbonylalkyl; für Alkenyl, Phenylalkenyl; für jeweils gegebenenfalls substituiertes Phenylalkyl oder Phenoxyalkyl; für jeweils gegebenenfalls substituiertes Cycloalkyl oder Cycloalkylalkyl; für gesättigtes oder ungesättigtes Heterocyclyl oder Heterocyclylalkyl mit jeweils 1 bis 4 Heteroatomen kombiniert aus der Reihe Stickstoff, Sauerstoff und Schwefel; für Tetrahydronaphthyl oder Indanyl steht,
- R¹⁴ und R¹⁵: unabhängig voneinander für Wasserstoff, Halogenalkyl, Alkoxyalkyl; für jeweils gegebenenfalls substituiertes Phenyl oder Phenylalkyl; für jeweils gegebenenfalls substituiertes Cycloalkyl oder Cycloalkylalkyl; für jeweils gegebenenfalls substituiertes, jeweils gesättigtes oder ungesättigtes Heterocyclyl oder Heterocyclylalkyl mit jeweils 1 bis 4 Heteroatomen aus der Reihe Stickstoff, Sauerstoff und Schwefel stehen,
- R¹⁴ und R¹⁵: außerdem gemeinsam für Alkylenoxyalkylen oder Alkylenthioalkylen stehen.

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die reinen Isomere als auch die Isomerengemische werden erfindungsgemäß beansprucht.

Weiterhin wurde gefunden, dass man die neuen Verbindungen der Formel (I) nach einem der im folgenden beschriebenen Verfahren erhalten kann.

Δ¹-Pyrroline der Formel (I) 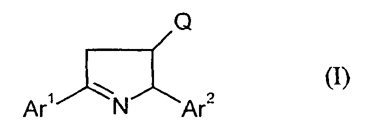 in welcher
- Ar¹, Ar², und Q: die oben angegebenen Bedeutungen haben,
lassen sich herstellen,
A-1.) indem man Iminochloride der Formel (II-a) 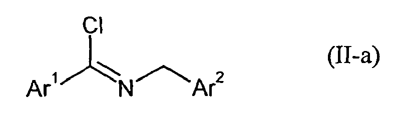 mit Dipolarophilen der Formel (III) 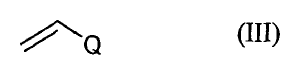 in welchen
   - Ar¹, Ar², und Q: die oben angegebenen Bedeutungen haben,
   in Gegenwart eines Säurebindemittels und gegebenenfalls in Gegenwart eines Verdünnungsmittels umsetzt, oder
A-2.) indem man Iminochloride der Formel (II-b) 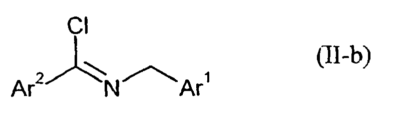 mit Dipolarophilen der Formel (III) 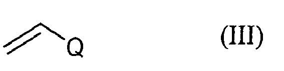 in welchen
   - Ar¹, Ar², und Q: die oben angegebenen Bedeutungen haben,
   in Gegenwart eines Säurebindemittels und gegebenenfalls in Gegenwart eines Verdünnungsmittels umsetzt, oder
B.) indem man Oxazolinone der Formel (IV) 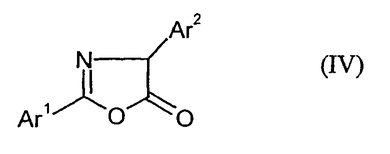 mit Dipolarophilen der Formel (III) 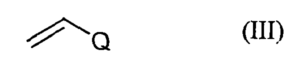 in welchen
   - Ar¹, Ar² und Q: die oben angegebenen Bedeutungen haben,
   gegebenenfalls in Gegenwart eines Verdünnungsmittels umsetzt, oder
C.) indem man Carbonsäuren der Formel (V) 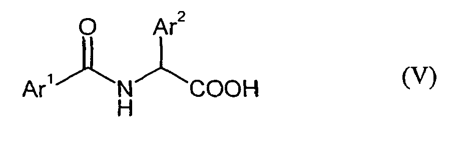 mit Dipolarophilen der Formel (III) 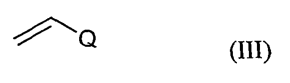 in welchen
   - Ar¹, Ar² und Q: die oben angegebenen Bedeutungen haben,
   in Gegenwart von Essigsäureanhydrid und gegebenenfalls in Gegenwart eines Verdünnungsmittels umsetzt.
D.) Δ¹-Pyrroline der Formel (I-a) 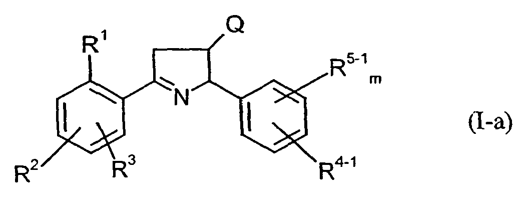
in welcher
- R¹, R², R³, Q und m: die oben angegebenen Bedeutungen haben,
- R⁴⁻¹: für A oder B-Z-D
steht, wobei
- A, B, Z und D: die oben angegebenen Bedeutungen haben, und
- R⁵⁻¹: für Fluor, Hydroxy, Cyano, Nitro, Alkyl, Alkoxy, Halogenalkyl, Halogenalkoxy, Trialkylsilyl, Alkoxycarbonyl, -CONR⁷R⁸, -OSO₂NR⁷R⁸, -S(O)ₒR⁶, -NR⁷R⁸, -NHCO₂R⁶, Haloalkylaminosulfonyl steht,
wobei
- R⁶, R⁷, R⁸ und o: die oben angegebenen Bedeutungen haben,
lassen sich herstellen, indem man Verbindungen der Formel (I-b) 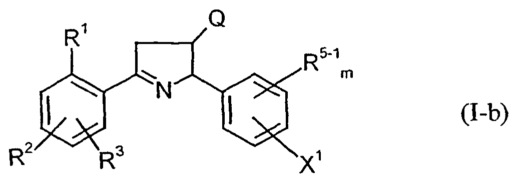 in welcher
- R¹, R², R³, R⁵⁻¹, Q und m: die oben angegebenen Bedeutungen haben und
- X¹: für Cl, Br, I, -OSO₂CF₃ steht
mit Boronsäuren der Formel (VI) 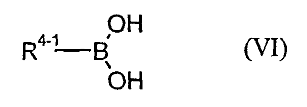 in welcher
- R⁴⁻¹: die oben angegebene Bedeutung hat,
in Gegenwart eines Katalysators, gegebenenfalls in Gegenwart eines Säurebindemittels und gegebenenfalls in Gegenwart eines Verdünnungsmittels umsetzt,
oder indem man Verbindungen der Formel (I-b)
in welcher
- X¹: für 2-(4,4,5,5-Tetramethyl-1,3,2-dioxoborolan) steht
mit (Hetero)cyclen der Formel (VII)

T-A (VII)

in welcher A die oben angegebene Bedeutung hat und
- T: für Cl, Br, I, -OSO₂CF₃ steht
in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Verbindungen sind durch die Formel (I) allgemein definiert. Bevorzugte Substituenten bzw. Bereiche der in den oben und nachstehend erwähnten Formeln aufgeführten Reste werden im folgenden erläutert.
- Ar¹: steht bevorzugt für den Rest 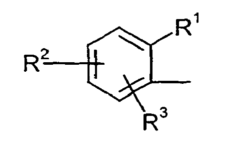
- Ar²: steht bevorzugt für den Rest 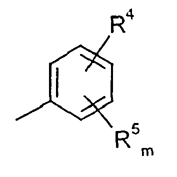
- m: steht bevorzugt für 0, 1, 2 oder 3.
- R¹: steht bevorzugt für Halogen, C₁-C₆-Alkyl, C₁-C₆-Alkoxy, C₁-C₆-Halogenalkyl, C₁-C₆-Halogenalkoxy, C₁-C₆-Alkoxy-C₁-C₆-alkyl, -S(O)ₒR⁶, -NR⁷R⁸.
- R² und R³: stehen unabhängig voneinander bevorzugt für Wasserstoff, Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkoxy-C₁-C₆-alkyl, -S(O)ₒR⁶, -NR⁷R⁸.
- R⁴: steht bevorzugt für Fluor, Chlor, Brom, Iod oder eine der folgenden in ortho- oder para-Position des Arylrings befindliche Gruppierungen
(l) -X-A
(m) -B-Z-D
(n) -Y-E
- R⁵: steht bevorzugt für Halogen, Hydroxy, Cyano, -CONH₂, -CSNH₂, Nitro, C₁-C₆-Alkyl, C₁-C₆-Alkylcarbonyl, C₁-C₆-Alkoxy, C₁-C₆-Halogenalkyl, C₁-C₆-Halogenalkoxy, (C₁-C₆-Halogenalkyl)sulfonyloxy, Tri(C₁-C₆-alkyl)-silyl, C₁-C₆-Alkoxycarbonyl, -CONR⁷R⁸, -OSO₂NR⁷R⁸, -S(O)ₒR⁶, -NR⁷R⁸, -NHCO₂R⁶, (C₁-C₆-Halogenalkyl)aminosulfonyl, Bis(C₁-C₆-alkoxy)boran, -B(OH)₂.
- X: steht bevorzugt für eine direkte Bindung, Sauerstoff, -S(O)ₒ, -NR⁶, Carbonyl, Carbonyloxy, Oxycarbonyl, Oxysulfonyl (OSO₂), C₁-C₄-Alkylen, C₂-C₄-Alkenylen, C₂-C₄-Alkinylen, C₁-C₄-Alkylenoxy, C₁-C₄-Oxyalkylen, C₁-C₄-Oxyalkylenoxy, C₁-C₄-Thioalkylen, Cyclopropylen, Oxiranylen.
- A: steht bevorzugt für jeweils gegebenenfalls einfach bis vierfach durch Reste aus der Liste W¹ substituiertes Phenyl, Naphthyl oder Tetrahydronaphthyl oder für gegebenenfalls einfach bis vierfach durch Reste aus der Liste W² substituiertes 5- bis 10-gliedriges, 1 oder 2 aromatische Ringe enthaltendes Heterocyclyl mit 1 bis 4 Heteroatomen, kombiniert aus 0 bis 4 Stickstoffatomen, 0 bis 2 Sauerstoffatomen und 0 bis 2 Schwefelatomen (insbesondere Tetrazolyl, Furyl, Benzofuryl, Thienyl, Benzothienyl, Pyrrolyl, Indolyl, Oxazolyl, Benzoxazolyl, Isoxazyl, Imidazyl, Pyrazyl, Thiazolyl, Benzothiazolyl, Pyridyl, Pyrimidinyl, Pyridazyl, Triazinyl, Triazyl, Chinolinyl oder Isochinolinyl).
- B: steht bevorzugt für gegebenenfalls einfach oder zweifach durch Reste aus der Liste W¹ substituiertes p-Phenylen.
- Z: steht bevorzugt für -(CH₂)ₙ-, Sauerstoff oder -S(O)ₒ-.
- D: steht bevorzugt für Wasserstoff, C₁-C₆-Alkyl, C₂-C₆-Alkenyl, C₂-C₆-Alkinyl, C₁-C₆-Halogenalkyl, C₂-C₆-Halogenalkenyl, (C₁-C₆-Halogenalkyl)sulfonyl, Di(C₁-C₆-alkyl)aminosulfonyl.
- Y: steht bevorzugt für eine direkte Bindung, Sauerstoff, Schwefel, -SO₂-, Carbonyl, Carbonyloxy, Oxycarbonyl, C₁-C₆-Alkylen, C₂-C₆-Alkenylen, C₂-C₆-Alkinylen, C₁-C₆-Halogenalkylen, C₂-C₆-Halogenalkenylen, C₁-C₄-Alkylenoxy, C₁-C₄-Oxyalkylen, C₁-C₄-Oxyalkylenoxy, C₁-C₄-Thioalkylen.
- E: steht bevorzugt für Wasserstoff, C₁-C₆-Alkyl, C₂-C₆-Alkenyl, C₂-C₆-Alkinyl, C₁-C₆-Halogenalkyl, C₂-C₆-Halogenalkenyl, (C₁-C₆-Halogenalkyl)sulfonyl, Di(C₁-C₆-alkyl)aminosulfonyl.
- W¹: steht bevorzugt für Cyano, Halogen, Formyl, Nitro, C₁-C₆-Alkyl, Tri(C₁-C₄-alkyl)silyl, C₁-C₆-Alkoxy, C₁-C₆-Halogenalkyl, C₁-C₆-Halogenalkoxy, C₂-C₆-Halogenalkenyloxy, (C₁-C₆-Halogenalkyl)sulfonyloxy, C₁-C₆-Alkylcarbonyl, C₁-C₆-Alkoxycarbonyl, Pentafluorthio, -S(O)ₒR⁶, -SO₂NR⁷R⁸, -OSO₂NR⁷R⁸, -OSO₂N(R⁶)CO₂R⁶, -OSO₂R¹², -C(R⁶)=N-O(R⁶).
- W²: steht bevorzugt für Cyano, Halogen, Formyl, Nitro, C₁-C₆-Alkyl, Tri(C₁-C₄-alkyl)silyl, C₁-C₆-Alkoxy, C₁-C₆-Halogenalkyl, C₁-C₆-Halogenalkoxy, C₂-C₆-Halogenalkenyloxy, (C₁-C₆-Halogenalkyl)sulfonyloxy, C₁-C₆-Alkylcarbonyl, C₁-C₆-Alkoxycarbonyl, -S(O)ₒR⁶, -SO₂NR⁷R⁸, -OSO₂NR⁷R⁸, -C(R⁶)=N-O(R⁶).
- n: steht bevorzugt für 0, 1, 2, 3 oder 4.
- Q: steht bevorzugt für -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, einen 5- bis 7-gliedrigen gesättigten oder ungesättigten Heterocyclus mit 2 bis 4 Heteroatomen aus der Reihe Stickstoff, Sauerstoff und Schwefel.
- Q: steht außerdem bevorzugt für -CO₂R¹³ oder -CONR¹⁴R¹⁵.
- o: steht bevorzugt für 0, 1 oder 2.
- R⁶: steht bevorzugt für Wasserstoff, C₁-C₆-Alkyl, C₁-C₆-Halogenalkyl.
- R⁷ und R⁸: stehen unabhängig voneinander bevorzugt für Wasserstoff, C₁-C₆-Alkyl, C₃-C₆-Cycloalkyl, C₁-C₆-Halogenalkyl, oder gemeinsam für Alkylen.
- R⁹: steht bevorzugt für Wasserstoff, C₁-C₆-Alkyl, C₃-C₆-Cycloalkyl, C₁-C₆-Halogenalkyl, Benzyl, Phenyl.
- R¹⁰: steht bevorzugt für C₁-C₆-Alkyl, C₁-C₆-Halogenalkyl, Benzyl.
- R¹¹: steht bevorzugt für C₁-C₆-Alkyl, Phenyl.
- R¹²: steht bevorzugt für C₁-C₆-Alkyl, C₁-C₆-Halogenalkyl, Benzyl oder Phenyl.
- R¹³: steht bevorzugt für Wasserstoff; für einfach bis dreifach, gleich oder verschieden durch Cyano, Nitro, C₁-C₄-Alkoxy, C₁-C₄-Alkoxycarbonyl oder 2-Pyrrolidinon substituiertes C₁-C₆-Alkyl; für Aminocarbonyl-C₁-C₆-alkyl, welches an der Aminogruppe gleich oder verschieden durch C₁-C₄-Alkyl, Phenyl oder Halogenphenyl substituiert sein kann; für C₂-C₈-Alkenyl, Phenyl-C₂-C₆-alkenyl, für Phenyl-C₁-C₄-alkyl oder Phenoxy-C₁-C₄-alkyl, welche jeweils im Phenylring einfach bis vierfach, gleich oder verschieden durch Halogen, C₁-C₄-Alkyl, C₁-C₄-Alkoxy oder C₁-C₄-Halogenalkoxy substituiert sein können; für C₃-C₆-Cycloalkyl oder C₃-C₆-Cycloalkyl-C₁-C₄-alkyl, welche jeweils im Cycloalkylring einfach bis dreifach durch C₁-C₄-Alkyl substituiert sein können; für 5- oder 6-gliedriges gesättigtes oder ungesättigtes Heterocyclyl oder Heterocyclyl-C₁-C₄-alkyl mit jeweils 1 bis 4 Heteroatomen kombiniert aus 0 bis 4 Stickstoffatomen, 0 bis 2 Sauerstoffatomen und 0 bis 2 Schwefelatomen (insbesondere Furyl, Thienyl, Pyridinyl, Furfuryl, Thenyl oder Pyridinylmethyl), wobei der Heterocyclus jeweils durch Halogen substituiert sein kann; für Tetrahydronaphthyl oder Indanyl.
- R¹⁴ und R¹⁵: stehen unabhängig voneinander bevorzugt für Wasserstoff, C₁-C₆-Halogenalkyl, C₁-C₆-Alkoxy-C₁-C₆-alkyl; für Phenyl oder Phenyl-C₁-C₄-alkyl, welche jeweils im Phenylring einfach bis vierfach, gleich oder verschieden durch Halogen, C₁-C₄-Alkyl, C₁-C₄-Alkoxy, C₁-C₄-Halogenalkyl oder C₁-C₄-Halogenalkoxy substituiert sein können; für C₃-C₆-Cycloalkyl oder C₃-C₆-Cycloalkyl-C₁-C₄-alkyl, welche jeweils im Cycloalkylring einfach bis dreifach, gleich oder verschieden durch Halogen, C₁-C₄-Alkyl, C₁-C₄-Alkoxy, C₁-C₄-Halogenalkyl oder C₁-C₄-Halogenalkoxy substituiert sein können; für 5- oder 6-gliedriges gesättigtes oder ungesättigtes Heterocyclyl oder Heterocyclyl-C₁-C₄-alkyl mit jeweils 1 bis 4 Heteroatomen kombiniert aus 0 bis 4 Stickstoffatomen, 0 bis 2 Sauerstoffatomen und 0 bis 2 Schwefelatomen (insbesondere Furyl, Thienyl, Tetrahydrofuryl, Furfuryl, Thenyl, Tetrahydrofurylmethyl, Thiazolyl), wobei der Heterocyclus jeweils durch Halogen oder C₁-C₄-Alkoxycarbonyl substituiert sein kann.
- R¹⁴ und R¹⁵: stehen außerdem gemeinsam bevorzugt für C₁-C₄-Alkylenoxy-C₁-C₄-alkylen oder C₁-C₄-Alkylenthio-C₁-C₄-alkylen.
- Ar¹: steht besonders bevorzugt für den Rest 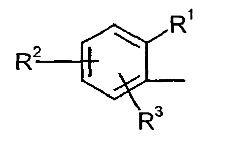
- Ar²: steht besonders bevorzugt für den Rest 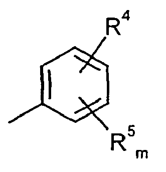
- m: steht besonders bevorzugt für 0, 1 oder 2.
- R¹: steht besonders bevorzugt für Fluor, Chlor, Brom, C₁-C₆-Alkyl, C₁-C₆-Alkoxy, jeweils durch Fluor oder Chlor substituiertes C₁-C₆-Alkyl oder C₁-C₆-Alkoxy.
- R² und R³: stehen unabhängig voneinander besonders bevorzugt für Wasserstoff, Fluor, Chlor, Brom, Iod, C₁-C₆-Alkyl, C₁-C₆-Alkoxy, jeweils durch Fluor oder Chlor substituiertes C₁-C₆-Alkyl oder C₁-C₆-Alkoxy.
- R⁴: steht besonders bevorzugt für Chlor, Brom, Iod oder eine der folgenden in ortho- oder para-Position des Arylrings befindlichen Gruppierungen
(l) -X-A
(m) -B-Z-D
(n) -Y-E
- R⁵: steht besonders bevorzugt für Fluor, Chlor, Brom, Iod, Hydroxy, Cyano, -CONH₂, -CSNH₂, Nitro, C₁-C₆-Alkyl, C₁-C₆-Alkylcarbonyl, C₁-C₆-Alkoxy, jeweils durch Fluor oder Chlor substituiertes C₁-C₆-Alkyl oder C₁-C₆-Alkoxy, -OSO₂CF₃, -CONR⁷R⁸, -OSO₂NR⁷R⁸, -S(O)ₒR⁶, -NR⁷R⁸, -NHCO₂R⁶, -B(OH)₂, 2-(4,4,5,5-Tetramethyl-1,3,2-dioxoborolan).
- X: steht besonders bevorzugt für eine direkte Bindung, Sauerstoff, Schwefel, -SO₂-, Carbonyl, Carbonyloxy, Oxycarbonyl, Oxysulfonyl (OSO₂), C₁-C₄-Alkylen, C₂-C₄-Alkenylen, C₂-C₄-Alkinylen, C₁-C₄-Alkylenoxy, C₁-C₄-Oxyalkylen, C₁-C₄-Oxyalkylenoxy, C₁-C₄-Thioalkylen, Cyclopropylen, Oxiranylen.
- A: steht besonders bevorzugt für jeweils gegebenenfalls einfach bis dreifach durch Reste aus der Liste W¹ substituiertes Phenyl, Naphthyl oder Tetrahydronaphthyl oder für gegebenenfalls einfach bis dreifach durch Reste aus der Liste W² substituiertes 5- bis 10-gliedriges, 1 oder 2 aromatische Ringe enthaltendes Heterocyclyl mit 1 bis 4 Heteroatomen, kombiniert aus 0 bis 4 Stickstoffatomen, 0 bis 2 Sauerstoffatomen und 0 bis 2 Schwefelatomen (insbesondere Tetrazolyl, Furyl, Benzofuryl, Thienyl, Benzothienyl, Pyrrolyl, Indolyl, Oxazolyl, Benzoxazolyl, Isoxazyl, Imidazyl, Pyrazyl, Thiazolyl, Benzothiazolyl, Pyridyl, Pyrimidinyl, Pyridazyl, Triazinyl, Triazyl, Chinolinyl oder Isochinolinyl).
- B: steht besonders bevorzugt für gegebenenfalls einfach oder zweifach durch Reste aus der Liste W¹ substituiertes p-Phenylen.
- Z: steht besonders bevorzugt für -(CH₂)ₙ-, Sauerstoff oder -S(O)ₒ-.
- D: steht besonders bevorzugt für Wasserstoff, C₁-C₆-Alkyl, C₂-C₆-Alkenyl, C₂-C₆-Alkinyl, Di(C₁-C₄-Alkyl)aminosulfonyl, jeweils durch Fluor oder Chlor substituiertes C₁-C₆-Alkyl, C₂-C₆-Alkenyl oder C₁-C₄-Alkylsulfonyl.
- Y: steht besonders bevorzugt für eine direkte Bindung, Sauerstoff, Schwefel, -SO₂-, Carbonyl, Carbonyloxy, Oxycarbonyl, C₁-C₆-Alkylen, C₂-C₆-Alkenylen, C₂-C₆-Alkinylen; jeweils durch Fluor oder Chlor substituiertes C₁-C₆-Alkylen oder C₂-C₆-Alkenylen; C₁-C₄-Alkylenoxy, C₁-C₄-Oxyalkylen, C₁-C₄-Oxyalkylenoxy, C₁-C₄-Thioalkylen.
- E: steht besonders bevorzugt für Wasserstoff, C₁-C₆-Alkyl, C₂-C₆-Alkenyl, C₂-C₆-Alkinyl, Di(C₁-C₆-alkyl)aminosulfonyl, jeweils durch Fluor oder Chlor substituiertes C₁-C₆-Alkyl, C₂-C₆-Alkenyl oder C₁-C₆-Alkylsulfonyl.
- W¹: steht besonders bevorzugt für Cyano, Fluor, Chlor, Brom, Iod, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, C₂-C₆-Halogenalkenyloxy, (C₁-C₄-Halogenalkyl)sulfonyloxy, C₁-C₄-Alkylcarbonyl, C₁-C₄-Alkoxycarbonyl, -S(O)ₒR⁶, -SO₂NR⁷R⁸, -OSO₂NR⁷R⁸, -OSO₂R¹², -C(R⁶)=N-O(R6).
- W²: steht besonders bevorzugt für Cyano, Fluor, Chlor, Brom, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, C₂-C₆-Halogenalkenyloxy, -OSO₂CF₃, C₁-C₄-Alkylcarbonyl, C₁-C₄-Alkoxycarbonyl, -S(O)ₒR⁶, -SO₂NR⁷R⁸, -OS0₂NR⁷R⁸, -C(R⁶)=N-O(R⁶).
- n: steht besonders bevorzugt für 0, 1, 2 oder 3.
- Q: steht besonders bevorzugt für -CO₂R⁹, -COR¹⁰, -CONR⁷R⁸, -CN, -PO(OR¹¹)₂, einen 5- bis 7-gliedrigen gesättigten oder ungesättigten Heterocyclus mit 2- bis 4 Heteroatomen aus der Reihe Stickstoff, Sauerstoff und Schwefel (insbesondere Dihydrodioxazin, Oxazolin, Thiazolin, Imidazolin, Tetrazol).
- Q: steht außerdem besonders bevorzugt für -CO₂R¹³ oder -CONR¹⁴R¹⁵.
- o: steht besonders bevorzugt für 0, 1 oder 2.
- R⁶: steht besonders bevorzugt für C₁-C₆-Alkyl oder jeweils durch Fluor oder Chlor substituiertes Methyl oder Ethyl.
- R⁷ und R⁸: stehen unabhängig voneinander besonders bevorzugt für Wasserstoff, C₁-C₆-Alkyl, C₃-C₆-Cycloalkyl, jeweils durch Fluor oder Chlor substituiertes C₁-C₆-Alkyl, oder gemeinsam für C₄-C₅-Alkylen.
- R⁹: steht besonders bevorzugt für Wasserstoff C₁-C₆-Alkyl, C₃-C₆-Cycloalkyl, durch Fluor oder Chlor substituiertes C₁-C₆-Alkyl, Benzyl, Phenyl.
- R¹⁰: steht besonders bevorzugt für C₁-C₆-Alkyl, durch Fluor oder Chlor substituiertes C₁-C₆-Alkyl.
- R¹¹: steht besonders bevorzugt für C₁-C₄-Alkyl, Phenyl.
- R¹²: steht besonders bevorzugt für C₁-C₄-Alkyl oder für durch Fluor oder Chlor substituiertes C₁-C₄-Alkyl.
- R¹³: steht besonders bevorzugt für Wasserstoff; für einfach oder zweifach, gleich oder verschieden durch Cyano, Nitro, Methoxy, Methoxycarbonyl oder 2-Pyrrolidinon substituiertes C₁-C₄-Alkyl; für Aminocarbonyl-C₁-C₄-alkyl, welches an der Aminogruppe gleich oder verschieden durch C₁-C₄-Alkyl, Phenyl oder Halogenphenyl substituiert sein kann; für C₂-C₆-Alkenyl, Phenyl-C₂-C₅-alkenyl, für Phenyl-C₁-C₄-alkyl oder Phenoxy-C₁-C₄-alkyl, welche jeweils im Phenylring einfach bis dreifach, gleich oder verschieden durch Fluor, Chlor, Methyl, Methoxy oder Trifluormethoxy substituiert sein können; für C₃-C₆-Cycloalkyl oder C₃-C₆-Cycloalkyl-C₁-C₄-alkyl, welche jeweils im Cycloalkylring einfach bis dreifach durch Methyl substituiert sein können; für Furyl, Thienyl, Pyridinyl, Furfuryl, Thenyl oder Pyridinylmethyl, welche jeweils im Heterocyclus durch Chlor substituiert sein können; für Tetrahydronaphthyl oder Indanyl.
- R¹⁴: steht besonders bevorzugt für Wasserstoff.
- R¹⁵: steht besonders bevorzugt für C₁-C₄-Halogenalkyl, C₁-C₄-Alkoxy-C₁-C₄-alkyl; für Phenyl oder Phenyl-C₁-C₄-alkyl, welche jeweils im Phenylring einfach bis vierfach, gleich oder verschieden durch Fluor, Chlor, Methyl, Methoxy, Trifluormethyl oder Trifluormethoxy substituiert sein können; für C₃-C₆-Cycloalkyl oder C₃-C₆-Cycloalkyl-C₁-C₄-alkyl, welche jeweils im Cycloalkylring einfach bis dreifach, gleich oder verschieden durch Fluor, Chlor, Methyl, Trifluormethyl oder Trifluormethoxy substituiert sein können; für Furyl, Thienyl, Tetrahydrofuryl, Furfuryl, Thenyl, Tetrahydrofurylmethyl, Thiazolyl oder durch C₁-C₄-Alkoxycarbonyl substituiertes Thiazolyl.
- R¹⁴ und R¹⁵: stehen außerdem gemeinsam besonders bevorzugt für Morpholino oder Thiomorpholino.
- Ar¹: steht ganz besonders bevorzugt für den Rest 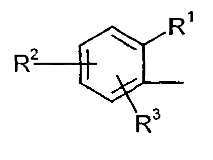
- Ar²: steht ganz besonders bevorzugt für den Rest 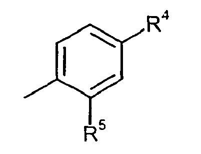
- R¹: steht ganz besonders bevorzugt für Fluor, Chlor, Brom, Methyl, Ethyl, n-Propyl, Isopropyl, n-Butyl, Isobutyl, sec-Butyl, tert-Butyl, Methoxy, Ethoxy, n-Propoxy, n-Butoxy, Isobutoxy, sec-Butoxy, tert-Butoxy.
- R² und R³: stehen unabhängig voneinander ganz besonders bevorzugt für Wasserstoff, Fluor, Chlor, Brom, Methyl, Ethyl, n-Propyl, Isopropyl, n-Butyl, Isobutyl, sec-Butyl, tert-Butyl, Methoxy, Ethoxy, n-Propoxy, n-Butoxy, Isobutoxy, sec-Butoxy, tert-Butoxy.
- R⁴: steht ganz besonders bevorzugt für Chlor, Brom oder eine der folgenden Gruppierungen
(l) -X-A
(m) -B-Z-D
(n) -Y-E
- R⁵: steht ganz besonders bevorzugt für Fluor, Chlor, Brom, Hydroxy, Methyl, Ethyl, Methoxy, Ethoxy, Methylthio, Ethylthio, Trifluormethyl, Difluormethoxy, Trifluormethoxy, Trifluormethylthio, -OSO₂CF₃, -OSO₂NMe₂ oder -SO₂CF₃.
- R⁵: steht außerdem ganz besonders bevorzugt für Wasserstoff.
- X: steht ganz besonders bevorzugt für eine direkte Bindung, Sauerstoff, Schwefel, -SO₂-, Carbonyl, -CH₂-, -(CH₂)₂-, -CH=CH- (E oder Z), -C≡C-, -CH₂O-, -(CH₂)₂O-, -OCH₂-, -SCH₂-, -S(CH₂)₂-, -OCH₂O-, -O(CH₂)₂O-.
- A: steht ganz besonders bevorzugt für gegebenenfalls einfach oder zweifach durch Reste aus der Liste W¹ substituiertes Phenyl, oder für jeweils gegebenenfalls einfach oder zweifach durch Reste aus der Liste W² substituiertes Tetrazolyl, Furyl, Benzofuryl, Thienyl, Benzothienyl, Pyrrolyl, Indolyl, Oxazolyl, Benzoxazolyl, Isoxazyl, Imidazyl, Pyrazyl, Thiazolyl, Benzothiazolyl, Pyridyl, Pyrimidinyl, Pyridazyl, Triazinyl, Triazyl.
- B: steht ganz besonders bevorzugt für gegebenenfalls einfach durch Reste aus der Liste W¹ substituiertes p-Phenylen.
- Z: steht ganz besonders bevorzugt für Sauerstoff, Schwefel oder -SO₂-.
- D: steht ganz besonders bevorzugt für Wasserstoff, Methyl, Ethyl, n-Propyl, Isopropyl, n-Butyl, Isobutyl, sec-Butyl, tert-Butyl, 2-Propenyl, Butenyl, Propargyl, Butinyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, -SO₂CF₃, -SO₂(CF₂)₃CF₃, -SO₂NMe₂.
- Y: steht ganz besonders bevorzugt für eine direkte Bindung, Sauerstoff, Schwefel, -SO₂-, Carbonyl, -CH₂-, -(CH₂)₂-, -CH=CH- (E oder Z), -C≡C-, -CH₂O-, -(CH₂)₂O-, -OCH₂-, -SCH₂-, -S(CH₂)₂-, -OCH₂O-, -O(CH₂)₂O-.
- E: steht ganz besonders bevorzugt für Wasserstoff, Methyl, Ethyl, n-Propyl, Isopropyl, n-Butyl, Isobutyl, sec-Butyl, tert-Butyl, 2-Propenyl, Butenyl, Propargyl, Butinyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, -SO₂CF₃, -SO₂(CF₂)₃CF₃, -SO₂NMe₂.
- W¹: steht ganz besonders bevorzugt für Cyano, Fluor, Chlor, Brom, Formyl, Nitro, Methyl, Ethyl, n-Propyl, Isopropyl, n-Butyl, Isobutyl, sec-Butyl, tert-Butyl, Methoxy, Ethoxy, n-Propoxy, n-Butoxy, Isobutoxy, sec-Butoxy, tert-Butoxy, Trifluormethoxy, Difluormethoxy, -CF₃, -CHF₂, -CClF₂, -CF₂CHFCl, -CF₂CH₂F, -CF₂CCl₃, -CH₂CF₃, -CF₂CHFCF₃, -CH₂CF₂H, -OCH₂CF₃, -CH₂CF₂CF₃, -CF₂CF₂H, -CF₂CHFCF₃, -SCF₃, -SCHF₂, -SO₂CHF₂, -SO₂CF₃, -SOCHF₂, -SOCF₃, -SO₂NMe₂, -OSO₂CH₃, -OSO₂CF₃, -OSO₂(CF2)₃CF₃, -COCH₃, -CO₂CH₃, -CO₂Et, -SO₂Me, -OSO₂NMe₂, -C(Me)=N-O(Et), -C(Et)=N-OMe.
- W²: steht ganz besonders bevorzugt für Cyano, Fluor, Chlor, Brom, Methyl, Ethyl, n-Propyl, Isopropyl, tert-Butyl, Trifluormethyl, Trifluormethoxy, Difluormethoxy, Trifluormethylthio, -OSO₂CF₃, -COCH₃, -CO₂CH₃, -OCH₂CF₃, -SO₂CF₃, -SO₂NMe₂, -OSO₂NMe₂, -C(Me)=N-O(Et), -C(Et)=N-OMe.
- Q: steht ganz besonders bevorzugt für -CO₂CH₃, -CO₂CH₂CH₃, -CO₂-n-Propyl, -CO₂-Isopropyl, -CO₂-n-Butyl, -CO₂-Isobutyl, -CO₂-sec-Butyl, -CO₂-tert-Butyl, -CO₂-n-Pentyl, -CO₂-Neopentyl, -CO₂-sec-Isoamyl, -CO₂-Pentan-3-yl, -CO₂-Cyclopentyl, -CO₂-n-Hexyl, -CO₂-Cyclohexyl, -CO₂-Trifluorethyl, -CO₂CH₂Ph, -CO₂Ph, -COCH₃, -COCH₂CH₃, -CO-n-Propyl, -CONHMe, -CONHEt, -CONH(n-Propyl), -CONH(Isopropyl), -CONH(n-Butyl), -CONH(tert-Butyl), -CONH(n-Pentyl), -CONH(n-Hexyl), -CONH(Cyclohexyl), -CONMe₂, -CONEt₂, -CON(n-Propyl)₂, -CON(Isopropyl)₂, -CON(n-Butyl)₂, -CON(n-Pentyl)₂, -CON(Me)Et, -CON(Me)n-Propyl, -CON(Me)n-Butyl, -CON(Me)n-Pentyl, -CN, -PO(OMe)₂, Pyrrolidinocarbonyl, Piperidinocarbonyl, -PO(OEt)₂, -PO(OPh)₂, Dihydrodioxacinyl, Oxazolyl, Thiazolyl, Imidazolyl, Tetrazolyl.
- Q: steht außerdem ganz besonders bevorzugt für -CO₂CH₂CN, -CO₂(CH₂)₂CN, -CO₂(CH₂)₃CN, -CO₂H, -CO₂CH₂C(CH₃)₂NO₂, -CO₂(CH₂)₂OCH₃, 2-Methoxycarbonyl-propyloxycarbonyl, 3-(2-Oxo-1-pyrrolidinyl)propyloxycarbonyl, N-4-Fluorphenyl-N-isopropyl-amino-2-oxo-ethyloxycarbonyl, -CO₂(CH₂)₃CH=CH₂, -CO₂(CH₂)₂C(CH₃)=CH₂, -CO₂CH₂CH=CH-Ph, 2-Chlorbenzyloxycarbonyl, 3-Chlorbenzyloxycarbonyl, 4-Chlorbenzyloxycarbonyl, 1-Phenylethyloxycarbonyl, 3-Phenylpropyloxycarbonyl, 2-Phenoxyethyloxycarbonyl, 4-Methylcyclohexyloxycarbonyl, Cyclohexylmethyloxycarbonyl, 1-Cyclohexylethyloxycarbonyl, 3-Furfuryloxycarbonyl, 2-Thenyloxycarbonyl, 3-Thenyloxycarbonyl, 2-Pyridinylmethyloxycarbonyl, 3-Pyridinylmethyloxycarbonyl, 6-Chlor-3-pyridinylmethyloxycarbonyl, 1-Tetrahydronaphthyloxycarbonyl, 1-Indanyloxycarbonyl, -CON(H)CH₂CF₃, Methoxyethylaminocarbonyl, 4-Trifluormethoxyphenyl-aminocarbonyl, Benzyl-aminocarbonyl, 2-Chlorbenzyl-aminocarbonyl, 3-Chlorbenzyl-aminocarbonyl, 4-Chlorbenzyl-aminocarbonyl, 3-Methylbenzyl-aminocarbonyl, 4-Methylbenzyl-aminocarbonyl, 2,4-Dichlorbenzyl-aminocarbonyl, 2-Methoxybenzyl-aminocarbonyl, 2,3-Dimethoxybenzyl-aminocarbonyl, 3,5-Dimethylbenzyl-aminocarbonyl, 2,4-Difluorbenzyl-aminocarbonyl, 4-Trifluormethylcyclohexyl-aminocarbonyl, Cyclohexylmethyl-aminocarbonyl, 2-Thenylaminocarbonyl, 2-Tetrahydrofurylmethyl-aminocarbonyl, 2-Thiazolyl-aminocarbonyl, 5-Methoxycarbonyl-2-thiazolyl-aminocarbonyl oder für Morpholinocarbonyl.
- Ar²: steht insbesondere ganz besonders bevorzugt für den Rest 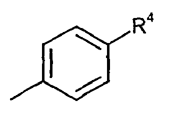
- R¹: steht insbesondere ganz besonders bevorzugt für Fluor oder Chlor.
- R²: steht insbesondere ganz besonders bevorzugt für Wasserstoff, Chlor oder Fluor.
- R⁴: steht insbesondere ganz besonders bevorzugt für -B-Z-D.
- B: steht insbesondere ganz besonders bevorzugt für p-Phenylen.
- Z: steht insbesondere ganz besonders bevorzugt für Sauerstoff oder Schwefel.
- D: steht insbesondere ganz besonders bevorzugt für -CF₃.
- Q: steht insbesondere ganz besonders bevorzugt für -CO₂CH₃, -CO₂CH₂CH₃, -CO₂-n-Propyl, -CO₂-Isopropyl, -CO₂-n-Butyl, -CO₂-Isobutyl, -CO₂-sec-Butyl, -CO₂-tert-Butyl, -CO₂-n-Pentyl, -CO₂-Neopentyl, -CO₂-sec-Isoamyl, -CO₂-Pentan-3-yl, -CO₂-Cyclopentyl, -CO₂-n-Hexyl, -CO₂-Cyclohexyl, -CONHMe, -CONHEt, -CONH(n-Propyl), -CONH(Isopropyl), -CONH(n-Butyl), -CONH(tert-Butyl), -CONH(n-Pentyl), -CONH(n-Hexyl), -CONH(Cyclohexyl), -CONMe₂, -CONEt₂, -CON(n-Propyl)₂, -CON(Isopropyl)₂, -CON(n-Butyl)₂, -CON(n-Pentyl)₂, -CON(Me)Et, -CON(Me)n-Propyl, -CON(Me)n-Butyl, -CON(Me)n-Pentyl, -CN, Pyrrolidinocarbonyl, Piperidinocarbonyl.
- Ar¹: steht hervorgehoben für den Rest 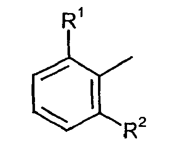
- Ar²: steht hervorgehoben für den Rest 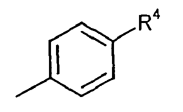
- R¹: steht hervorgehoben für Fluor oder Chlor.
- R²: steht hervorgehoben für Wasserstoff, Chlor oder Fluor.
- R⁴: steht hervorgehoben für -B-Z-D.
- B: steht hervorgehoben für p-Phenylen.
- Z: steht hervorgehoben für Sauerstoff oder Schwefel.
- D: steht hervorgehoben für -CF₃.
- Q: steht hervorgehoben für -CO₂CH₃, -CO₂CH₂CH₃, -CO₂-n-Propyl, -CO₂-Isopropyl, -CO₂-n-Butyl, -CO₂-Isobutyl, -CO₂-sec-Butyl, -CO₂-tert-Butyl, -CO₂-n-Pentyl, -CO₂-Neopentyl, -CO₂-sec-Isoamyl, -CO₂-Pentan-3-yl, -CO₂-Cyclopentyl, -CO₂-n-Hexyl, -CO₂-Cyclohexyl, -CONHMe, -CONHEt, -CONH(n-Propyl), -CONH(Isopropyl), -CONH(n-Butyl), -CONH(tert-Butyl), -CONH(n-Pentyl), -CONH(n-Hexyl), -CONH(Cyclohexyl), -CONMe₂, -CONEt₂, -CON(n-Propyl)₂, -CON(Isopropyl)₂, -CON(n-Butyl)₂, -CON(n-Pentyl)₂, -CON(Me)Et, -CON(Me)n-Propyl, -CON(Me)n-Butyl, -CON(Me)n-Pentyl, -CN, Pyrrolidinocarbonyl, Piperidinocarbonyl.
- Q: steht außerdem hervorgehoben für -CO₂H, -CO₂CH₂CN, -CO₂(CH₂)₂CN, -CO₂(CH₂)₃CN, -CO₂CH₂C(CH₃)₂NO₂, -CO₂(CH₂)₂OCH₃, 2-Methoxycarbonyl-propyloxycarbonyl, 3-(2-Oxo-1-pyrrolidinyl)propyloxycarbonyl, N-4-Fluorphenyl-N-isopropyl-amino-2-oxo-ethyloxycarbonyl, -CO₂(CH₂)₃CH=CH₂, -CO₂(CH₂)₂C(CH₃)=CH₂, -CO₂CH₂CH=CH-Ph, 2-Chlorbenzyloxycarbonyl, 3-Chlorbenzyloxycarbonyl, 4-Chlorbenzyloxycarbonyl, 1-Phenylethyloxycarbonyl, 3-Phenylpropyloxycarbonyl, 2-Phenoxyethyloxycarbonyl, 4-Methylcyclohexyloxycarbonyl, Cyclohexylmethyloxycarbonyl, 1-Cyclohexylethyloxycarbonyl, 3-Furfuryloxycarbonyl, 2-Thenyloxycarbonyl, 3-Thenyloxycarbonyl, 2-Pyridinylmethyloxycarbonyl, 3-Pyridinylmethyloxycarbonyl, 6-Chlor-3-pyridinylmethyloxycarbonyl, 1-Tetrahydronaphthyloxycarbonyl, 1-Indanyloxycarbonyl, -CON(H)CH₂CF₃, Methoxyethylaminocarbonyl, 4-Trifluormethoxyphenyl-aminocarbonyl, Benzylaminocarbonyl, 2-Chlorbenzyl-aminocarbonyl, 3-Chlorbenzyl-aminocarbonyl, 4-Chlorbenzyl-aminocarbonyl, 3-Methylbenzyl-aminocarbonyl, 4-Methylbenzyl-aminocarbonyl, 2,4-Dichlorbenzyl-aminocarbonyl, 2-Methoxybenzyl-aminocarbonyl, 2,3-Dimethoxybenzyl-aminocarbonyl, 3,5-Dimethylbenzyl-aminocarbonyl, 2,4-Difluorbenzyl-aminocarbonyl, 4-Trifluormethylcyclohexyl-aminocarbonyl, Cyclohexylmethyl-aminocarbonyl, 2-Thenylaminocarbonyl, 2-Tetrahydrofurylmethyl-aminocarbonyl, 2-Thiazolyl-aminocarbonyl, 5-Methoxycarbonyl-2-thiazolyl-aminocarbonyl oder für Morpholinocarbonyl.

Insbesondere bevorzugt sind auch Verbindungen der allgemeinen Formel (I-c) 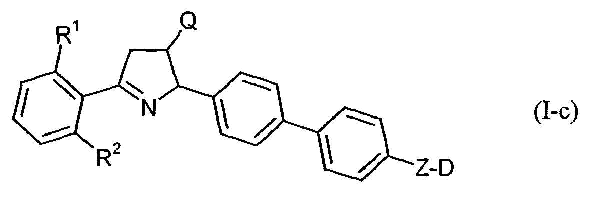 in welcher
- R¹, R², Z, D und Q: die oben angegebenen Bedeutungen haben.

Insbesondere bevorzugt sind auch Verbindungen der allgemeinen Formeln (I-d), (I-e), (I-f), (I-g), (I-h) und (I-i) 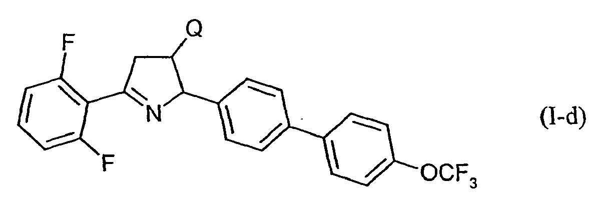 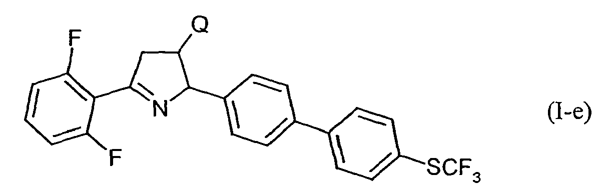 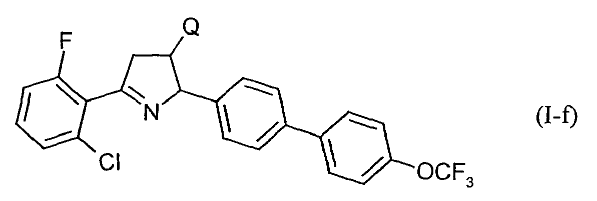 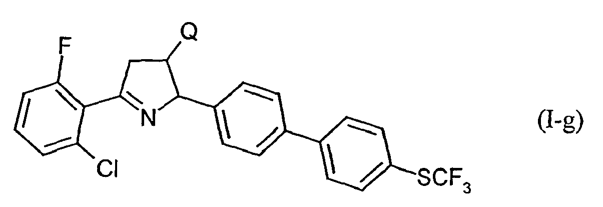 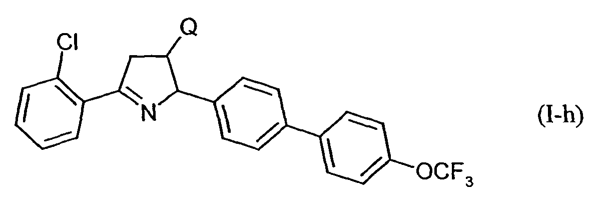 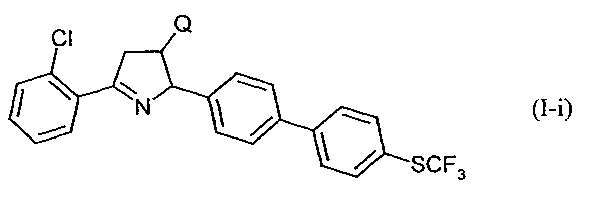 in denen
- Q: für -CO₂R⁹, -CONHR⁸ oder -CONR⁷R⁸ steht,
wobei R⁷, R⁸ und R⁹ die oben angegebenen Bedeutungen haben.

Insbesondere bevorzugt sind auch Verbindungen der allgemeinen Formeln (I-d), (I-e), (I-f), (I-g), (I-h) und (I-i)
in welchen
- Q: für CO₂R¹³ oder -CONR¹⁴R¹⁵ steht,
wobei R¹³, R¹⁴ und R¹⁵ die oben angegebenen Bedeutungen haben.

Insbesondere bevorzugt sind auch Verbindungen der allgemeinen Formeln (I-d), (I-e), (I-f), (I-g), (I-h) und (I-i)
in welchen
- Q: für -CO₂CH₃, -CO₂CH₂CH₃, -CO₂-n-Propyl, -CO₂-Isopropyl, -CO₂-n-Butyl, -CO₂-Isobutyl, -CO₂-sec-Butyl, -CO₂-tert-Butyl, -CO₂-n-Pentyl, -CO₂-Neopentyl, -CO₂-sec-Isoamyl, -CO₂-Pentan-3-yl, -CO₂-Cyclopentyl, -CO₂-n-Hexyl, -CO₂-Cyclohexyl, -CONHCH₃, -CONHCH₂CH₃, -CONH(n-Propyl), -CONH(Isopropyl), -CONH(n-Butyl), -CONH(tert-Butyl), -CONH(n-Pentyl), -CONH(n-Hexyl), -CONH(Cyclohexyl), -CONMe₂, -CONEt₂, -CON(n-Propyl)₂, -CON(Isopropyl)₂, -CON(n-Butyl)₂, -CON(n-Pentyl)₂, -CON(Me)Et, -CON(Me)n-Propyl, -CON(Me)n-Butyl, -CON(Me)n-Pentyl, Pyrrolidinocarbonyl, Piperidinocarbonyl steht.

Insbesondere bevorzugt sind auch Verbindungen der allgemeinen Formeln (I-d), (I-e), (I-f), (I-g), (I-h) und (I-i)
in welchen
- Q: für -CO₂CH₂CN, -CO₂(CH₂)₂CN, -CO₂(CH₂)₃CN, -CO₂CH₂C(CH₃)₂NO₂, -CO₂(CH₂)₂OCH₃, -CO₂H, 2-Methoxycarbonyl-propyloxycarbonyl, 3-(2-Oxo-1-pyrrolidinyl)propyloxycarbonyl, N-4-Fluorphenyl-N-isopropyl-amino-2-oxo-ethyloxycarbonyl, -CO₂(CH₂)₃CH=CH₂, -CO₂(CH₂)₂C(CH₃)=CH₂, -CO₂CH₂CH=CH-Ph, 2-Chlorbenzyloxycarbonyl, 3-Chlorbenzyloxycarbonyl, 4-Chlorbenzyloxycarbonyl, 1-Phenylethyloxycarbonyl, 3-Phenylpropyloxycarbonyl, 2-Phenoxyethyloxycarbonyl, 4-Methylcyclohexyloxycarbonyl, Cyclohexylmethyloxycarbonyl, 1-Cyclohexylethyloxycarbonyl, 3-Furfuryloxycarbonyl, 2-Thenyloxycarbonyl, 3-Thenyloxycarbonyl, 2-Pyridinylmethyloxycarbonyl, 3-Pyridinylmethyloxycarbonyl, 6-Chlor-3-pyridinylmethyloxycarbonyl, 1-Tetrahydronaphthyloxycarbonyl, 1-Indanyloxycarbonyl, -CON(H)CH₂CF₃, Methoxyethylaminocarbonyl, 4-Trifluormethoxyphenyl-aminocarbonyl, Benzyl-aminocarbonyl, 2-Chlorbenzyl-aminocarbonyl, 3-Chlorbenzyl-aminocarbonyl, 4-Chlorbenzyl-aminocarbonyl, 3-Methylbenzyl-aminocarbonyl, 4-Methylbenzyl-aminocarbonyl, 2,4-Dichlorbenzylaminocarbonyl, 2-Methoxybenzyl-aminocarbonyl, 2,3-Dimethoxybenzylaminocarbonyl, 3,5-Dimethylbenzyl-aminocarbonyl, 2,4-Difluorbenzyl-aminocarbonyl, 4-Trifluormethylcyclohexyl-aminocarbonyl, Cyclohexylmethylaminocarbonyl, 2-Thenyl-aminocarbonyl, 2-Tetrahydrofurylmethyl-aminocarbonyl, 2-Thiazolyl-aminocarbonyl, 5-Methoxycarbonyl-2-thiazolyl-aminocarbonyl oder für Morpholinocarbonyl steht.

Eine weitere bevorzugte Gruppe sind die Verbindungen der Formel (I), in welchen
- Q: für -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, einen 5- bis 7-gliedrigen gesättigten oder ungesättigten Heterocyclus mit 2 bis 4 Heteroatomen aus der Reihe Stickstoff, Sauerstoff und Schwefel steht, und
- Ar¹, Ar², R⁶, R⁷, R⁸, R⁹, R¹⁰ und R¹¹: die oben angegebenen allgemeinen Bedeutungen haben.

Eine weitere bevorzugte Gruppe sind die Verbindungen der Formel (I), in welchen
- Q: für -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, einen 5- bis 7-gliedrigen gesättigten oder ungesättigten Heterocyclus mit 2 bis 4 Heteroatomen aus der Reihe Stickstoff, Sauerstoff und Schwefel, steht und
- Ar¹, Ar², R⁶, R⁷, R⁸, R⁹, R¹⁰ und R¹¹: die oben angegebenen bevorzugten Bedeutungen haben.

Eine weitere bevorzugte Gruppe sind die Verbindungen der Formel (I), in welchen
- Q: für -CO₂R⁹, -COR¹⁰, -CONR⁷R⁸, -CN, -PO(OR¹¹)₂, einen 5- bis 7-gliedrigen gesättigten oder ungesättigten Heterocyclus mit 2- bis 4 Heteroatomen aus der Reihe Stickstoff, Sauerstoff und Schwefel (insbesondere Dihydrodioxacin, Oxazolin, Thiazolin, Imidazolin, Tetrazol) steht und
- Ar¹, Ar², R⁶, R⁷, R⁸, R⁹, R¹⁰ und R¹¹: die oben angegebenen besonders bevorzugten Bedeutungen haben.

Eine weitere bevorzugte Gruppe sind die Verbindungen der Formel (I), in welchen
- Q: für -CO₂CH₃, -CO₂CH₂CH₃, -CO₂-n-Propyl, -CO₂-Isopropyl, -CO₂-n-Butyl, -CO₂-Isobutyl, -CO₂-sec-Butyl, -CO₂-tert-Butyl, -CO₂-n-Pentyl, -CO₂-Neopentyl, -CO₂-sec-Isoamyl, -CO₇-Pentan-3-yl, -CO₂-Cyclopentyl, -CO₂-n-Hexyl, -CO₂-Cyclohexyl, -CO₂-Trifluorethyl, -CO₂CH₂Ph, -CO₂Ph, -COCH₃, -COCH₂CH₃, -CO-n-Propyl, -CONHMe, -CONHEt, -CONH(n-Propyl), -CONH(Isopropyl), -CONH(n-Butyl), -CONH(tert-Butyl), -CONH(n-Pentyl), -CONH(n-Hexyl), -CONH(Cyclohexyl), -CONMe₂, -CONEt₂, -CON(n-Propyl)₂, -CON(Isopropyl)₂, -CON(n-Butyl)₂, -CON(n-Pentyl)₂, -CON(Me)Et, -CON(Me)n-Propyl, -CON(Me)n-Butyl, -CON(Me)n-Pentyl, -CN, -PO(OMe)₂, Pyrrolidinocarbonyl, Piperidinocarbonyl, -PO(OEt)₂, -PO(OPh)₂, Dihydrodioxacinyl, Oxazolyl, Thiazolyl, Imidazolyl, Tetrazolyl steht und
- Ar¹, Ar², R⁶, R⁷, R⁸, R⁹, R¹⁰ und R¹¹: die oben angegebenen ganz besonders bevorzugten Bedeutungen haben.

Die allgemeine Formel (I) besitzt zwei, hier durch * gekennzeichnete, Asymmetriezentren.

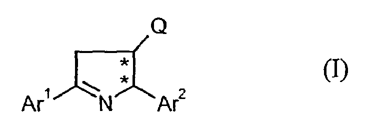

Bevorzugt sind Verbindungen der allgemeinen Formel (I), in denen die Substituenten Q und Ar² an den beiden chiralen Zentren cis-ständig sind.

In den oben genannten Definitionen stehen Oxyalkylen bzw. Thioalkylen für -O-Alkyl- bzw. -S-Alkyl-, wobei die Bindung z.B. an Ar² über das Sauerstoff- bzw. Schwefelatom erfolgt und am Alkylrest gegebenenfalls weitere Substituenten wie z.B. A in -X-A gebunden sind. Alkylenoxy bzw. Alkylenthio stehen für -Alkyl-O-bzw. -Alkyl-S-, wobei die Bindung z.B. an Ar² jeweils über den Alkylrest erfolgt und am Sauerstoff- bzw. Schwefelatom gegebenenfalls weitere Substituenten wie z.B. A in -X-A gebunden sind. Oxyalkylenoxy steht für -O-Alkyl-O.

Heterocyclyl steht in der vorliegenden Beschreibung für einen gesättigten oder ungesättigten cyclischen Kohlenwasserstoff in welchem einer oder mehrere Kohlenstoffatome durch ein oder mehrere Heteroatome ausgetauscht sind. Heteroatome steht dabei bevorzugt für O, S, N, P, insbesondere für O, S und N.

Bevorzugt, besonders bevorzugt oder ganz besonders bevorzugt sind Verbindungen, welche die unter bevorzugt, besonders bevorzugt oder ganz besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Mehrere Reste mit denselben Indizes wie beispielsweise m Reste R⁵ für m >1, können gleich oder verschieden sein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Verwendet man N-(4-Brombenzyl)-2-chlorphenyl-carboximidoyl-chlorid und Acrylsäurenitril als Ausgangsstoffe, so kann der Verlauf des erfindungsgemäßen Verfahrens (A-1) durch das folgende Formelschema veranschaulicht werden.

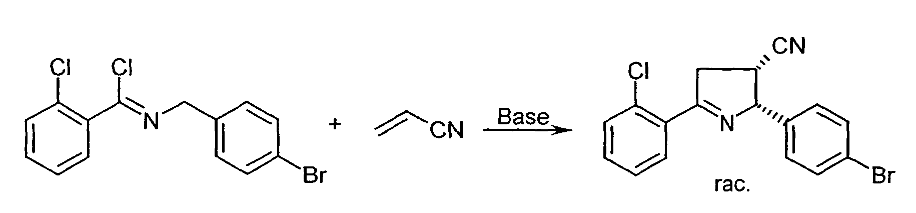

Verwendet man N-(2-chlorbenzyl)-4-bromphenyl-carboximidoyl-chlorid und Acrylsäurenitril als Ausgangsstoffe, so kann der Verlauf des erfindungsgemäßen Verfahrens (A-2) durch das folgende Formelschema veranschaulicht werden.

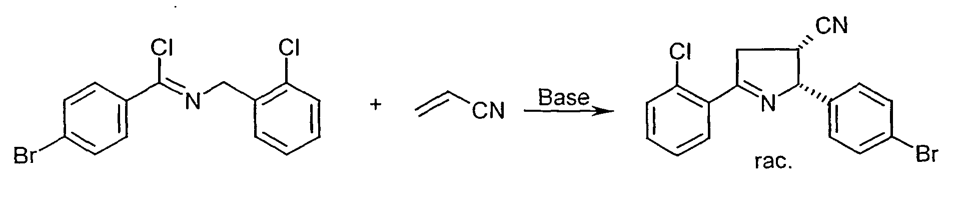

Verwendet man 2-Chlor-N-[(4'-isopropyl-1,1'-biphenyl-4-yl)methyl]benzolcarboximidoyl-chlorid und Acrylnitril als Ausgangsstoffe, so kann der Verlauf des erfindungsgemäßen Verfahrens (A-1) durch das folgende Formelschema veranschaulicht werden.

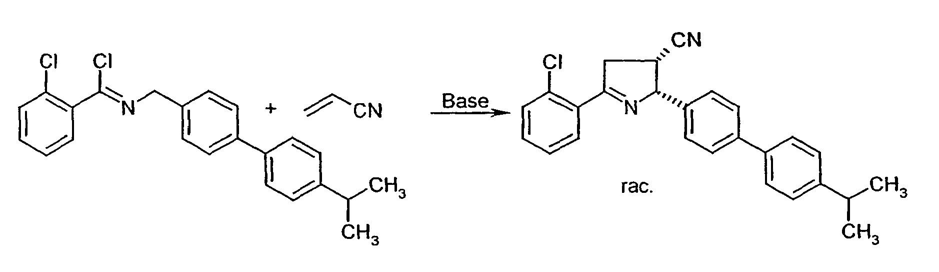

Verwendet man 2-(2'-Chlorbenzyl)-4-[4'-(trifluormethoxy)-1,1'-biphenyl]oxazolidin-5-on und Acrylsäuremethylester als Ausgangsstoffe, so kann der Verlauf des erfindungsgemäßen Verfahrens (B) durch das folgende Formelschema veranschaulicht werden.

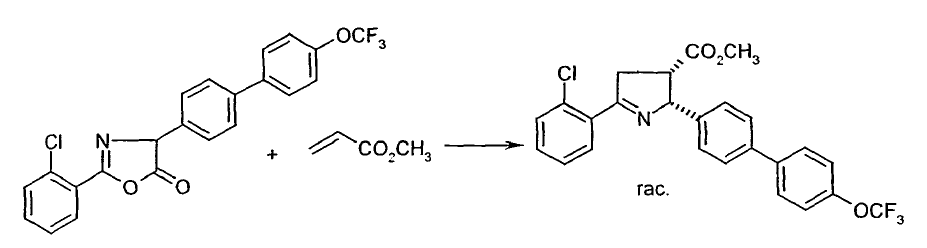

Verwendet man N-(2-Chlorbenzoyl)-2-[(4'-trifluormethoxy)biphenyl]glycin und Essigsäureanhydrid und Acrylsäureethylester als Ausgangsstoffe, so kann der Verlauf des erfindungsgemäßen Verfahrens (C) durch das folgende Formelschema veranschaulicht werden. 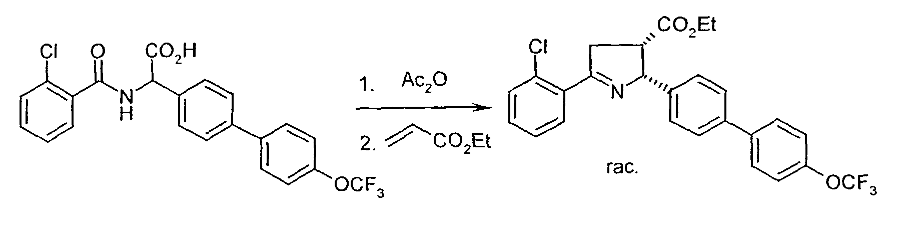 Verwendet man 2-(4-Bromphenyl)-5-(2,6-difluorphenyl)-3,4-dihydro-2H-pyrrole-3-carbonsäureethylester und 4-Trifluormethoxy-phenyl-boronsäure als Ausgangsstoffe, so kann der Verlauf des erfindungsgemäßen Verfahrens (D) durch das folgende Formelschema veranschaulicht werden.

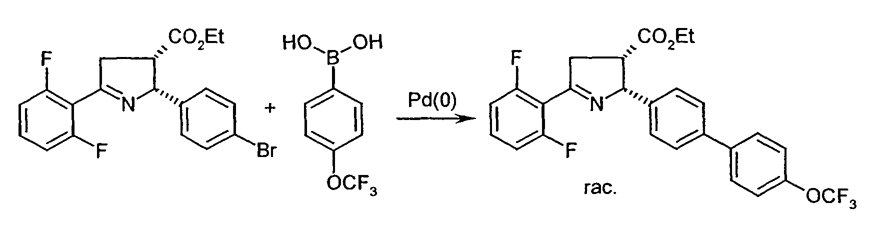

Erläuterungen der Verfahren und Zwischenprodukte:

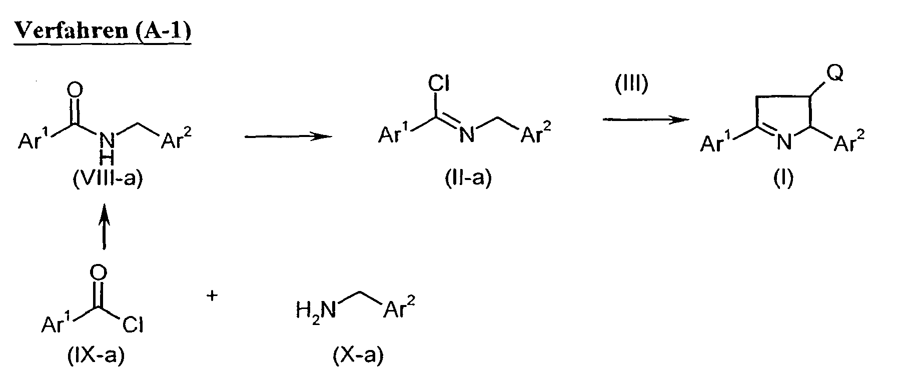

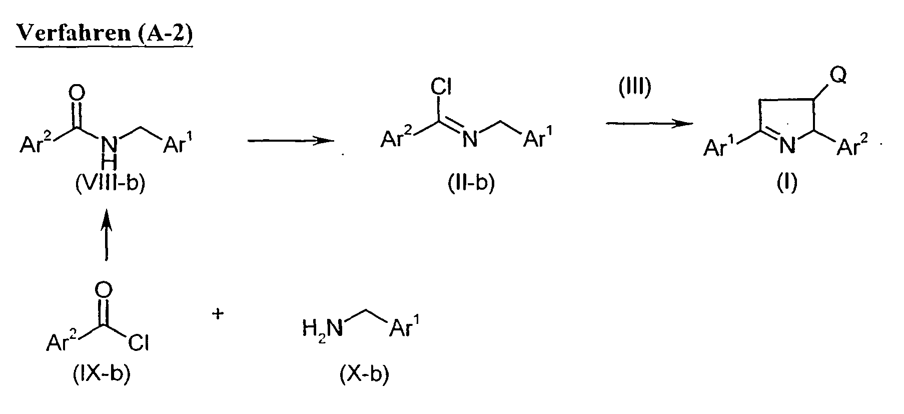

Die bei der Durchführung der erfindungsgemäßen Verfahren (A-1) und (A-2) als Ausgangsstoffe benötigten Iminochloride sind durch die Formeln (II-a) und (II-b) allgemein definiert. In diesen Formeln stehen Ar¹ und Ar² bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Die zur Durchführung der erfindungsgemäßen Verfahren (A-1) und (A-2) als Ausgangsstoffe benötigten Iminochloride der Formeln (II-a) und (II-b) lassen sich herstellen, indem man Amide der Formeln (VIII-a) und (VIII-b) mit einem Chlorierungsreagenz (z.B. SOCl₂) in Gegenwart eines Verdünnungsmittels umsetzt. (Für weitere Methoden siehe "The Chemistry of the Carbon-Nitrogen Double Bond", in Patai, Interscience, New York, 1970, S. 597-662).

Die bei der Durchführung der erfindungsgemäßen Verfahren (A-1) und (A-2) als Ausgangsstoffe benötigten Amide sind durch die Formeln (VIII-a) und (VIII-b) allgemein definiert. In diesen Formeln stehen Ar¹ und Ar² bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Amide der Formeln (VIII-a) und (VIII-b) lassen sich z.B. herstellen, indem man Säurechloride der Formeln (IX-a) und (IX-b) mit Benzylaminen der Formeln (X-a) und (X-b) in Gegenwart einer Base (z.B. Et₃N oder NaOH) und in Gegenwart eines Verdünnungsmittels umsetzt.

Die bei der Durchführung der erfindungsgemäßen Verfahren (A-1) und (A-2) als Ausgangsstoffe benötigten Säurechloride und Benzylamine sind durch die Formeln (IX-a) und (IX-b) bzw. durch die Formeln (X-a) und (X-b) allgemein definiert. In diesen Formeln stehen Ar¹ und Ar² bevorzugt, besonders bevorzugt bzw. ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Säurechloride der Formeln (IX-a) und (IX-b) sind allgemein bekannt. Benzylamine der Formeln (X-a) und (X-b) sind zum Teil bekannt, können aber aus den entsprechenden Nitrilen bzw. Aldehyden über bekannte Methoden (Reduktion bzw. reduktive Aminierung) hergestellt werden (s. z.B. Vogel's Textbook Of Practical Organic Chemistry, Fifth Edition 1989, John Wiley and Sons, New York, Author: Vogel, Arthur, Israel; Revised by Furniss, Hannaford, Smith, and Tatchell, ISBN 0-582-46236-3).

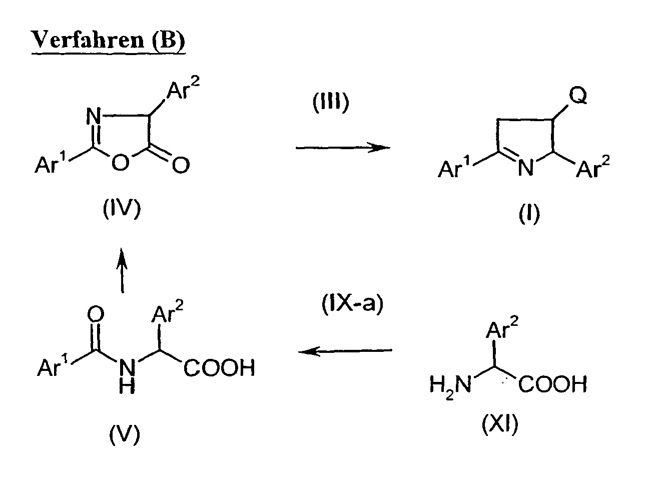

Die bei der Durchführung des erfindungsgemäßen Verfahrens (B) als Ausgangsstoffe benötigten Oxazolidinone sind durch die Formel (IV) allgemein definiert. In dieser Formel stehen Ar¹ und Ar² bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Die zur Durchführung des erfindungsgemäßen Verfahrens (B) als Ausgangsstoffe benötigten Oxazolidinone der Formel (IV) lassen sich analog zu den in der Literatur bekannten Beispielen aus N-Benzoyl-aminosäuren der Formel (V) herstellen. Oxazolidinone der Formel (IV) werden anschließend mit Dipolarophilen der Formel (III) umgesetzt (vgl. z.B. Chem. Ber. 1970, **103**, 2368-2387, und dort zitierte Literatur).

Die bei der Durchführung des erfindungsgemäßen Verfahrens (B) als Ausgangsstoffe benötigten N-Benzoyl-aminosäuren sind durch die Formel (V) allgemein definiert. In dieser Formel stehen Ar¹ und Ar² bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Verbindungen der Formel (V) lassen sich aus Aminosäure-Derivaten der Formel (XI) durch Umsetzung mit Säurechloriden der Formel (IX-a) in Gegenwart einer Base und eines Verdünnungsmittels erhalten. Aminosäuren der Formel (XI) sind zum Teil kommerziell erhältlich und/oder können über bekannte Verfahren hergestellt werden (z.B. Tetrahedron 1994, **50,** 1539-1650 und dort zitierte Literatur).

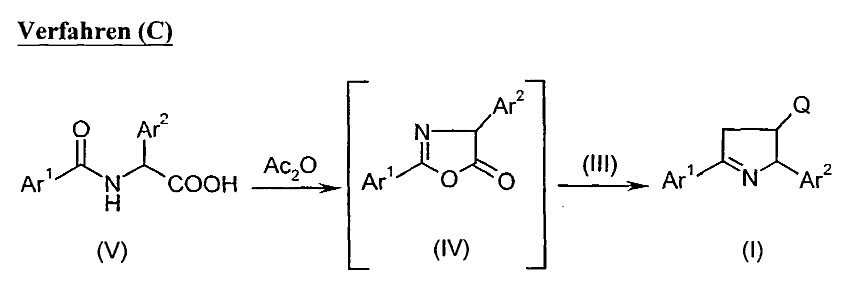

N-Benzoyl-aminosäuren der Formel (V) können zur Herstellung der erfindungsgemäßen Verbindungen der Formel (I) auch in einer Tandem-Reaktion umgesetzt werden. Hierzu werden die Oxazolidinone der Formel (IV) *in situ* aus Verbindungen der Formel (V) und Essigsäureanhydrid (vgl. z.B. Chem. Ber. 1970, **103**, 2365-2387) hergestellt und anschließend gemäß Verfahren (B) mit Dipolarophilen der Formel (III) umgesetzt.

Die bei der Durchführung der erfindungsgemäßen Verfahren (A-1), (A-2), (B) und (C) als Ausgangsstoffe benötigten Dipolarophile sind durch die Formel (III) allgemein definiert. In dieser Formel steht Q bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Verbindungen der Formel (III) (Q = Ester, Nitril, Keton, Amid, Phosphorester, Sulfon) sind allgemein bekannt bzw. kommerziell erhältlich.

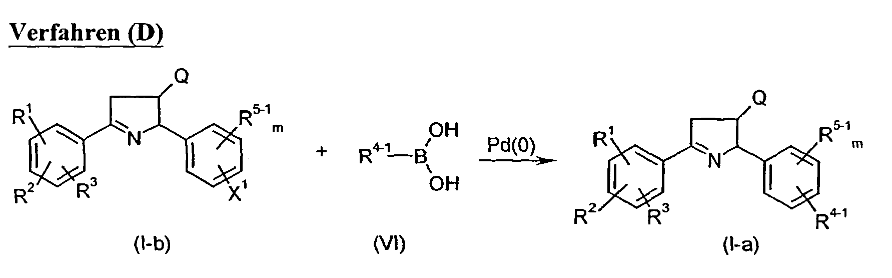

Für den Fall, dass Ar² im erfindungsgemäßen Wirkstoff der Formel (I) für ein gegebenenfalls substituiertes Biphenyl steht, lassen sich Verbindungen der Formel (I-a) herstellen, indem man Verbindungen der Formel (I-b) mit Boronsäuren der Formel (VI) in Gegenwart eines Palladium-Katalysators und in Gegenwart einer Base und in Gegenwart eines Verdünnungsmittel kuppelt.

In Verbindungen der Formeln (I-a) bzw. (I-b) stehen R¹, R², R³ und m bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.
- R⁵⁻¹: steht bevorzugt für Fluor, Hydroxy, Cyano, Nitro, C₁-C₆-Alkyl, C₁-C₆-Alkoxy, C₁-C₆-Halogenalkyl, C₁-C₆-Halogenalkoxy, Tri(C₁-C₆-alkyl)-silyl, C₁-C₆-Alkoxycarbonyl, -CONR⁷R⁸, -OSO₂NR⁷R⁸, -S(O)ₒR⁶, -NR⁷R⁸, -NHCO₂R⁶, (C₁-C₆-Halogenalkyl)aminosulfonyl.
- R⁵⁻¹: steht besonders bevorzugt für Fluor, Hydroxy, Cyano, Nitro, C₁-C₆-Alkyl, C₁-C₆-Alkoxy, jeweils durch Fluor oder Chlor substituiertes C₁-C₆-Alkyl oder C₁-C₆-Alkoxy, -CONR⁷R⁸, -OSO₂NR⁷R⁸, -S(O)ₒR⁶, -NR⁷R⁸, -NHCO₂R⁶.
- R⁵⁻¹: steht ganz besonders bevorzugt für Fluor, Hydroxy, Methyl, Ethyl, Methoxy, Ethoxy, Trifluormethyl, Difluormethoxy, Trifluormethoxy, -OSO₂NMe₂ oder -S0₂CF₃.

In Verbindungen der Formel (I-b) steht X¹ bevorzugt für Chlor, Brom oder Iod, besonders bevorzugt für Chlor oder Brom, ganz besonders bevorzugt für Brom.

In Verbindungen der Formeln (I-a) und (VI) steht R⁴⁻¹ für A oder -B-Z-D. A, B, Z und D stehen dabei bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Verbindungen der Formel (I-b) werden über Verfahren A, B oder C hergestellt. Boronsäuren der Formel (VI) sind z. Teil bekannt, können aber auch ausgehend vom (Brom)aromaten durch Lithiierung bzw. Br-Li (Mg)-austausch und anschließende Umsetzung mit Trisalkoxyborverbindungen hergestellt werden (vgl. z.B. Tetrahedron Lett. 1993, **34**, 8237-8240).

Weiterhin können Verbindungen der Formel (I-a) ausgehend von Verbindungen der Formel (I-b) [X¹ = 2-(4,4,5,5-Tetramethyl-1,3,2-dioxoborolan] und (Hetero)cyclen der Formel (VII) analog Literatur bekannten Methoden (J.Org. Chem. 1995, **60**, 7508; Tetrahedron Lett. 1997, 38, 3841) hergestellt werden.

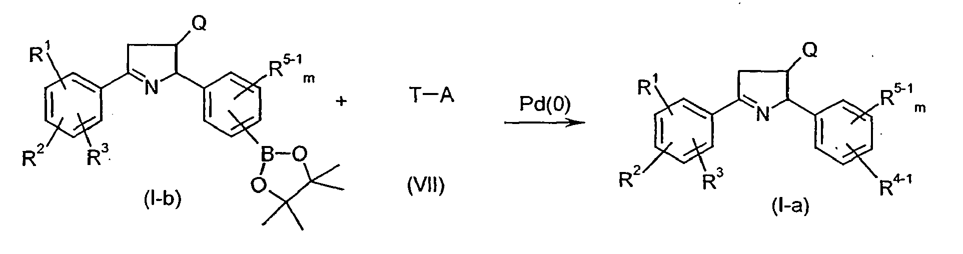

In den (Hetero)cyclen der Formel (VII) steht T bevorzugt für Chlor, Brom oder Iod, besonders bevorzugt für Chlor oder Brom, ganz besonders bevorzugt für Chlor. A steht bevorzugt, besonders bevorzugt etc. für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden. Verbindungen der Formel (VII) sind bekannt oder können nach bekannten Verfahren hergestellt werden.

Bei der Durchführung des erfindungsgemäßen Verfahrens (D) setzt man im allgemeinen einen Palladium-Katalysator ein, der wiederum mit oder ohne Zusatz von weiteren Liganden verwendet werden kann. Vorzugsweise verwendet man als Katalysator PdCl₂(dppf) [dppf = 1,1'-Bis(diphenylphosphino)ferrocene], Pd(PPh₃)₄, PdCl₂(PPh₃)₂, PdCl₂(CH₃CN)₂, Pd₂(dba)₃ [dba = Dibenzylidenaceton] oder Pd(OAc)₂, besonders bevorzugt PdCl₂(dppf), Pd(PPh₃)₄, PdCl₂(PPh₃)₂ oder Pd(OAc)₂, ganz besonders bevorzugt PdCl₂(dppf) oder Pd(PPh₃)₄. Als Liganden kommen Triarylphosphine, Trialkylphosphine oder Arsine in Frage. Vorzugsweise verwendet man dppf, PPh₃, P(t-Bu)₃, Pcy₃ oder AsPh₃, besonders bevorzugt dppf.

Bei der Durchführung der erfindungsgemäßen Verfahren (A-1), (A-2), (B), (C) und (D) erhält man Verbindungen der Formel (I) bzw. (I-a), in welchen Q für -CO₂H steht, indem man z.B. die entsprechenden Methylester nach bekannten Verfahren verseift (vgl. T.W. Greene, P.G.M. Wuts, Protective Groups in Organic Synthesis, 2^{nd} edition, S. 232-234).

Als Säurebindemittel kommen bei der Durchführung der erfindungsgemäßen Verfahren (A-1), (A-2), (B), (C) und (D) jeweils alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert.

Als Verdünnungsmittel kommen bei der Durchführung der erfindungsgemäßen Verfahren (A-1), (A-2), (B), (C) und (D) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan.

Die Reaktionstemperaturen können bei der Durchführung der erfindungsgemäßen Verfahren (A-1), (A-2), (B), (C) und (D) jeweils in einem größeren Bereich variiert werden. Im allgemeinen arbeitet man bei Temperaturen zwischen 0°C und 140°C, vorzugsweise zwischen 10°C und 120°C.

Bei der Durchführung der erfindungsgemäßen Verfahren (A-1), (A-2), (B), (C) und (D) arbeitet man im allgemeinen jeweils unter Atmosphärendruck. Es ist aber auch möglich, jeweils unter erhöhtem oder vermindertem Druck zu arbeiten.

Bei der Durchführung des erfindungsgemäßen Verfahrens (A-1) oder (A-2) setzt man auf 1 Mol an Iminochlorid der Formeln (II-a) oder (II-b) im allgemeinen 2 Mol an Dipolarophil der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch extrahiert, wäscht, trocknet und den Rückstand gegebenenfalls nach üblichen Methoden, wie Chromatographie oder Umkristallisation, von eventuell noch vorhandenen Verunreinigungen befreit.

Bei der Durchführung des erfindungsgemäßen Verfahrens (B) setzt man auf 1 Mol an Oxazolinon der Formel (IV) im allgemeinen 2 Mol an Dipolarophil der Formel (III) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch extrahiert, wäscht, trocknet und den Rückstand gegebenenfalls nach üblichen Methoden, wie Chromatographie oder Umkristallisation, von eventuell noch vorhandenen Verunreinigungen befreit.

Bei der Durchführung des erfindungsgemäßen Verfahrens (C) setzt man auf 1 Mol an Carbonsäure der Formel (V) im allgemeinen 2 Mol an Dipolarophil der Formel (III) sowie 1 bis 3 Mol an Essigsäureanhydrid ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch extrahiert, wäscht, trocknet und den Rückstand gegebenenfalls nach üblichen Methoden, wie Chromatographie oder Umkristallisation, von eventuell noch vorhandenen Verunreinigungen befreit.

Bei der Durchführung des erfindungsgemäßen Verfahrens (D) setzt man auf 1 Mol an Verbindungen der Formeln (I) im allgemeinen 1 Mol an Dipolarophil der Formel (III) oder auch einen Überschuss daran, sowie 1 bis 5 Mol an Säurebindemittel und 1/20 mol Katalysator ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch zwischen Wasser und Methyl-tert-butylether verteilt, die organische Phase über Natriumsulfat trocknet und den Rückstand gegebenenfalls nach üblichen Methoden, wie Chromatographie oder Umkristallisation, von eventuell noch vorhandenen Verunreinigungen befreit.

Die erfindungsgemäßen Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen der Formel (I) zeichnen sich insbesondere durch eine hervorragende Wirkung gegen Raupen, Käferlarven, Spinnmilben, Blattläuse und Minierfliegen aus.

Die erfindungsgemäßen Verbindungen der Formel (I) zeichnen sich insbesondere ganz besonders durch hervorragende Wirkung gegen Larven des Meerettichblattkäfers (Phaedon cochleariae), Raupen des Eulenfalters (Spodoptera frugiperda), Larven der Grünen Reiszikade (Nephotettix cincticeps), Pfirsichblattläuse (Myzus persicae) und alle Stadien der gemeinen Spinnmilbe (Tetranychus urticae) aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der erfindungsgemäßen Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln;
als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate;
als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit anderen, auch bekannten Wirkstoffen, wie Insektiziden, Lockstoffen, Sterilantien, Bakteriziden, Akariziden, Nematiziden, Fungiziden, wachstumsregulierenden Stoffen oder Herbiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Edifenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur, Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol,
Validamycin A, Vinclozolin, Viniconazol,
Zarilamid, Zineb, Ziram sowie
Dagger G, OK-8705, OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol;
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
²-[(1-Methylethyl)-sulfonyl]-5-(trichlormethyl)-1,3,4-thiadiazol,
2-[[6-Deoxy-4-O-(4-O-methyl-β-D-glycopyranosyl)-α-D-glucopyranosyl)-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl)-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-pyridin-3-yl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,
4-[(3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon, Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat
N-Cyanomethyl-4-trifluormethyl-nicotinamid
3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der erfindungsgemäßen Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch über additive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
   Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen die Entwicklungsstadien von Zecken wie zum Beispiel Amblyomma hebraeum, gegen parasitierende Fliegen wie zum Beispiel gegen Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die erfindungsgemäßen Wirkstoffe der Formel (I) als Formulierungen (beispielsweise Pulver, Emulsionen, fließfähige Mittel), die die erfindungsgemäßen Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und - türen, Sperrholz, Spanplatten, Tischlerarbeiten oder Holzprodukte, die ganz allgemein beim Hausbau oder in der Bautischlerei Verwendung finden.

Die erfindungsgemäßen Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der erfindungsgemäßen Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).
Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyfenozide und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb; oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten des weiteren die üblichen Bestandteile wie z.B. in Ungerer, *Chem. Ind*. **1985,** 37, 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die erfindungsgemäßen Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
   Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
   Aus der Ordnung der Araneae z.B. Aviculariidae, Araneidae.
   Aus der Ordnung der Opiliones z.B. Pseudoscorpiones chelifer, Pseudoscorpiones cheiridium, Opiliones phalangium.
   Aus der Ordnung der Isopoda z.B. Oniscus asellus, Porcellio scaber.
   Aus der Ordnung der Diplopoda z.B. Blaniulus guttulatus, Polydesmus spp..
   Aus der Ordnung der Chilopoda z.B. Geophilus spp..
   Aus der Ordnung der Zygentoma z.B. Ctenolepisma spp., Lepisma saccharina, Lepismodes inquilinus.
   Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
   Aus der Ordnung der Saltatoria z.B. Acheta domesticus.
   Aus der Ordnung der Dermaptera z.B. Forficula auricularia.
   Aus der Ordnung der Isoptera z.B. Kalotermes spp., Reticulitermes spp.
   Aus der Ordnung der Psocoptera z.B. Lepinatus spp., Liposcelis spp.
   Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
   Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
   Aus der Ordnung der Lepidoptera z.B. Achroia grisella, Galleria mellonella, Plodia interpunctella, Tinea cloacella, Tinea pellionella, Tineola bisselliella.
   Aus der Ordnung der Siphonaptera z.B. Ctenocephalides canis, Ctenocephalides felis, Pulex irritans, Tunga penetrans, Xenopsylla cheopis.
   Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
   Aus der Ordnung der Anoplura z.B. Pediculus humanus capitis, Pediculus humanus corporis, Phthirus pubis.
   Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die Herstellung und die Verwendung der erfindungsgemäßen Stoffe geht aus den folgenden Beispielen hervor.

### Herstellungsbeispiele

### Beispiel 1

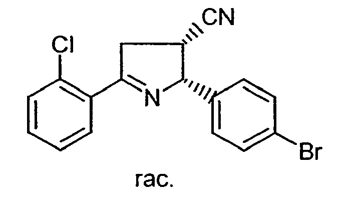

### Synthese 1.Stufe:

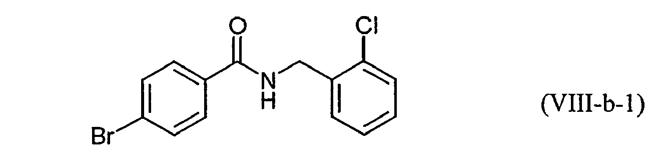

Zu einer Mischung bestehend aus 100.5 g (0.50 mol) 4-Brombenzoesäure, 1 ml Dimethylformamid und 1200 ml Dichlormethan wird unter Rühren eine Lösung von 63.5 g (0.50 mol) Oxalychlorid in 50 ml Dichlormethan zugetropft. Nach 4-stündigem Rühren und nach Beendigung der Gasentwicklung (HCl, CO, CO₂) kühlt man unter Argon auf -60°C und tropft innerhalb von 10 Minuten ein Gemisch aus 70.8 g (0.50 mol) 2-Chlorbenzylamin und 161.0 g (1.25 mol) N,N-Diisopropylethylamin hinzu. Nach Rühren über Nacht wird die so erhaltene gelbe Lösung nacheinander dreimal mit je 250 ml 2N Salzsäure, einmal mit 300 ml Wasser und einmal mit 300 ml gesättigter Natriumchlorid-Lösung gewaschen. Nach Trocknen der organischen Phase über Natriumsulfat und vollständigem Einengen erhält man einen Rückstand, der mit 200 ml Dichlormethan bei Raumtemperatur aufgerührt und anschließend abgesaugt wird. Den so erhaltenen Rückstand nimmt man in 200 ml Methyl-tert-butylether auf, erhitzt kurz zum Sieden, lässt Abkühlen und saugt erneut ab. Man erhält 108 g (83% der Theorie) 4-Brom-N-(2-chlorbenzyl)benzamid vom Schmelzpunkt 133°C.

### Synthese 2. Stufe:

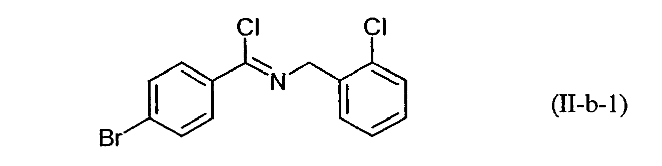

Eine Mischung aus 106.4 g (0.34 mol) der Verbindung (VIII-b-1) und 200 ml Thionylchlorid wird für 2 h zum Rückfluss erhitzt. Nach Abkühlen auf Raumtemperatur entfernt man das überschüssige Thionylchlorid unter vermindertem Druck. Das so erhaltene gelbe Öl kristallisiert nach kurzem Stehen bei Raumtemperatur.
Man erhält 112.5 g (97 % der Theorie) 4-Brom-N-(2-chlorbenzyl)phenylcarboximidoyl-chlorid vom Schmelzpunkt 58 bis 61°C.

### Synthese 3. Stufe:

Eine Mischung aus 55.9 g (163 mmol) der Verbindung (II-b-1), 17.3 g (327 mmol) Acrylsäurenitril und 33.0 g (327 mmol) Triethylamin in 300 ml Dichlormethan wird für 7 d unter Licht- und Luftausschluss bei Raumtemperatur stehen gelassen. Anschließend wäscht man mit dreimal 100 ml 2N Salzsäure, einmal mit 100 ml Wasser und einmal mit 100 ml konzentrierter Natriumchlorid-Lösung. Nach Trocknen über Natriumsulfat wird unter vermindertem Druck vollständig eingeengt und der Rückstand mehrfach an Kieselgel mit Dichlormethan/Cyclohexan chromatographiert.
Man erhält ((2S,3R), (2R,3S))-2-(4-Bromphenyl)-5-(2-chlorphenyl)-3,4-dihydro-2H-pyrrole-3-carbonitril vom Schmelzpunkt 110°C.

### Beispiel 2

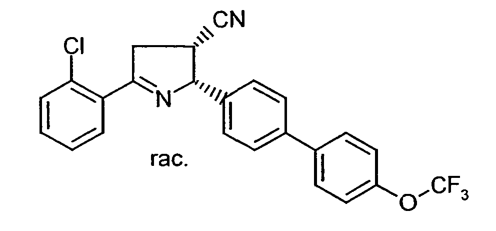

Eine Mischung aus 0.997 g (2.78 mmol) der Verbindung (I-b-1), 0.606 g (2.90 mmol) 4-Trifluormethoxy-phenyl-boronsäure, 0.16 g (0.14 mmol) Tetrakis-triphenylphosphin-Palladium, 20 ml 1,2-Dimethoxyethan und 5.0 ml 20 proz. Natriumcarbonat-Lösung wird für 30 Minuten unter Argon auf 90°C erhitzt. Nach Abkühlen auf Raumtemperatur nimmt man den Ansatz in 30 ml Wasser und 30 ml Methyl-tert-butylether auf. Nach Abtrennen der organischen Phase wird die wässrige Phase erschöpfend mit Methyl-tert-butylether extrahiert. Man trocknet die vereinigten organischen Phasen über Natriumsulfat und engt unter vermindertem Druck vollständig ein. Kristallisation aus Dichlormethan/Methyl-tert-butylether ergibt einen weißen Feststoff.
Man erhält 320 mg (26% d. Th.) ((2S,3R), (2R,3S)-5-(2-Chlorphenyl)-2-[4'-(trifluormethoxy)-1,1'-biphenyl-4-yl]-3,4-dihydro-2H-pyrrol-3-carbonitril vom Schmelzpunkt 132°C.

### Beispiel 3

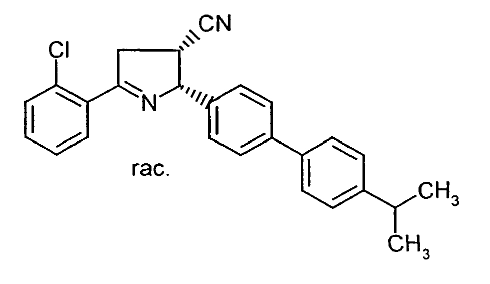

Eine Mischung aus 0.803 g (2.23 mmol) der Verbindung (I-b-1), 0.732 g (4.46 mmol) 4-Isopropyl-phenyl-boronsäure, 0.130 g (0.12 mmol) Tetrakis-triphenylphosphin-Palladium, 15 ml 1,2-Dimethoxyethan und 3.7 ml 20 proz. Natriumcarbonat-Lösung wird für 40 Minuten unter Argon auf 90°C erhitzt. Nach Abkühlen auf Raumtemperatur nimmt man den Ansatz in 30 ml Wasser und 30 ml Methyl-tert-butylether auf. Nach Abtrennen der organischen Phase wird die wässrige Phase erschöpfend mit Methyl-tert-butylether extrahiert. Man trocknet die vereinigten organischen Phasen über Natriumsulfat und engt unter vermindertem Druck vollständig ein. Chromatographie des so erhaltenen Rückstandes an Kieselgel mit Cyclohexan/Essigsäureethylester als Eluens ergibt das gewünschte Produkt.
Man erhält 230 mg (26% d. Th.) ((2S,3R), (2R,3S)-5-(2-Chlorphenyl)-2-[4'-(isopropyl)-1,1'-biphenyl-4-yl]-3,4-dihydro-2H-pyrrol-3-carbonitril vom Schmelzpunkt 127-128°C.

### Beispiel 4

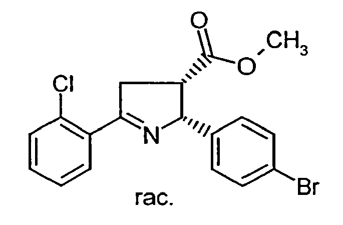

Eine Mischung aus 55.9 g (163 mmol) der Verbindung (II-b-1), 28.1 g (327 mmol) Acrylsäuremethylester und 33.0 g (327 mmol) Triethylamin in 300 ml Dichlormethan wird für 7 d unter Licht- und Luftausschluss bei Raumtemperatur stehen gelassen. Anschließend wäscht man dreimal mit je 100 ml 2N Salzsäure, einmal mit 100 ml Wasser und einmal mit 100 ml Konz. Natriumchlorid-Lösung. Nach Trocknen über Natriumsulfat wird unter vermindertem Druck vollständig eingeengt und der Rückstand mehrfach an Kieselgel mit Dichlormethan/Cyclohexan chromatographiert.
Man erhält ((2S,3R), (2R,3S)-2-(4-Bromphenyl)-5-(2-chlorphenyl)-3,4-dihydro-2H-pyrrol-3-carbonsäuremethylester vom Schmelzpunkt 134°C.

### Beispiel 5

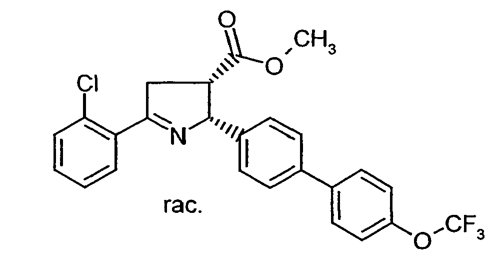

Eine Mischung aus 1.09 g (2.78 mmol) der Verbindung (I-b-2), 0.606 g (2.90 mmol) 4-Trifluormethoxy-phenyl-boronsäure, 0.16 g (0.14 mmol) Tetrakis-triphenylphosphin-Palladium, 20 ml 1,2-Dimethoxyethan und 5.0 ml 20 prozentige Natriumcarbonat-Lösung wird für 30 Minuten unter Argon auf 90°C erhitzt. Nach Abkühlen auf Raumtemperatur nimmt man den Ansatz in 30 ml Wasser und 30 ml Methyl-tert-butylether auf. Nach Abtrennen der organischen Phase wird die wässrige Phase erschöpfend mit Methyl-tert-butylether extrahiert. Man trocknet die vereinigten organischen Phasen über Natriumsulfat und engt unter vermindertem Druck vollständig ein. Der Rückstand wird an Kieselgel mit Cyclohexan/Essigsäureethylester als Eluens chromatographiert.
Man erhält 620 mg (47% d. Th.) ((2S, 3R), (2R, 3S))-5-(2-Chlorphenyl)-2-[4'-(trifluormethoxy)-1,1'-biphenyl-4-yl]-3,4-dihydro-2H-pyrrol-3-carbonsäuremethylester als gelbliches Öl.
log P (pH 2.3) = 5.05.

### Beispiel 6

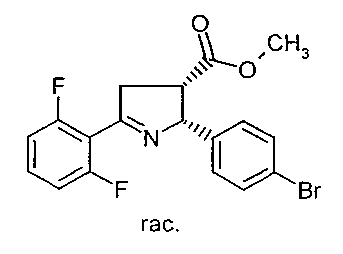

### Synthese 1. Stufe:

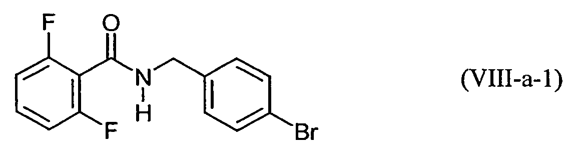

Zu einer Lösung von 73.3 g (464 mmol) 2,6-Difluorbenzoesäure in 1000 ml Dichlormethan und 2 ml Dimethylformamid wird eine Lösung von 64.8 g (510 mmol) Oxalylchlorid in 200 ml Dichlormethan bei 20°C unter Argon zugetropft (1 h, Innentemperatur fällt ohne Argonstrom auf 15°C). Nach Ende der starken Gasentwicklung (CO₂, CO, HCl) wird auf 35°C erwärmt und 5 min nachgerührt. Anschließend gibt man 103.2 g (464 mmol) 4-Brombenzylaminhydrochlorid zu und tropft eine Lösung von 140.5 g (1391 mmol) Triethylamin in 100 ml Dichlormethan bei 0°C zu der Lösung des Säurechlorids. Nach Beendigung der Reaktion gibt man 200 ml Dichlormethan zu. Dann wird zweimal mit je 500 ml 2N HCl, zweimal mit je 250 ml 2 N NaOH ausgeschüttelt, anschließend neutral gewaschen und einmal mit 100 ml konzentrierter Natriumchloridlösung extrahiert. Nach Trocknen über Natriumsulfat wird filtriert und das Filtrat unter vermindertem Druck vollständig eingeengt. Die gewünschte Verbindung kristallisiert aus Dichlormethan/n-Hexan.
Man erhält 125.7 g (83 % d. Th.) N-(4-Brombenzyl)-2,6-difluorbenzamid vom Schmelzpunkt 142°C.

### Synthese 2. Stufe:

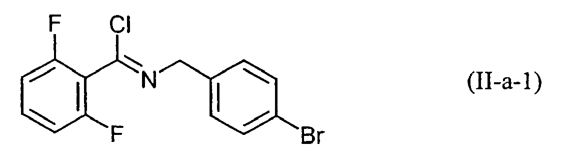

25 g (76.7 mmol) der Verbindung (VIII-a-1) wird in 60 ml Thionylchlorid für 2 h unter Rückfluss erhitzt. Anschließend engt man unter vermindertem Druck vollständig ein.

Der so erhaltene Rückstand wird ohne weitere Reinigung für die Folgereaktionen eingesetzt.

### Synthese 3. Stufe:

Eine Mischung von 25.4 g (73.7 mmol) der Verbindung (II-a-1), 25.3 g Acrylsäuremethylester (294 mmol) und 29.8 g (295 mmol) Triethylamin wird für 14 d unter Licht- und Luftausschluss stehen gelassen.
Zur Aufarbeitung nimmt man in 500 ml Dichlormethan auf und extrahiert die organische Phase dreimal mit je 500 ml 2N Salzsäure. Nach Trocknen über Natriumsulfat wird unter vermindertem Druck eingeengt und der so erhaltene Rückstand mehrfach an Kieselgel mit Dichlormethan/Methanol als Laufmittel chromatographiert.
Man erhält ((2S,3R), (2R,3S))-2-(4-Bromphenyl)-5-(2,6-difluorphenyl)-3,4-dihydro-2H-pyrrol-3-carbonsäuremethylester vom Schmelzpunkt 133°C.

### Beispiel 7

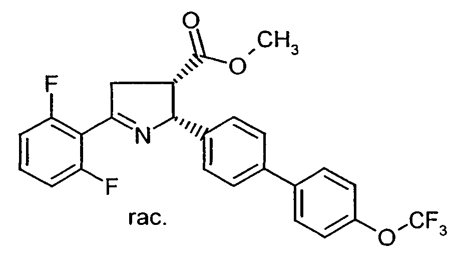

Eine Mischung aus 1.10 g (2.78 mmol) der Verbindung (I-b-3), 0.606 g (2.90 mmol) 4-Trifluormethoxy-phenyl-boronsäure, 0.16 g (0.14 mmol) Tetrakis-triphenylphosphin-Palladium, 20 ml 1,2-Dimethoxyethan und 5.0 ml 20 proz. NatriumcarbonatLösung wird für 30 Minuten unter Argon auf 90°C erhitzt. Nach Abkühlen auf Raumtemperatur nimmt man den Ansatz in 30 ml Wasser und 30 ml Methyl-tert-butylether auf. Nach Abtrennen der organischen Phase wird die wässrige Phase erschöpfend mit Methyl-tert-butylether extrahiert. Man trocknet die vereinigten organischen Phasen über Natriumsulfat und engt unter vermindertem Druck vollständig ein. Kristallisation aus Dichlormethan/Methyl-tert-butylether ergibt einen weißen Feststoff.
Man erhält 480 mg (39% d. Th.) ((2S,3R), (2R,3S)-5-(2,6-Difluorphenyl)-2-[4'-(trifluormethoxy)-1,1'-biphenyl-4-yl]-3,4-dihydro-2H-pyrrol-3-carbonsäuremethylester vom Schmelzpunkt 125°C.

### Beispiel 8

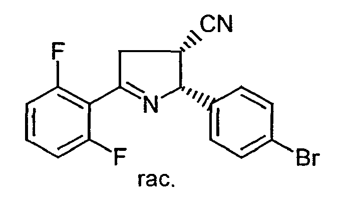

Eine Mischung aus 25.4 g (76.6 mmol) der Verbindung (II-a-1), 16.2 g (303 mmol) Acrylsäurenitril und 30.7 g (303 mmol) Triethylamin in 300 ml Dichlormethan wird für 7 d unter Licht- und Luftausschluss bei Raumtemperatur stehen gelassen. Anschließend wäscht man dreimal mit je 100 ml 2N Salzsäure, einmal mit 100 ml Wasser und einmal mit 100 ml konzentrierter Natriumchlorid-Lösung. Nach Trocknen über Natriumsulfat wird unter vermindertem Druck vollständig eingeengt und der Rückstand mehrfach an Kieselgel mit Dichlormethan/Cyclohexan als Eluens chromatographiert.
Man erhält ((2S,3R), (2R,3S))-2-(4-Bromphenyl)-5-(2,6-difluorphenyl)-3,4-dihydro-2H-pyrrol-3-carbonitril vom Schmelzpunkt 159°C.

### Beispiel 9

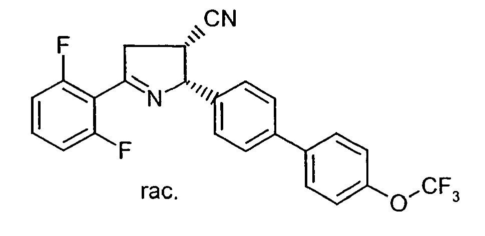

Eine Mischung aus 1.00 g (2.78 mmol) der Verbindung (I-b-4), 0.606 g (2.90 mmol) 4-Trifluormethoxy-phenyl-boronsäure, 0.16 g (0.14 mmol) Tetrakis-triphenylphosphin-Palladium, 20 ml 1,2-Dimethoxyethan und 5.0 ml 20 proz. NatriumcarbonatLösung wird für 30 Minuten unter Argon auf 90°C erhitzt. Nach Abkühlen auf Raumtemperatur nimmt man den Ansatz in 30 ml Wasser und 30 ml Methyl-tert-butylether auf. Nach Abtrennen der organischen Phase wird die wässrige Phase erschöpfend mit Methyl-tert-butylether extrahiert. Man trocknet die vereinigten organischen Phasen über Natriumsulfat und engt unter vermindertem Druck vollständig ein. Kristallisation aus Dichlormethan/Methyl-tert-butylether ergibt einen hellgelben Feststoff.
Man erhält 520 mg (42% d. Th.) ((2S,3R), (2R,3S)-5-(2,6-Difluorphenyl)-2-[4'-(trifluormethoxy)-1,1'-biphenyl-4-yl]-3,4-dihydro-2H-pyrrol-3-carbonitril vom Schmelzpunkt: 145°C.

Die in der folgenden Tabelle aufgeführten Verbindungen können entsprechend einem der oben beschriebenen erfindungsgemäßen Verfahren hergestellt werden.

| Nr. | Struktur | LogP | Fp/°C |
|---|---|---|---|
| 10 | 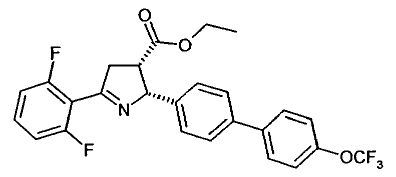 | 4.73^{a)} | |
| 11 | 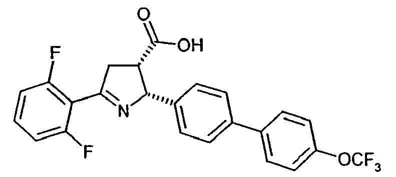 | 3.35^{a)} | |
| 12 | 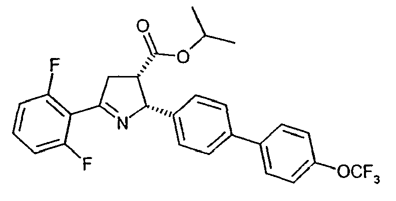 | 5,05^{a)} | |
| 13 | 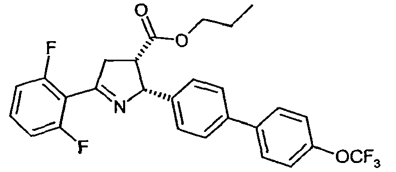 | | |
| 14 | 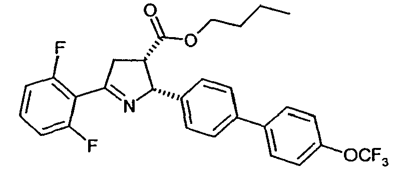 | 5,41^{a)} | |
| | | 5,49^{b)} | |
| 15 | 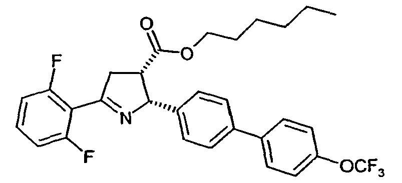 | 6,11^{a)} | |
| | | 6,18^{b)} | |
| 16 | 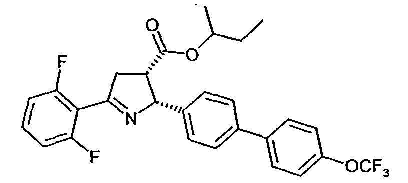 | 5,40^{a)} | |
| | | 5,48^{b)} | |
| 17 | 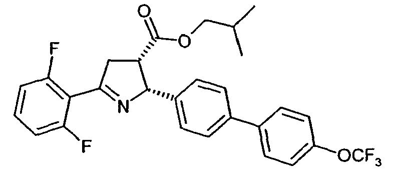 | 5,38^{a)} | |
| | | 5,47^{b)} | |
| 18 | 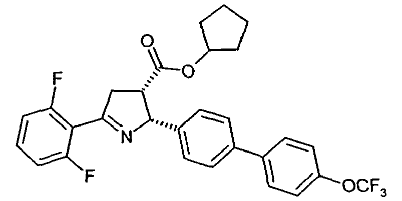 | 5,47^{a)} | |
| | | 5,54^{b)} | |
| 19 | 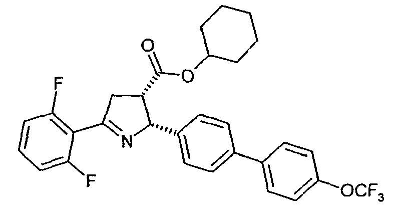 | 5,85^{a)} | |
| | | 5,91^{b)} | |
| 20 | 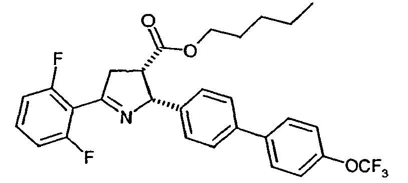 | 5,84^{a)} | |
| 21 | 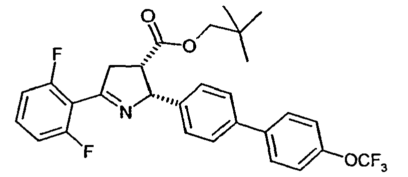 | 5,78^{a)} | |
| 22 | 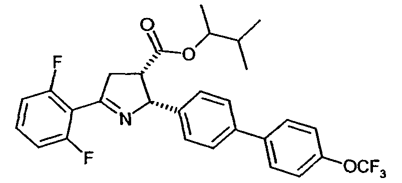 | 5,71^{a)} | |
| 23 | 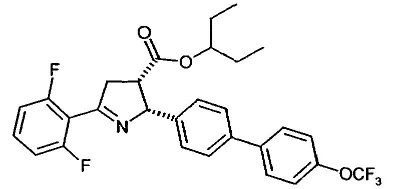 | 5,78^{a)} | |
| 24 | 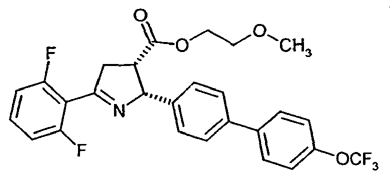 | 4,37^{a)} | |
| 25 | 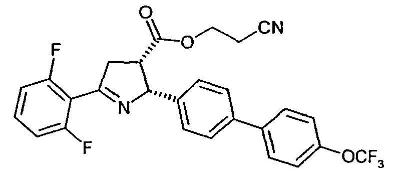 | 4,08^{a)} | |
| 26 | 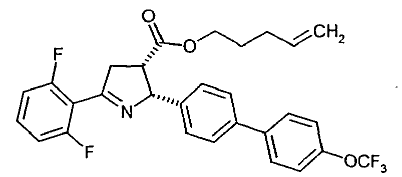 | 5,48^{a)} | |
| 27 | 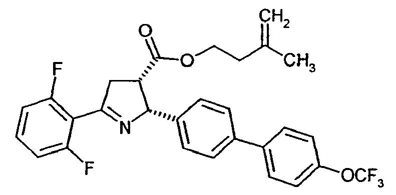 | 5,48^{a)} | |
| 28 | 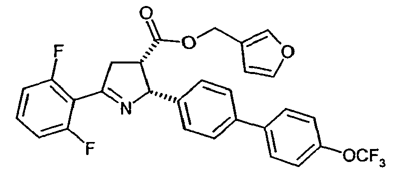 | 4,87^{a)} | |
| 29 | 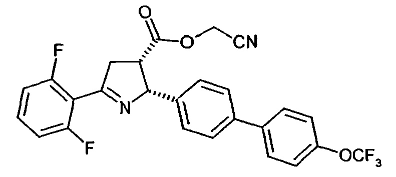 | 4,23^{a)} | |
| 30 | 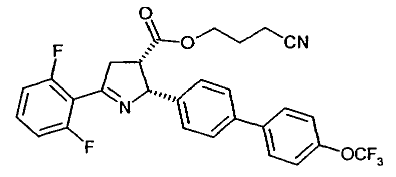 | 4,18^{a)} | |
| 31 | 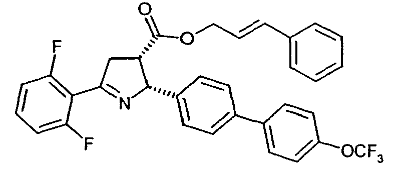 | 5,55^{a)} | |
| 32 | 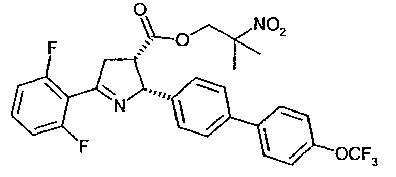 | 4,77^{a)} | |
| 33 | 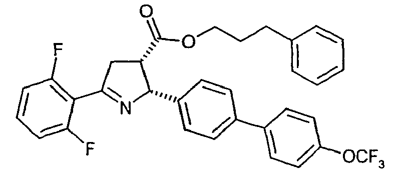 | 5,78^{a)} | |
| 34 | 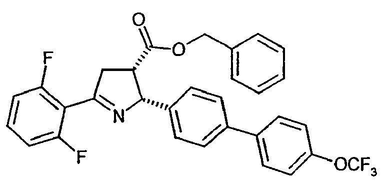 | 5,31^{a)} | |
| 35 | 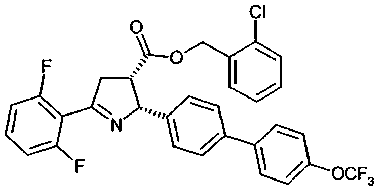 | 5,65^{a)} | |
| 36 | 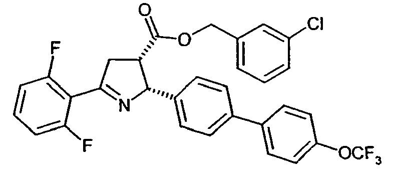 | 5,58^{a)} | |
| 37 | 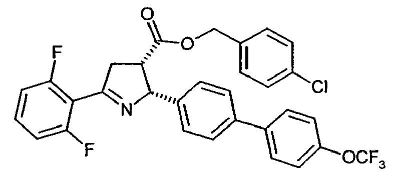 | | |
| 38 | 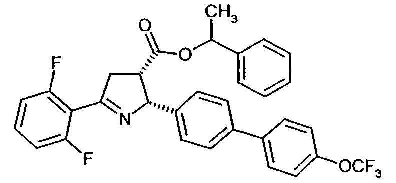 | 5,54^{a)} | |
| 39 | 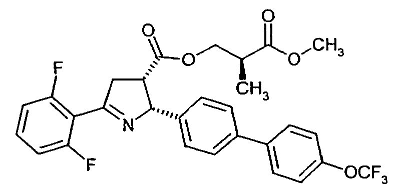 | 4,67^{a)} | |
| 40 | 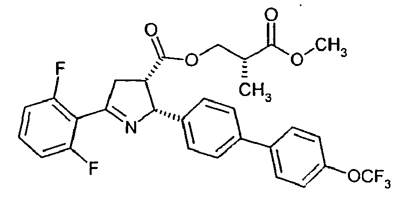 | 4,67^{a)} | |
| 41 | 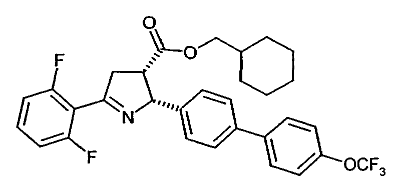 | 6,28^{a)} | |
| 42 | 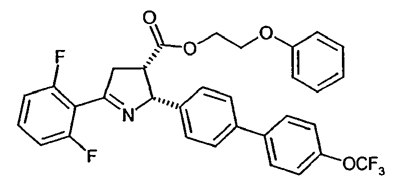 | 5,21^{a)} | |
| 43 | 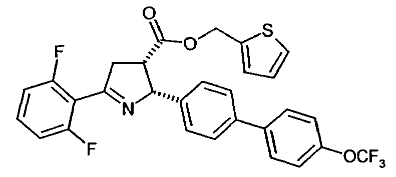 | 5,19^{a)} | |
| 44 | 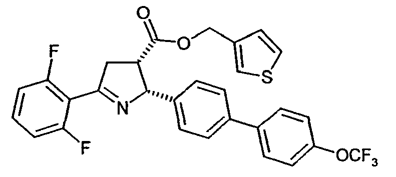 | 5,14^{a)} | |
| 45 | 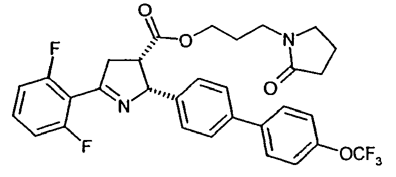 | 3,72^{a)} | |
| 46 | 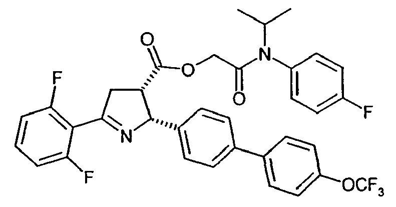 | 4,98^{a)} | |
| 47 | 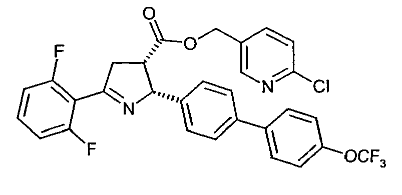 | 4,82^{a)} | |
| 48 | 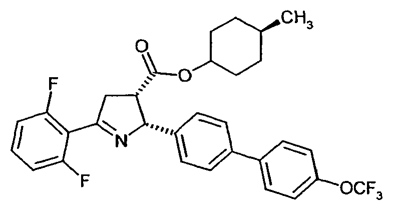 | 6,22^{a)} | |
| 49 | 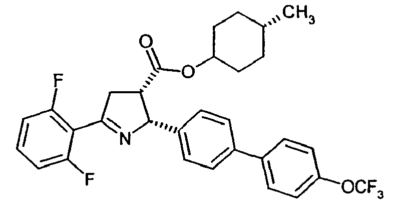 | 6,28^{a)} | |
| 50 | 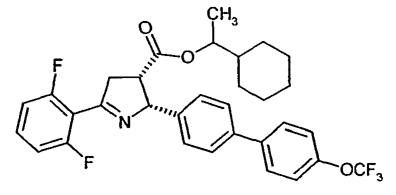 | 6,54^{a)} | |
| 51 | 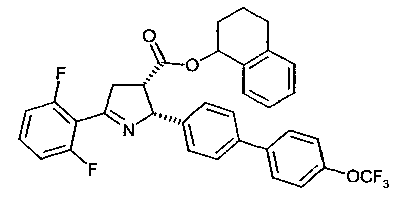 | 5,97^{a)} | |
| 52 | 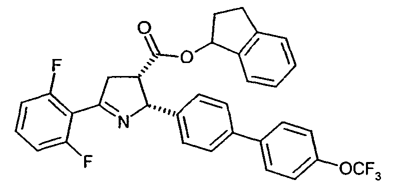 | 5,71^{a)} | |
| 53 | 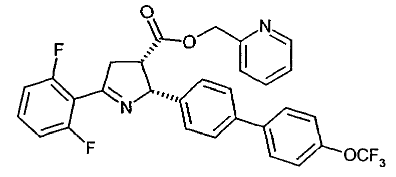 | 4,13^{a)} | |
| 54 | 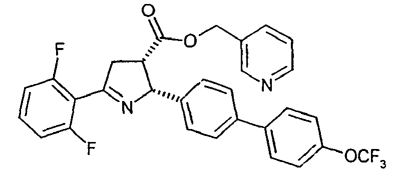 | 3,59^{a)} | |
| 55 | 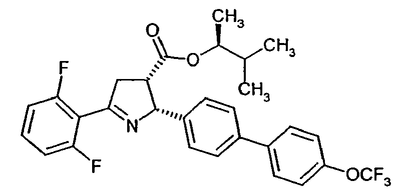 | 5,74^{a)} | |
| | | 5,77^{b)} | |
| 56 | 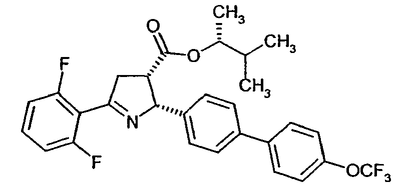 | 5,65^{a)} | |
| | | 5,68^{b)} | |
| 57 | 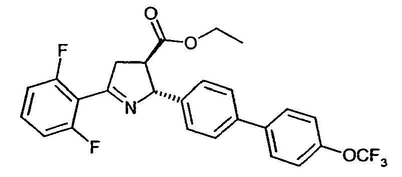 | | |
| 58 | 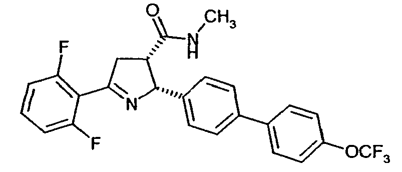 | 3,26^{a)} | |
| 59 | 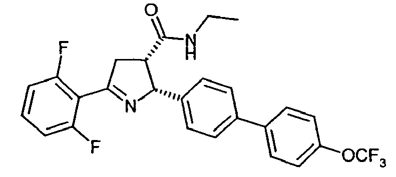 | 3,49^{a)} | |
| 60 | 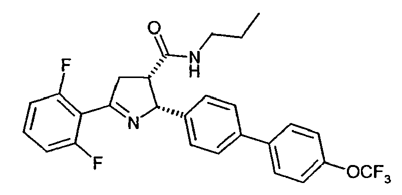 | 3,78^{a)} | |
| 61 | 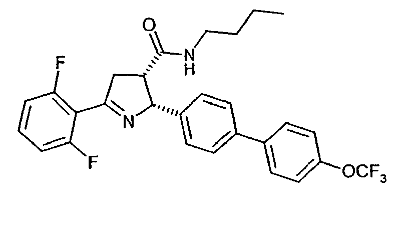 | 4,03^{a)} | |
| 62 | 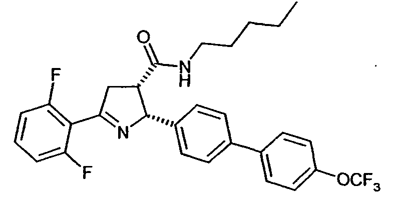 | 4,37^{a)} | |
| 63 | 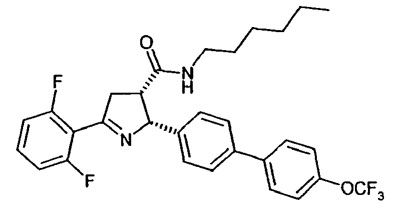 | 4,67^{a)} | |
| 64 | 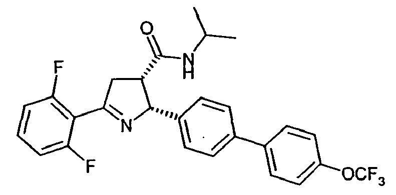 | 3,74^{a)} | |
| 65 | 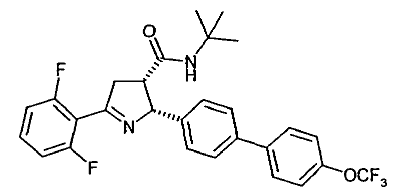 | 4,23^{a)} | |
| 66 | 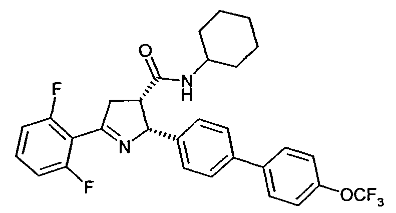 | 4,37^{a)} | |
| 67 | 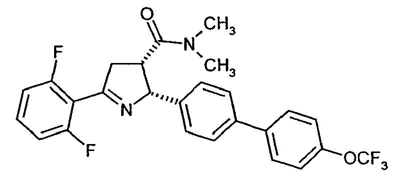 | 3,61^{a)} | |
| 68 | 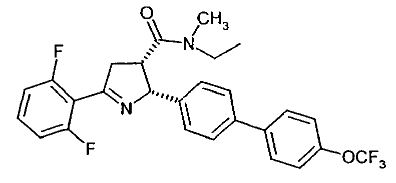 | 3,90^{a)} | |
| 69 | 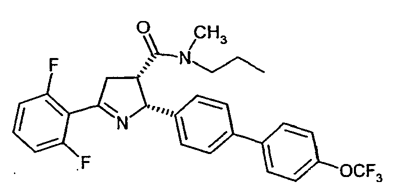 | 4,23^{a)} | |
| 70 | 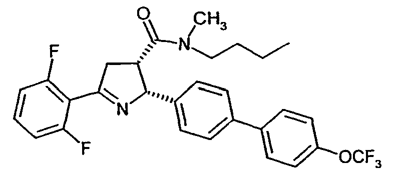 | 4,56^{a)} | |
| 71 | 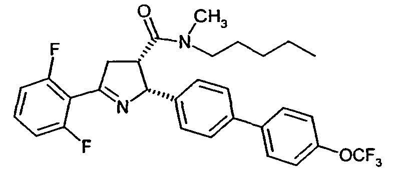 | 4,92^{a)} | |
| 72 | 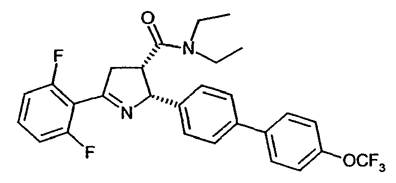 | 4,18^{a)} | |
| 73 | 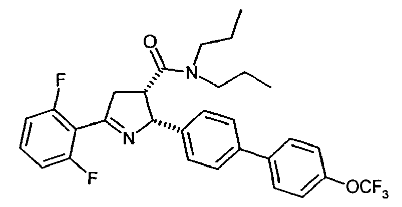 | 4,92^{a)} | |
| 74 | 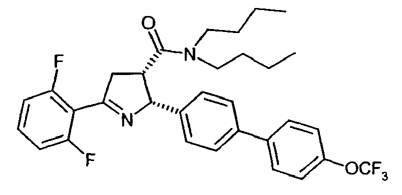 | 5,65^{a)} | |
| 75 | 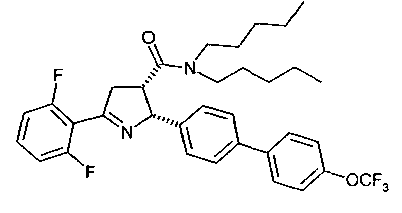 | 6,43^{a)} | |
| 76 | 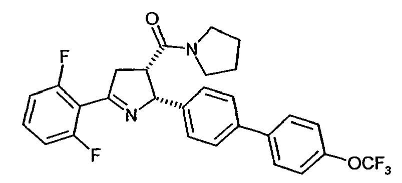 | 3,82^{a)} | |
| 77 | 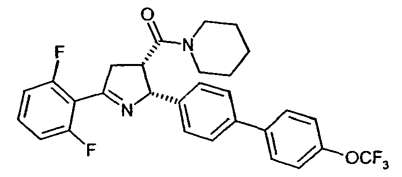 | 4,32^{a)} | |
| 78 | 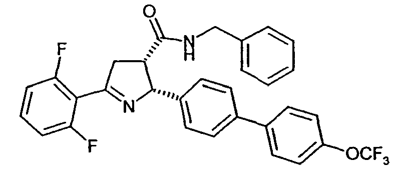 | 3,94^{a)} | |
| 79 | 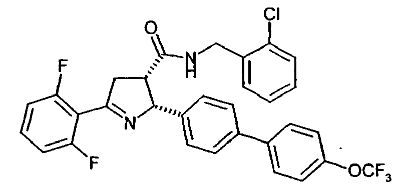 | 4,46^{a)} | |
| 80 | 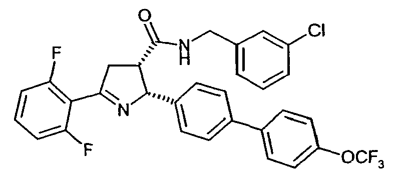 | 4,46^{a)} | |
| 81 | 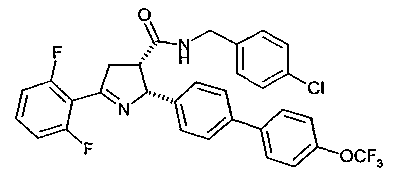 | 4,41^{a)} | |
| 82 | 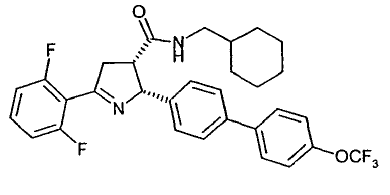 | 4,67^{a)} | |
| 83 | 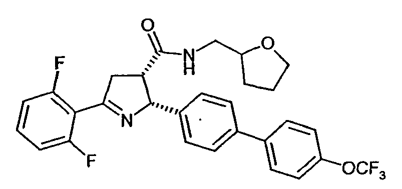 | 3,49^{a)} | |
| 84 | 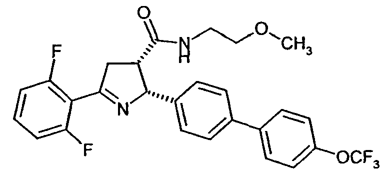 | 3,33^{a)} | |
| 85 | 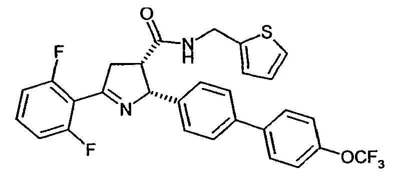 | 4,03^{a)} | |
| 86 | 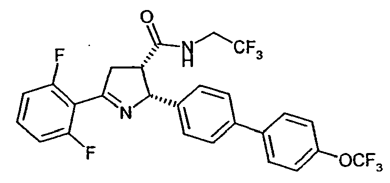 | 3,94^{a)} | |
| 87 | 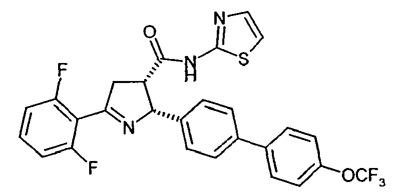 | 3,78^{a)} | |
| 88 | 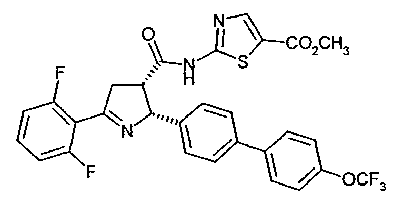 | 3,94^{a)} | |
| 89 | 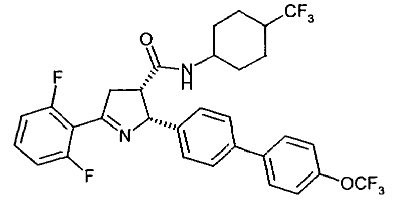 | 4,46^{a)} | |
| 90 | 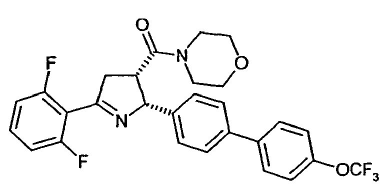 | 3,49^{a)} | |
| 91 | 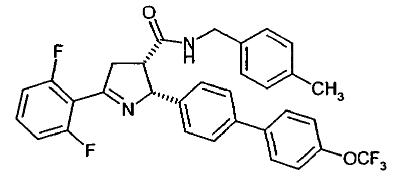 | 4,37^{a)} | |
| 92 | 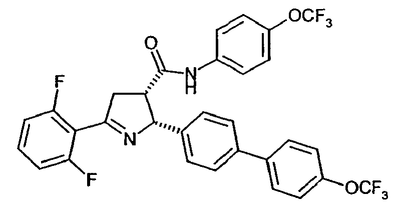 | 4,87^{a)} | |
| 93 | 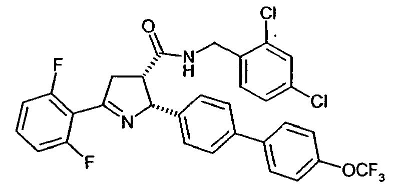 | 4,87^{a)} | |
| 94 | 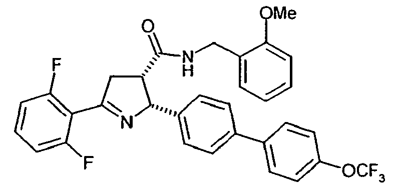 | 4,18^{a)} | |
| 95 | 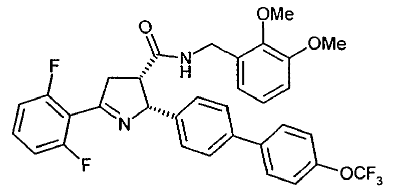 | 4,03^{a)} | |
| 96 | 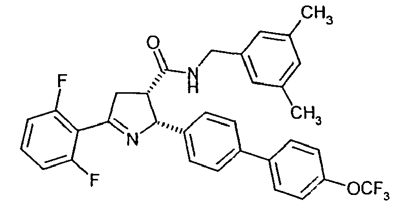 | 4,72^{a)} | |
| 97 | 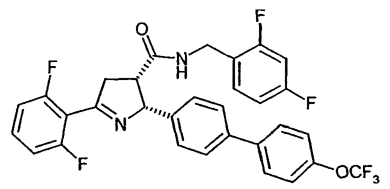 | 4,23^{a)} | |
| 98 | 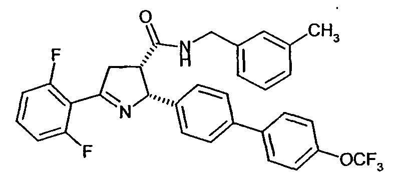 | 4,41^{a)} | |

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1% wässriger Phosphorsäure und Acetonitril als Eluenten; linearer Gradient von 10% Acetonitril bis 90% Acetonitril (in der Tabelle mit ^{a)} markiert).

Die Bestimmung erfolgt im neutralen Bereich bei pH 7.5 mit 0,01-molare wässriger Phosphatpuffer-Lösung und Acetonitril als Eluenten; linearer Gradient von 10 % Acetonitril bis 90 % Acetonitril (in der Tabelle mit ^{b)} markiert).

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Liriomyza-Test

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die von Eiern und Entwicklungsstadien der Thripse (Liriomyza trifolii) befallen sind, werden in eine Wirkstoffzubereitung der gewünschten Konzentration getaucht.

Nach der gewünschten Zeit wird die Wirkung in % bestimmt. Dabei bedeutet 100%, dass alle Thripse abgetötet wurden; 0% bedeutet, dass keine Thripse abgetötet wurden.

Wirkstoffe, Wirkstoffkonzentrationen und Versuchsergebnisse gehen aus der folgenden Tabelle hervor.

**Tabelle A: Liromyza-Test**

| Wirkstoff | Wirkstoffkonzentration in ppm | Abtötungsgrad in % nach 7 Tagen |
|---|---|---|
| 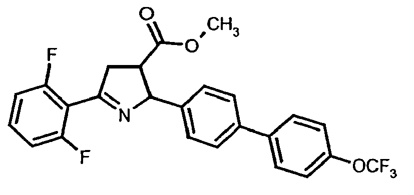 | 1000 | 100 |

### Beispiel B

### Meloidogyne-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100%, dass keine Gallen gefunden wurden; 0% bedeutet, dass die Zahl der Gallen an den behandelten Pflanzen der der unbehandelten Kontrolle entspricht.

Wirkstoffe, Wirkstoffkonzentrationen und Versuchsergebnisse gehen aus der folgenden Tabelle hervor.

**Tabelle B: Meloidogyne-Test**

| Wirkstoff | Wirkstoffkonzentration in ppm | Abtötungsgrad in % nach 14 Tagen |
|---|---|---|
| 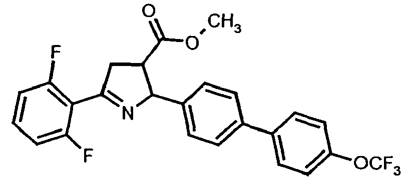 | 20 | 100 |
| 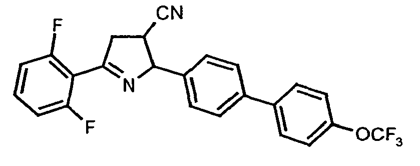 | 20 | 90 |

### Beispiel C

### Phaedon-Larven-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochloeariae) besetzt, solange die Blätter noch feucht sind.

Nach der gewünschten Zeit wird die Abtötung in % bestimmt. Dabei bedeutet 100%, dass alle Käferlarven abgetötet wurden; 0% bedeutet, dass keine Käferlarven abgetötet wurden.

Wirkstoffe, Wirkstoffkonzentrationen und Versuchsergebnisse gehen aus der folgenden Tabelle hervor.

**Tabelle C: Phaedon-Larven-Test**

| Wirkstoff | Wirkstoffkonzentration in ppm | Abtötungsgrad in % nach 7 Tagen |
|---|---|---|
| 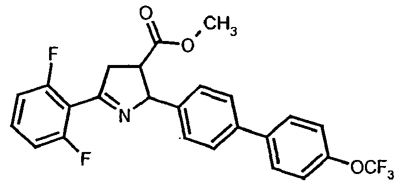 | 1000 | 100 |
| 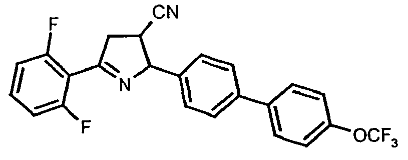 | 1000 | 100 |
| 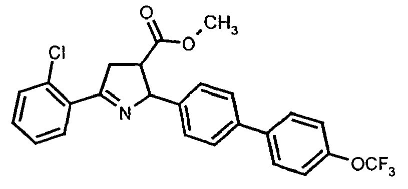 | 1000 | 100 |

### Beispiel D

### Spodoptera frugiperda-Test

| | |
|---|---|
| Lösungsmittel: | 30 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurmes (Spodoptera frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Wirkstoffe, Wirkstoffkonzentrationen und Versuchsergebnisse gehen aus der folgenden Tabelle hervor.

**Tabelle D: Spodoptera frugiperda -Test**

| Wirkstoff | Wirkstoffkonzentration in ppm | Abtötungsgrad in % nach 7 Tagen |
|---|---|---|
| 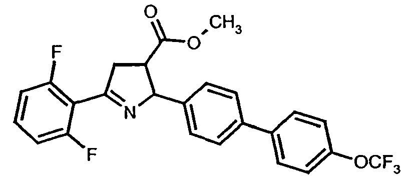 | 1000 | 100 |
| 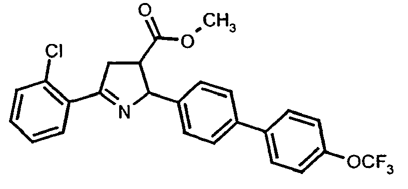 | 1000 | 100 |
| 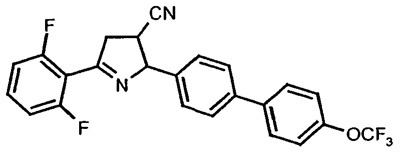 | 1000 | 100 |
| 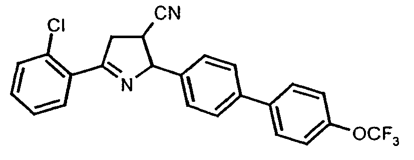 | 1000 | 100 |
| 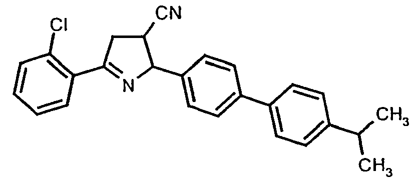 | 1000 | 100 |

### Beispiel E

**Diabrotica balteata - Test** (Larven im Boden) Grenzkonzentrations-Test / Bodeninsekten - Behandlung transgener Pflanzen

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel F

**Heliothis virescens - Test** (Behandlung transgener Pflanzen)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

## Patentansprüche

1. Δ¹-Pyrroline der Formel (I) 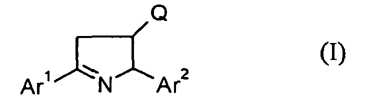 in welcher
Ar¹ für den Rest 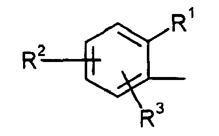 steht und
Ar² für den Rest 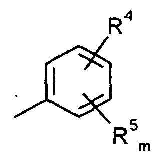 steht, in denen
m für 0, 1, 2, 3 oder 4 steht,
R¹ für Halogen, Alkyl, Alkoxy, Halogenalkyl, Halogenalkoxy, Alkoxyalkyl, -S(O)ₒR⁶, -NR⁷R⁸ steht,
R² und R³ unabhängig voneinander für Wasserstoff, Halogen, Cyano, Nitro, Alkyl, Alkoxy, Halogenalkyl, Halogenalkoxy, Alkoxyalkyl, -S(O)ₒR⁶, -NR⁷R⁸ stehen,
R⁴ für Halogen oder eine der folgenden Gruppierungen
(l) -X-A
(m) -B-Z-D
(n) -Y-E
steht,
R⁵ für Halogen, Hydroxy, Cyano, -CONH₂, -CSNH₂, Nitro, Alkyl, Alkylcarbonyl, Alkoxy, Halogenalkyl, Halogenalkoxy, Halogenalkylsulfonyloxy, Trialkylsilyl, Alkoxycarbonyl, -CONR⁷R⁸, -OSO₂NR⁷R⁸, -S(O)ₒR⁶, -NR⁷R⁸, -NHCO₂R⁶, Halogenalkylaminosulfonyl, Bisalkoxyboran, -B(OH)₂ steht,
X für eine direkte Bindung, Sauerstoff, -S(O)ₒ, -NR⁶, Carbonyl, Carbonyloxy, Oxycarbonyl, Oxysulfonyl (OSO₂), Alkylen, Alkenylen, Alkinylen, Alkylenoxy, Oxyalkylen, Oxyalkylenoxy, Thioalkylen, Cyclopropylen, Oxiranylen steht,
A für jeweils gegebenenfalls einfach oder mehrfach durch Reste aus der Liste W¹ substituiertes Phenyl, Naphthyl oder Tetrahydronaphthyl oder für gegebenenfalls einfach oder mehrfach durch Reste aus der Liste W² substituiertes 5- bis 10-gliedriges, gesättigtes oder ungesättigtes Heterocyclyl mit einem oder mehreren Heteroatomen aus der Reihe Stickstoff, Sauerstoff und Schwefel steht,
B für gegebenenfalls einfach oder zweifach durch Reste aus der Liste W¹ substituiertes p-Phenylen steht,
Z für -(CH₂)ₙ-, Sauerstoff oder -S(O)ₒ- steht,
D für Wasserstoff, Alkyl, Alkenyl, Alkinyl, Halogenalkyl, Halogenalkenyl, Halogenalkylsulfonyl, Dialkylaminosulfonyl steht,
Y für eine direkte Bindung, Sauerstoff, Schwefel, -SO₂-, Carbonyl, Carbonyloxy, Oxycarbonyl, Alkylen, Alkenylen, Alkinylen, Alkylenoxy, Oxyalkylen, Oxyalkylenoxy, Thioalkylen, Halogenalkylen, Halogenalkenylen steht,
E für Wasserstoff, Alkyl, Alkenyl, Alkinyl, Halogenalkyl, Halogenalkenyl, Halogenalkylsulfonyl, Dialkylaminosulfonyl steht,
W¹ für Cyano, Halogen, Formyl, Nitro, Alkyl, Trialkylsilyl, Alkoxy, Halogenalkyl, Halogenalkoxy, Halogenalkenyloxy, Halogenalkylsulfonyloxy, Alkylcarbonyl, Alkoxycarbonyl, Pentafluorthio, -S(O)ₒR⁶, -SO₂NR⁷R⁸, -OSO₂NR⁷R⁸, -OSO₂N(R⁶)CO₂R⁶, -OSO₂R¹², -C(R⁶)=N-O(R⁶) steht,
W² für Cyano, Halogen, Formyl, Nitro, Alkyl, Trialkylsilyl, Alkoxy, Halogenalkyl, Halogenalkoxy, Halogenalkenyloxy, Halogenalkylsulfonyloxy, Alkylcarbonyl, Alkoxycarbonyl, -S(O)ₒR⁶, -SO₂NR⁷R⁸, -OSO₂NR⁷R⁸, -C(R⁶)=N-O(R⁶) steht,
n für 0, 1, 2, 3 oder 4 steht,
Q für -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, einen 5- bis 7-gliedrigen gesättigten oder ungesättigten Heterocyclus mit 2 bis 4 Heteroatomen aus der Reihe Stickstoff, Sauerstoff und Schwefel steht,
Q außerdem für -CO₂R¹³ oder -CONR¹⁴R¹⁵ steht,
o für 0, 1 oder 2 steht,
R⁶ für Wasserstoff, Alkyl oder Halogenalkyl steht,
R⁷ und R⁸ unabhängig voneinander für Wasserstoff, Alkyl, Cycloalkyl, Halogenalkyl, oder gemeinsam für Alkylen stehen,
R⁹ für Wasserstoff, Alkyl, Cycloalkyl, Halogenalkyl, Aralkyl, Phenyl steht,
R¹⁰ für Alkyl, Halogenalkyl, Aralkyl steht,
R¹¹ für Alkyl, Aryl steht,
R¹² für Alkyl, Halogenalkyl, Aralkyl oder Aryl steht,
R¹³ für Wasserstoff; für einfach oder mehrfach substituiertes Alkyl; für gegebenenfalls substituiertes Aminocarbonylalkyl; für Alkenyl, Phenylalkenyl; für jeweils gegebenenfalls substituiertes Phenylalkyl oder Phenoxyalkyl; für jeweils gegebenenfalls substituiertes Cycloalkyl oder Cycloalkylalkyl; für gesättigtes oder ungesättigtes Heterocyclyl oder Heterocyclylalkyl mit jeweils 1 bis 4 Heteroatomen kombiniert aus der Reihe Stickstoff, Sauerstoff und Schwefel; für Tetrahydronaphthyl oder Indanyl steht,
R¹⁴ und R¹⁵ unabhängig voneinander für Wasserstoff, Halogenalkyl, Alkoxyalkyl; für jeweils gegebenenfalls substituiertes Phenyl oder Phenylalkyl; für jeweils gegebenenfalls substituiertes Cycloalkyl oder Cycloalkylalkyl; für jeweils gegebenenfalls substituiertes, jeweils gesättigtes oder ungesättigtes Heterocyclyl oder Heterocyclylalkyl mit jeweils 1 bis 4 Heteroatomen aus der Reihe Stickstoff, Sauerstoff und Schwefel stehen,
R¹⁴ und R¹⁵ außerdem gemeinsam für Alkylenoxyalkylen oder Alkylenthioalkylen stehen.

2. Verbindungen der Formel (I) gemäß Anspruch 1, in welchen
Ar¹ für den Rest 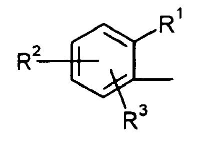 steht und
Ar² für den Rest 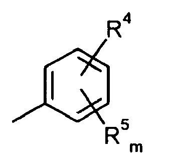 steht, worin
m für 0, 1, 2 oder 3 steht,
R¹ für Halogen, C₁-C₆-Alkyl, C₁-C₆-Alkoxy, C₁-C₆-Halogenalkyl, C₁-C₆-Halogenalkoxy, C₁-C₆-Alkoxy-C₁-C₆-alkyl, -S(O)ₒR⁶, -NR⁷R⁸ steht,
R² und R³ unabhängig voneinander für Wasserstoff, Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkoxy-C₁-C₆-alkyl, -S(O)ₒR⁶, -NR⁷R⁸ stehen,
R⁴ für Fluor, Chlor, Brom, Iod oder eine der folgenden in ortho- oder para-Position des Arylrings befindliche Gruppierungen
(l) -X-A
(m) -B-Z-D
(n) -Y-E
steht,
R⁵ für Halogen, Hydroxy, Cyano, -CONH₂, -CSNH₂, Nitro, C₁-C₆-Alkyl, C₁-C₆-Alkylcarbonyl, C₁-C₆-Alkoxy, C₁-C₆-Halogenalkyl, C₁-C₆-Halogenalkoxy, (C₁-C₆-Halogenalkyl)sulfonyloxy, Tri(C₁-C₆-alkyl)-silyl, C₁-C₆-Alkoxycarbonyl, -CONR⁷R⁸, -OSO₂NR⁷R⁸, -S(O)ₒR⁶, -NR⁷R⁸, -NHCO₂R⁶, (C₁-C₆-Halogenalkyl)aminosulfonyl, Bis(C₁-C₆-alkoxy)boran, -B(OH)₂ steht,
X für eine direkte Bindung, Sauerstoff, -S(O)ₒ, -NR⁶, Carbonyl, Carbonyloxy, Oxycarbonyl, Oxysulfonyl (OSO₂), C₁-C₄-Alkylen, C₂-C₄-Alkenylen, C₂-C₄-Alkinylen, C₁-C₄-Alkylenoxy, C₁-C₄-Oxyalkylen, C₁-C₄-Oxyalkylenoxy, C₁-C₄-Thioalkylen, Cyclopropylen, Oxiranylen steht,
A für jeweils gegebenenfalls einfach bis vierfach durch Reste aus der Liste W¹ substituiertes Phenyl, Naphthyl oder Tetrahydronaphthyl oder für gegebenenfalls einfach bis vierfach durch Reste aus der Liste W² substituiertes 5- bis 10-gliedriges, 1 oder 2 aromatische Ringe enthaltendes Heterocyclyl mit 1 bis 4 Heteroatomen, kombiniert aus 0 bis 4 Stickstoffatomen, 0 bis 2 Sauerstoffatomen und 0 bis 2 Schwefelatomen (insbesondere Tetrazolyl, Furyl, Benzofuryl, Thienyl, Benzothienyl, Pyrrolyl, Indolyl, Oxazolyl, Benzoxazolyl, Isoxazyl, Imidazyl, Pyrazyl, Thiazolyl, Benzothiazolyl, Pyridyl, Pyrimidinyl, Pyridazyl, Triazinyl, Triazyl, Chinolinyl oder Isochinolinyl) steht,
B für gegebenenfalls einfach oder zweifach durch Reste aus der Liste W¹ substituiertes p-Phenylen steht,
Z für -(CH₂)ₙ-, Sauerstoff oder -S(O)ₒ- steht,
D für Wasserstoff, C₁-C₆-Alkyl, C₂-C₆-Alkenyl, C₂-C₆-Alkinyl, C₁-C₆-Halogenalkyl, C₂-C₆-Halogenalkenyl, (C₁-C₆-Halogenalkyl)sulfonyl, Di(C₁-C₆-alkyl)aminosulfonyl steht,
Y für eine direkte Bindung, Sauerstoff, Schwefel, -SO₂-, Carbonyl, Carbonyloxy, Oxycarbonyl, C₁-C₆-Alkylen, C₂-C₆-Alkenylen, C₂-C₆-Alkinylen, C₁-C₆-Halogenalkylen, C₂-C₆-Halogenalkenylen, C₁-C₄-Alkylenoxy, C₁-C₄-Oxyalkylen, C₁-C₄-Oxyalkylenoxy, C₁-C₄-Thioalkylen steht,
E für Wasserstoff, C₁-C₆-Alkyl, C₂-C₆-Alkenyl, C₂-C₆-Alkinyl, C₁-C₆-Halogenalkyl, C₂-C₆-Halogenalkenyl, (C₁-C₆-Halogenalkyl)sulfonyl, Di(C₁-C₆-alkyl)aminosulfonyl steht,
W¹ für Cyano, Halogen, Formyl, Nitro, C₁-C₆-Alkyl, Tri(C₁-C₄-alkyl)silyl, C₁-C₆-Alkoxy, C₁-C₆-Halogenalkyl, C₁-C₆-Halogenalkoxy, C₂-C₆-Halogenalkenyloxy, (C₁-C₆-Halogenalkyl)sulfonyloxy, C₁-C₆-Alkylcarbonyl, C₁-C₆-Alkoxycarbonyl, Pentafluorthio, -S(O)ₒR⁶, -SO₂NR⁷R⁸, -OSO₂NR⁷R⁸, -OSO₂N(R⁶)CO₂R⁶, -OSO₂R¹², -C(R⁶)=N-O(R⁶) steht,
W² für Cyano, Halogen, Formyl, Nitro, C₁-C₆-Alkyl, Tri(C₁-C₄-alkyl)silyl, C₁-C₆-Alkoxy, C₁-C₆-Halogenalkyl, C₁-C₆-Halogenalkoxy, C₂-C₆-Halogenalkenyloxy, (C₁-C₆-Halogenalkyl)sulfonyloxy, C₁-C₆-Alkylcarbonyl, C₁-C₆-Alkoxycarbonyl, -S(O)ₒR⁶, -SO₂NR⁷R⁸, -OSO₂NR⁷R⁸, -C(R⁶)=N-O(R⁶) steht,
n für 0, 1, 2, 3 oder 4 steht,
Q für -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, einen 5- bis 7-gliedrigen gesättigten oder ungesättigten Heterocyclus mit 2 bis 4 Heteroatomen aus der Reihe Stickstoff, Sauerstoff und Schwefel steht,
Q außerdem für -CO₂R¹³ oder -CONR¹⁴R¹⁵ steht,
o für 0, 1 oder 2 steht,
R⁶ für Wasserstoff, C₁-C₆-Alkyl, C₁-C₆-Halogenalkyl steht,
R⁷ und R⁸ unabhängig voneinander für Wasserstoff, C₁-C₆-Alkyl, C₃-C₆-Cycloalkyl, C₁-C₆-Halogenalkyl, oder gemeinsam für Alkylen stehen,
R⁹ für Wasserstoff, C₁-C₆-Alkyl, C₃-C₆-Cycloalkyl, C₁-C₆-Halogenalkyl, Benzyl, Phenyl steht,
R¹⁰ für C₁-C₆-Alkyl, C₁-C₆-Halogenalkyl, Benzyl steht,
R¹¹ für C₁-C₆-Alkyl, Phenyl steht,
R¹² für C₁-C₆-Alkyl, C₁-C₆-Halogenalkyl, Benzyl oder Phenyl steht,
R¹³ für Wasserstoff; für einfach bis dreifach, gleich oder verschieden durch Cyano, Nitro, C₁-C₄-Alkoxy, C₁-C₄-Alkoxycarbonyl oder 2-Pyrrolidinon substituiertes C₁-C₆-Alkyl; für Aminocarbonyl-C₁-C₆-alkyl, welches an der Aminogruppe gleich oder verschieden durch C₁-C₄-Alkyl, Phenyl oder Halogenphenyl substituiert sein kann; für C₂-C₈-Alkenyl, Phenyl-C₂-C₆-alkenyl; für Phenyl-C₁-C₄-alkyl oder Phenoxy-C₁-C₄-alkyl, welche jeweils im Phenylring einfach bis vierfach, gleich oder verschieden durch Halogen, C₁-C₄-Alkyl, C₁-C₄-Alkoxy oder C₁-C₄-Halogenalkoxy substituiert sein können; für C₃-C₆-Cycloalkyl oder C₃-C₆-Cycloalkyl-C₁-C₄-alkyl, welche jeweils im Cycloalkylring einfach bis dreifach durch C₁-C₄-Alkyl substituiert sein können; für 5- oder 6-gliedriges gesättigtes oder ungesättigtes Heterocyclyl oder Heterocyclyl-C₁-C₄-alkyl mit jeweils 1 bis 4 Heteroatomen kombiniert aus 0 bis 4 Stickstoffatomen, 0 bis 2 Sauerstoffatomen und 0 bis 2 Schwefelatomen (insbesondere Furyl, Thienyl, Pyridinyl, Furfuryl, Thenyl oder Pyridinylmethyl), wobei der Heterocyclus jeweils durch Halogen substituiert sein kann; für Tetrahydronaphthyl oder Indanyl steht,
R¹⁴ und R¹⁵ unabhängig voneinander für Wasserstoff, C₁-C₆-Halogenalkyl, C₁-C₆-Alkoxy-C₁-C₆-alkyl; für Phenyl oder Phenyl-C₁-C₄-alkyl, welche jeweils im Phenylring einfach bis vierfach, gleich oder verschieden durch Halogen, C₁-C₄-Alkyl, C₁-C₄-Alkoxy, C₁-C₄-Halogenalkyl oder C₁-C₄-Halogenalkoxy substituiert sein können; für C₃-C₆-Cycloalkyl oder C₃-C₆-Cycloalkyl-C₁-C₄-alkyl, welche jeweils im Cycloalkylring einfach bis dreifach, gleich oder verschieden durch Halogen, C₁-C₄-Alkyl, C₁-C₄-Alkoxy, C₁-C₄-Halogenalkyl oder C₁-C₄-Halogenalkoxy substituiert sein können; für 5- oder 6-gliedriges gesättigtes oder ungesättigtes Heterocyclyl oder Heterocyclyl-C₁-C₄-alkyl mit jeweils 1 bis 4 Heteroatomen kombiniert aus 0 bis 4 Stickstoffatomen, 0 bis 2 Sauerstoffatomen und 0 bis 2 Schwefelatomen (insbesondere Furyl, Thienyl, Tetrahydrofuryl, Furfuryl, Thenyl, Tetrahydrofurylmethyl, Thiazolyl), wobei der Heterocyclus jeweils durch Halogen oder C₁-C₄-Alkoxycarbonyl substituiert sein kann stehen,
R¹⁴ und R¹⁵ außerdem gemeinsam für C₁-C₄-Alkylenoxy-C₁-C₄-alkylen oder C₁-C₄-Alkylenthio-C₁-C₄-alkylen stehen.

3. Verbindungen der Formel (I) gemäß Anspruch 1, in welchen
Ar¹ für den Rest 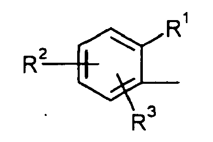 steht und
Ar² für den Rest 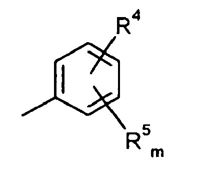 steht, in welchen
m für 0, 1 oder 2 steht,
R¹ für Fluor, Chlor, Brom, C₁-C₆-Alkyl, C₁-C₆-Alkoxy, jeweils durch Fluor oder Chlor substituiertes C₁-C₆-Alkyl oder C₁-C₆-Alkoxy steht,
R² und R³ unabhängig voneinander für Wasserstoff, Fluor, Chlor, Brom, Iod, C₁-C₆-Alkyl, C₁-C₆-Alkoxy, jeweils durch Fluor oder Chlor substituiertes C₁-C₆-Alkyl oder C₁-C₆-Alkoxy stehen,
R⁴ für Chlor, Brom, Iod oder eine der folgenden in ortho- oder para-Position des Arylrings befindlichen Gruppierungen
(l) -X-A
(m) -B-Z-D
(n) -Y-E
steht,
R⁵ für Fluor, Chlor, Brom, Iod, Hydroxy, Cyano, -CONH₂, -CSNH₂, Nitro, C₁-C₆-Alkyl, C₁-C₆-Alkylcarbonyl, C₁-C₆-Alkoxy, jeweils durch Fluor oder Chlor substituiertes C₁-C₆-Alkyl oder C₁-C₆-Alkoxy, -OSO₂CF₃, -CONR⁷R⁸, -OSO₂NR⁷R⁸, -S(O)ₒR⁶, -NR⁷R⁸, -NHCO₂R⁶, -B(OH)₂, 2-(4,4,5,5-Tetramethyl-1,3,2-dioxoborolan) steht,
X für eine direkte Bindung, Sauerstoff, Schwefel, -SO₂-, Carbonyl, Carbonyloxy, Oxycarbonyl, Oxysulfonyl (OSO₂), C₁-C₄-Alkylen, C₂-C₄-Alkenylen, C₂-C₄-Alkinylen, C₁-C₄-Alkylenoxy, C₁-C₄-Oxyalkylen, C₁-C₄-Oxyalkylenoxy, C₁-C₄-Thioalkylen, Cyclopropylen, Oxiranylen steht,
A für jeweils gegebenenfalls einfach bis dreifach durch Reste aus der Liste W¹ substituiertes Phenyl, Naphthyl oder Tetrahydronaphthyl oder für gegebenenfalls einfach bis dreifach durch Reste aus der Liste W² substituiertes 5- bis 10-gliedriges, 1 oder 2 aromatische Ringe enthaltendes Heterocyclyl mit 1 bis 4 Heteroatomen, kombiniert aus 0 bis 4 Stickstoffatomen, 0 bis 2 Sauerstoffatomen und 0 bis 2 Schwefelatomen (insbesondere Tetrazolyl, Furyl, Benzofuryl, Thienyl, Benzothienyl, Pyrrolyl, Indolyl, Oxazolyl, Benzoxazolyl, Isoxazyl, Imidazyl, Pyrazyl, Thiazolyl, Benzothiazolyl, Pyridyl, Pyrimidinyl, Pyridazyl, Triazinyl, Triazyl, Chinolinyl oder Isochinolinyl) steht,
B für gegebenenfalls einfach oder zweifach durch Reste aus der Liste W¹ substituiertes p-Phenylen steht,
Z für -(CH₂)ₙ-, Sauerstoff oder -S(O)ₒ- steht,
D für Wasserstoff, C₁-C₆-Alkyl, C₂-C₆-Alkenyl, C₂-C₆-Alkinyl, Di(C₁-C₄-Alkyl)aminosulfonyl, jeweils durch Fluor oder Chlor substituiertes C₁-C₆-Alkyl, C₂-C₆-Alkenyl oder C₁-C₄-Alkylsulfonyl steht,
Y für eine direkte Bindung, Sauerstoff, Schwefel, -SO₂-, Carbonyl, Carbonyloxy, Oxycarbonyl, C₁-C₆-Alkylen, C₂-C₆-Alkenylen, C₂-C₆-Alkinylen; jeweils durch Fluor oder Chlor substituiertes C₁-C₆-Alkylen oder C₂-C₆-Alkenylen; C₁-C₄-Alkylenoxy, C₁-C₄-Oxyalkylen, C₁-C₄-Oxyalkylenoxy, C₁-C₄-Thioalkylen steht,
E für Wasserstoff, C₁-C₆-Alkyl, C₂-C₆-Alkenyl, C₂-C₆-Alkinyl, Di(C₁-C₆-alkyl)aminosulfonyl, jeweils durch Fluor oder Chlor substituiertes C₁-C₆-Alkyl, C₂-C₆-Alkenyl oder C₁-C₆-Alkylsulfonyl steht,
W¹ für Cyano, Fluor, Chlor, Brom, Iod, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, C₂-C₆-Halogenalkenyloxy, (C₁-C₄-Halogenalkyl)sulfonyloxy, C₁-C₄-Alkylcarbonyl, C₁-C₄-Alkoxycarbonyl, -S(O)ₒR⁶, -SO₂NR⁷R⁸, -OSO₂NR⁷R⁸, -OSO₂R¹², -C(R⁶)=N-O(R⁶) steht,
W² für Cyano, Fluor, Chlor, Brom, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, C₂-C₆-Halogenalkenyloxy, -OSO₂CF₃, C₁-C₄-Alkylcarbonyl, C₁-C₄-Alkoxycarbonyl, -S(O)ₒR⁶, -SO₂NR⁷R⁸, -OSO₂NR⁷R⁸, -C(R⁶)=N-O(R⁶) steht,
n für 0, 1, 2 oder 3 steht,
Q für -CO₂R⁹, -COR¹⁰, -CONR⁷R⁸, -CN, -PO(OR¹¹)₂, einen 5- bis 7-gliedrigen gesättigten oder ungesättigten Heterocyclus mit 2- bis 4 Heteroatomen aus der Reihe Stickstoff, Sauerstoff und Schwefel (insbesondere Dihydrodioxazin, Oxazolin, Thiazolin, Imidazolin, Tetrazol) steht,
Q außerdem für -CO₂R¹³ oder -CONR¹⁴R¹⁵ steht,
o für 0, 1 oder 2 steht,
R⁶ für C₁-C₆-Alkyl oder jeweils durch Fluor oder Chlor substituiertes Methyl oder Ethyl steht,
R⁷ und R⁸ unabhängig voneinander für Wasserstoff, C₁-C₆-Alkyl, C₃-C₆-Cycloalkyl, jeweils durch Fluor oder Chlor substituiertes C₁-C₆-Alkyl, oder gemeinsam für C₄-C₅-Alkylen stehen,
R⁹ für Wasserstoff, C₁-C₆-Alkyl, C₃-C₆-Cycloalkyl, durch Fluor oder Chlor substituiertes C₁-C₆-Alkyl, Benzyl, Phenyl steht,
R¹⁰ für C₁-C₆-Alkyl, durch Fluor oder Chlor substituiertes C₁-C₆-Alkyl steht,
R¹¹ für C₁-C₄-Alkyl, Phenyl steht,
R¹² für C₁-C₄-Alkyl oder für durch Fluor oder Chlor substituiertes C₁-C₄-Alkyl steht,
R¹³ für Wasserstoff; für einfach oder zweifach, gleich oder verschieden durch Cyano, Nitro, Methoxy, Methoxycarbonyl oder 2-Pyrrolidinon substituiertes C₁-C₄-Alkyl; für Aminocarbonyl-C₁-C₄-alkyl, welches an der Aminogruppe gleich oder verschieden durch C₁-C₄-Alkyl, Phenyl oder Halogenphenyl substituiert sein kann; für C₂-C₆-Alkenyl, Phenyl-C₂-C₅-alkenyl; für Phenyl-C₁-C₄-alkyl oder Phenoxy-C₁-C₄-alkyl, welche jeweils im Phenylring einfach bis dreifach, gleich oder verschieden durch Fluor, Chlor, Methyl, Methoxy oder Trifluormethoxy substituiert sein können; für C₃-C₆-Cycloalkyl oder C₃-C₆-Cycloalkyl-C₁-C₄-alkyl, welche jeweils im Cycloalkylring einfach bis dreifach durch Methyl substituiert sein können; für Furyl, Thienyl, Pyridinyl, Furfuryl, Thenyl oder Pyridinylmethyl, welche jeweils im Heterocyclus durch Chlor substituiert sein können; für Tetrahydronaphthyl oder Indanyl steht,
R¹⁴ für Wasserstoff steht,
R¹⁵ für C₁-C₄-Halogenalkyl, C₁-C₄-Alkoxy-C₁-C₄-alkyl; für Phenyl oder Phenyl-C₁-C₄-alkyl, welche jeweils im Phenylring einfach bis vierfach, gleich oder verschieden durch Fluor, Chlor, Methyl, Methoxy, Trifluormethyl oder Trifluormethoxy substituiert sein können; für C₃-C₆-Cycloalkyl oder C₃-C₆-Cycloalkyl-C₁-C₄-alkyl, welche jeweils im Cycloalkylring einfach bis dreifach, gleich oder verschieden durch Fluor, Chlor, Methyl, Trifluormethyl oder Trifluormethoxy substituiert sein können; für Furyl, Thienyl, Tetrahydrofuryl, Furfuryl, Thenyl, Tetrahydrofurylmethyl, Thiazolyl oder durch C₁-C₄-Alkoxycarbonyl substituiertes Thiazolyl steht,
R¹⁴ und R¹⁵ außerdem gemeinsam für Morpholino oder Thiomorpholino stehen.

4. Verbindungen der Formel (I) gemäß Anspruch 1, in welchen
Ar¹ für den Rest 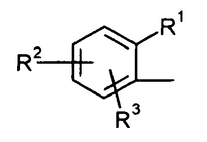 steht und
Ar² für den Rest 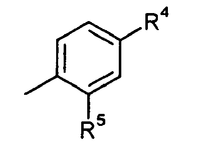 steht, in welchen
R¹ für Fluor, Chlor, Brom, Methyl, Ethyl, n-Propyl, Isopropyl, n-Butyl, Isobutyl, sec-Butyl, tert-Butyl, Methoxy, Ethoxy, n-Propoxy, n-Butoxy, Isobutoxy, sec-Butoxy, tert-Butoxy steht,
R² und R³ unabhängig voneinander für Wasserstoff, Fluor, Chlor, Brom, Methyl, Ethyl, n-Propyl, Isopropyl, n-Butyl, Isobutyl, sec-Butyl, tert-Butyl, Methoxy, Ethoxy, n-Propoxy, n-Butoxy, Isobutoxy, sec-Butoxy, tert-Butoxy stehen,
R⁴ für Chlor, Brom oder eine der folgenden Gruppierungen
(l) -X-A
(m) -B-Z-D
(n) -Y-E
steht,
R⁵ für Fluor, Chlor, Brom, Hydroxy, Methyl, Ethyl, Methoxy, Ethoxy, Methylthio, Ethylthio, Trifluormethyl, Difluormethoxy, Trifluormethoxy, Trifluormethylthio, -OSO₂CF₃, -OSO₂NMe₂ oder -SO₂CF₃ steht,
R⁵ außerdem für Wasserstoff steht,
X für eine direkte Bindung, Sauerstoff, Schwefel, -SO₂-, Carbonyl, -CH₂-, -(CH₂)₂-, -CH=CH- (E oder Z), -C≡C-, -CH₂O-, -(CH₂)₂O-, -OCH₂-, -SCH₂-, -S(CH₂)₂-, -OCH₂O-, -O(CH₂)₂O- steht,
A für gegebenenfalls einfach oder zweifach durch Reste aus der Liste W¹ substituiertes Phenyl, oder für jeweils gegebenenfalls einfach oder zweifach durch Reste aus der Liste W² substituiertes Tetrazolyl, Furyl, Benzofuryl, Thienyl, Benzothienyl, Pyrrolyl, Indolyl, Oxazolyl, Benzoxazolyl, Isoxazyl, Imidazyl, Pyrazyl, Thiazolyl, Benzothiazolyl, Pyridyl, Pyrimidinyl, Pyridazyl, Triazinyl, Triazyl steht,
B für gegebenenfalls einfach durch Reste aus der Liste W¹ substituiertes p-Phenylen steht,
Z für Sauerstoff, Schwefel oder -SO₂- steht,
D für Wasserstoff, Methyl, Ethyl, n-Propyl, Isopropyl, n-Butyl, Isobutyl, sec-Butyl, tert-Butyl, 2-Propenyl, Butenyl, Propargyl, Butinyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, -SO₂CF₃, -SO₂(CF₂)₃CF₃, -SO₂NMe₂ steht,
Y für eine direkte Bindung, Sauerstoff, Schwefel, -SO₂-, Carbonyl, -CH₂-, -(CH₂)₂-, -CH=CH- (E oder Z), -C≡C-, -CH₂O-, -(CH₂)₂O-, -OCH₂-, -SCH₂-, -S(CH₂)₂-, -OCH₂O-, -O(CH₂)₂O- steht,
E für Wasserstoff, Methyl, Ethyl, n-Propyl, Isopropyl, n-Butyl, Isobutyl, sec-Butyl, tert-Butyl, 2-Propenyl, Butenyl, Propargyl, Butinyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, -SO₂CF₃, -SO₂(CF₂)₃CF₃, -SO₂NMe₂ steht,
W¹ für Cyano, Fluor, Chlor, Brom, Formyl, Nitro, Methyl, Ethyl, n-Propyl, Isopropyl, n-Butyl, Isobutyl, sec-Butyl, tert-Butyl, Methoxy, Ethoxy, n-Propoxy, n-Butoxy, Isobutoxy, sec-Butoxy, tert-Butoxy, Trifluormethoxy, Difluormethoxy, -CF₃, -CHF₂, -CClF₂, -CF₂CHFCl, -CF₂CH₂F, -CF₂CCl₃, -CH₂CF₃, -CF₂CHFCF₃, -CH₂CF₂H, -OCH₂CF₃, -CH₂CF₂CF₃, -CF₂CF₂H, -CF₂CHFCF₃, -SCF₃, -SCHF₂, -SO₂CHF₂, -SO₂CF₃, -SOCHF₂, -SOCF₃, -SO₂NMe₂, -OSO₂CH₃, -OSO₂CF₃, -OSO₂(CF2)₃CF₃, -COCH₃, -CO₂CH₃, -CO₂Et, -SO₂Me, -OSO₂NMe₂, -C(Me)=N-O(Et), -C(Et)=N-OMe steht,
W² für Cyano, Fluor, Chlor, Brom, Methyl, Ethyl, n-Propyl, Isopropyl, tert-Butyl, Trifluormethyl, Trifluormethoxy, Difluormethoxy, Trifluormethylthio, -OSO₂CF₃, -COCH₃, -CO₂CH₃, -OCH₂CF₃, -SO₂CF₃, -SO₂NMe₂, -OSO₂NMe₂, -C(Me)=N-O(Et), -C(Et)=N-OMe steht,
Q für -CO₂CH₃, -CO₂CH₂CH₃, -CO₂-n-Propyl, -CO₂-Isopropyl, -CO₂-n-Butyl, -CO₂-Isobutyl, -CO₂-sec-Butyl, -CO₂-tert-Butyl, -CO₂-n-Pentyl, -CO₂-Neopentyl, -CO₂-sec-Isoamyl, -CO₂-Pentan-3-yl, -CO₂-Cyclopentyl, -CO₂-n-Hexyl, -CO₂-Cyclohexyl, -CO₂-Trifluorethyl, -CO₂CH₂Ph, -CO₂Ph, -COCH₃, -COCH₂CH₃, -CO-n-Propyl, -CONHMe, -CONHEt, -CONH(n-Propyl), -CONH(Isopropyl), -CONH(n-Butyl), -CONH(tert-Butyl), -CONH(n-Pentyl), -CONH(n-Hexyl), -CONH(Cyclohexyl), -CONMe₂, -CONEt₂, -CON(n-Propyl)₂, -CON(Isopropyl)₂, -CON(n-Butyl)₂, -CON(n-Pentyl)₂, -CON(Me)Et, -CON(Me)n-Propyl, -CON(Me)n-Butyl, -CON(Me)n-Pentyl, -CN, -PO(OMe)₂, Pyrrolidinocarbonyl, Piperidinocarbonyl, -PO(OEt)₂, -PO(OPh)₂, Dihydrodioxacinyl, Oxazolyl, Thiazolyl, Imidazolyl, Tetrazolyl steht,
Q außerdem für -CO₂CH₂CN, -CO₂(CH₂)₂CN, -CO₂(CH₂)₃CN, -CO₂CH₂C(CH₃)₂NO₂, -CO₂(CH₂)₂OCH₃, -CO₂H, 2-Methoxycarbonyl-propyloxycarbonyl, 3-(2-Oxo-1-pyrrolidinyl)propyloxycarbonyl, N-4-Fluorphenyl-N-isopropyl-amino-2-oxo-ethyloxycarbonyl, -CO₂(CH₂)₃CH=CH₂, -CO₂(CH₂)₂C(CH₃)=CH₂, -CO₂CH₂CH=CH-Ph, 2-Chlorbenzyloxycarbonyl, 3-Chlorbenzyloxycarbonyl, 4-Chlorbenzyloxycarbonyl, 1-Phenylethyloxycarbonyl, 3-Phenylpropyloxycarbonyl, 2-Phenoxyethyloxycarbonyl, 4-Methylcyclohexyloxycarbonyl, Cyclohexylmethyloxycarbonyl, 1-Cyclohexylethyloxycarbonyl, 3-Furfuryloxycarbonyl, 2-Thenyloxycarbonyl, 3-Thenyloxycarbonyl, 2-Pyridinylmethyloxycarbonyl, 3-Pyridinylmethyloxycarbonyl, 6-Chlor-3-pyridinylmethyloxycarbonyl, 1-Tetrahydronaphthyloxycarbonyl, 1-Indanyloxycarbonyl, -CON(H)CH₂CF₃, Methoxyethylaminocarbonyl, 4-Trifluormethoxyphenyl-aminocarbonyl, Benzyl-aminocarbonyl, 2-Chlorbenzyl-aminocarbonyl, 3-Chlorbenzyl-aminocarbonyl, 4-Chlorbenzyl-aminocarbonyl, 3-Methylbenzyl-aminocarbonyl, 4-Methylbenzyl-aminocarbonyl, 2,4-Dichlorbenzyl-aminocarbonyl, 2-Methoxybenzyl-aminocarbonyl, 2,3-Dimethoxybenzyl-aminocarbonyl, 3,5-Dimethylbenzyl-aminocarbonyl, 2,4-Difluorbenzyl-aminocarbonyl, 4-Trifluormethylcyclohexylaminocarbonyl, Cyclohexylmethyl-aminocarbonyl, 2-Thenyl-aminocarbonyl, 2-Tetrahydrofurylmethyl-aminocarbonyl, 2-Thiazolylaminocarbonyl, 5-Methoxycarbonyl-2-thiazolyl-aminocarbonyl oder für Morpholinocarbonyl steht,

5. Verbindungen der Formel (I) gemäß Anspruch 1, in welchen
Ar¹ für den Rest 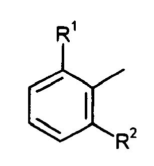 steht,
Ar² für den Rest 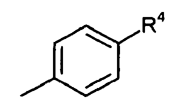 steht,
R¹ für Fluor oder Chlor steht,
R² für Wasserstoff, Chlor oder Fluor steht,
R⁴ für -B-Z-D steht,
B für p-Phenylen steht,
Z für Sauerstoff oder Schwefel steht,
D für -CF₃ steht,
Q für -CO₂CH₃, -CO₂CH₂CH₃, -CO₂-n-Propyl, -CO₂-Isopropyl, -CO₂-n-Butyl, -CO₂-Isobutyl, -CO₂-sec-Butyl, -CO₂-tert-Butyl, -CO₂-n-Pentyl, -CO₂-Neopentyl, -CO₂-sec-Isoamyl, -CO₂-Pentan-3-yl, -CO₂-Cyclopentyl, -CO₂-n-Hexyl, -CO₂-Cyclohexyl, -CONHMe, -CONHEt, -CONH(n-Propyl), -CONH(Isopropyl), -CONH(n-Butyl), -CONH(tert-Butyl), -CONH(n-Pentyl), -CONH(n-Hexyl), -CONH(Cyclohexyl), -CONMe₂, -CONEt₂, -CON(n-Propyl)₂, -CON(Isopropyl)₂, -CON(n-Butyl)₂, -CON(n-Pentyl)₂, -CON(Me)Et, -CON(Me)n-Propyl, -CON(Me)n-Butyl, -CON(Me)n-Pentyl, -CN, Pyrrolidinocarbonyl, Piperidinocarbonyl steht,
Q außerdem für -CO₂H, -CO₂CH₂CN, -CO₂(CH₂)₂CN, -CO₂(CH₂)₃CN, -CO₂CH₂C(CH₃)₂NO₂, -CO₂(CH₂)₂OCH₃, 2-Methoxycarbonyl-propyloxycarbonyl, 3-(2-Oxo-1-pyrrolidinyl)propyloxycarbonyl, N-4-Fluorphenyl-N-isopropyl-amino-2-oxo-ethyloxycarbonyl, -CO₂(CH₂)₃CH=CH₂, -CO₂(CH₂)₂C(CH₃)=CH₂, -CO₂CH₂CH=CH-Ph, 2-Chlorbenzyloxycarbonyl, 3-Chlorbenzyloxycarbonyl, 4-Chlorbenzyloxycarbonyl, 1-Phenylethyloxycarbonyl, 3-Phenylpropyloxycarbonyl, 2-Phenoxyethyloxycarbonyl, 4-Methylcyclohexyloxycarbonyl, Cyclohexylmethyloxycarbonyl, 1-Cyclohexylethyloxycarbonyl, 3-Furfuryloxycarbonyl, 2-Thenyloxycarbonyl, 3-Thenyloxycarbonyl, 2-Pyridinylmethyloxycarbonyl, 3-Pyridinylmethyloxycarbonyl, 6-Chlor-3-pyridinylmethyloxycarbonyl, 1-Tetrahydronaphthyloxycarbonyl, 1-Indanyloxycarbonyl, -CON(H)CH₂CF₃, Methoxyethylaminocarbonyl, 4-Trifluormethoxyphenyl-aminocarbonyl, Benzyl-aminocarbonyl, 2-Chlorbenzyl-aminocarbonyl, 3-Chlorbenzyl-aminocarbonyl, 4-Chlorbenzyl-aminocarbonyl, 3-Methylbenzyl-aminocarbonyl, 4-Methylbenzyl-aminocarbonyl, 2,4-Dichlorbenzyl-aminocarbonyl, 2-Methoxybenzyl-aminocarbonyl, 2,3-Dimethoxybenzyl-aminocarbonyl, 3,5-Dimethylbenzylaminocarbonyl, 2,4-Difluorbenzyl-aminocarbonyl, 4-Trifluormethylcyclohexyl-aminocarbonyl, Cyclohexylmethyl-aminocarbonyl, 2-Thenyl-aminocarbonyl, 2-Tetrahydrofurylmethyl-aminocarbonyl, 2-Thiazolyl-aminocarbonyl, 5-Methoxycarbonyl-2-thiazolylaminocarbonyl oder für Morpholinocarbonyl steht.

6. Verbindungen der Formel (I) gemäß Anspruch 1, wobei
Ar¹, Q und m die in den Ansprüchen 1 bis 5 angegebenen Bedeutungen haben, und
Ar² für den Rest 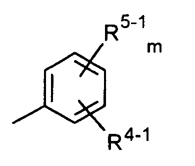 steht, wobei
R⁴⁻¹ für A oder B-Z-D
steht, wobei
A, B, Z und D die in den Ansprüchen 1 bis 5 angegebenen Bedeutungen haben,
und
R⁵⁻¹ für Fluor, Hydroxy, Cyano, Nitro, Alkyl, Alkoxy, Halogenalkyl, Halogenalkoxy, Trialkylsilyl, Alkoxycarbonyl, -CONR⁷R⁸, -OSO₂NR⁷R⁸, -S(O)ₒR⁶, -NR⁷R⁸, -NHCO₂R⁶, Haloalkylaminosulfonyl steht,
wobei
R⁶, R⁷, R⁸ und o die in den Ansprüchen 1 bis 5 angegebenen Bedeutungen haben.

7. Verfahren zur Herstellung von Verbindungen der Formel (I) gemäß einem der Ansprüche 1 bis 6, **dadurch gekennzeichnet**,
A-1.) dass man Iminochloride der Formel (II-a) 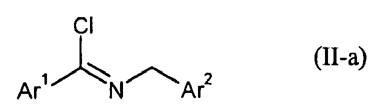 mit Dipolarophilen der Formel (III) 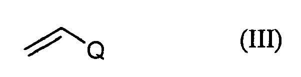 in welchen
Ar¹, Ar², und Q die in den Ansprüchen 1 bis 5 angegebenen Bedeutungen haben,
in Gegenwart eines Säurebindemittels und gegebenenfalls in Gegenwart eines Verdünnungsmittels umsetzt, oder
A-2.) dass man Iminochloride der Formel (II-b) 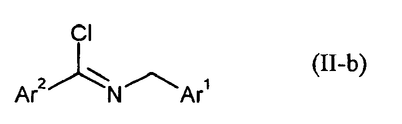 mit Dipolarophilen der Formel (III) 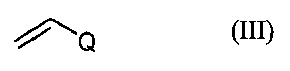 in welchen
Ar¹, Ar², und Q die in den Ansprüchen 1 bis 5 angegebenen Bedeutungen haben,
in Gegenwart eines Säurebindemittels und gegebenenfalls in Gegenwart eines Verdünnungsmittels umsetzt, oder
B.) dass man Oxazolinone der Formel (IV) 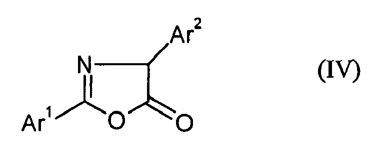 mit Dipolarophilen der Formel (III) 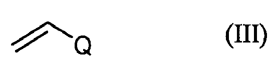 in welchen
Ar¹, Ar², und Q die in den Ansprüchen 1 bis 5 angegebenen Bedeutungen haben,
gegebenenfalls in Gegenwart eines Verdünnungsmittels umsetzt, oder
C.) dass man Carbonsäuren der Formel (V) 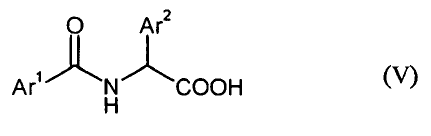 mit Dipolarophilen der Formel (III) 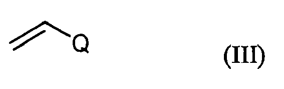 in welchen
Ar¹, Ar², und Q die in den Ansprüchen 1 bis 5 angegebenen Bedeutungen haben,
in Gegenwart von Essigsäureanhydrid und gegebenenfalls in Gegenwart eines Verdünnungsmittels umsetzt, oder
D.) dass man Δ¹-Pyrroline der Formel (I-a) 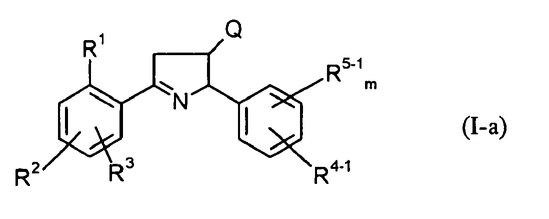 in welcher
R¹, R², R³, Q und m die in den Ansprüchen 1 bis 5 angegebenen Bedeutungen haben,
R⁴⁻¹ für A oder B-Z-D
steht, wobei
A, B, Z und D die in den Ansprüchen 1 bis 5 angegebenen Bedeutungen haben,
und
R⁵⁻¹ für Fluor, Hydroxy, Cyano, Nitro, Alkyl, Alkoxy, Halogenalkyl, Halogenalkoxy, Trialkylsilyl, Alkoxycarbonyl, -CONR⁷R⁸, -OSO₂NR⁷R⁸, -S(O)ₒR⁶, -NR⁷R⁸, -NHCO₂R⁶, Haloalkylaminosulfonyl steht,
wobei
R⁶, R⁷, R⁸ und o die in den Ansprüchen 1 bis 5 angegebenen Bedeutungen haben,
erhält, indem man Verbindungen der Formel (I-b) 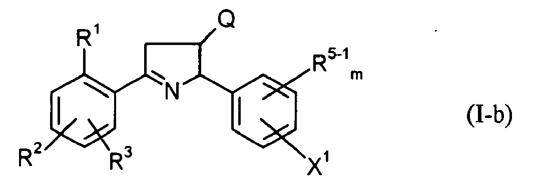 in welcher
R¹, R², R³, R⁵⁻¹, Q und m die in den Ansprüchen 1 bis 5 angegebenen Bedeutungen haben und
X¹ für Cl, Br, I, -OSO₂CF₃ steht
mit Boronsäuren der Formel (VI) 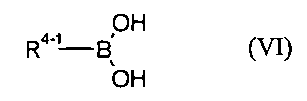 in welcher
R⁴⁻¹ die in diesem Anspruch angegebene Bedeutung hat,
in Gegenwart eines Katalysators, gegebenenfalls in Gegenwart eines Säurebindemittels und gegebenenfalls in Gegenwart eines Verdünnungsmittels umsetzt
oder
dass man Verbindungen der Formel (I-a) erhält, indem man Verbindungen der Formel (I-b)
in welcher
X¹ für 2-(4,4,5,5-Tetramethyl-1,3,2-dioxoborolan) steht
mit (Hetero)cyclen der Formel (VII)
T-A (VII)
in welcher A die in den Ansprüchen 1 bis 5 angegebene Bedeutung hat und
T für Cl, Br, I, -OSO₂CF₃ steht,
in Gegenwart eines Katalysators, gegebenenfalls in Gegenwart eines Säurebindemittels und gegebenenfalls in Gegenwart eines Verdünnungsmittels umsetzt.

8. Schädlingsbekämpfungsmittel, **gekennzeichnet durch** einen Gehalt an mindestens einer Verbindung der Formel (I) gemäß einem der Ansprüche 1 bis 6 neben Streckmitteln und/oder oberflächenaktiven Stoffen.

9. Verwendung von Verbindungen der Formel (I) gemäß einem der Ansprüche 1 bis 6 zur Bekämpfung von Schädlingen, ausgenommen zur therapeutischen Behandlung des menschlichen oder tierischen Körpers.

10. Verfahren zur Bekämpfung von Schädlingen, **dadurch gekennzeichnet, dass** man Verbindungen der Formel (I) gemäß einem der Ansprüche 1 bis 6 auf Schädlinge und/oder ihren Lebensraum einwirken läßt, ausgenommen zur therapeutischen Behandlung des menschlichen oder tierischen Körpers.

11. Verwendung von Verbindungen der Formel (I) gemäß einem der Ansprüche 1 bis 6 zur Herstellung eines Tierarzneimittels gegen tierische Parasiten, insbesondere Ektoparasiten.

12. Verfahren zur Herstellung von Schädlingsbekämpfungsmitteln, **dadurch gekennzeichnet, dass** man Verbindungen der Formel (I) gemäß einem der Ansprüche 1 bis 6 mit Streckmitteln und/oder oberflächenaktiven Stoffen vermischt.

13. Verwendung von Verbindungen der Formel (I) gemäß einem der Ansprüche 1 bis 6 zur Herstellung von Schädlingsbekämpfungsmitteln.

## Claims

1. Δ¹-Pyrrolines of the formula (I) 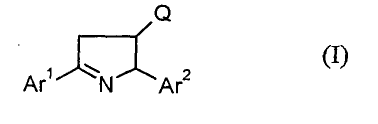 in which
Ar¹ represents the radical 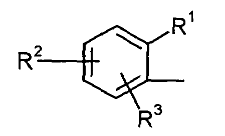 and
Ar² represents the radical 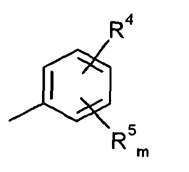 in which
m represents 0,1, 2, 3 or 4,
R¹ represents halogen, alkyl, alkoxy, halogenoalkyl, halogenoalkoxy, alkoxyalkyl, -S(O)ₒR⁶ or -NR⁷R⁸,
R² and R³ independently of one another each represent hydrogen, halogen, cyano, nitro, alkyl, alkoxy, halogenoalkyl, halogenoalkoxy, alkoxyalkyl, -S(O)ₒR⁶ or -NR⁷R⁸,
R⁴ represents halogen or one of the groupings below
(l) -X-A
(m) -B-Z-D
(n) -Y-E,
R⁵ represents halogen, hydroxyl, cyano, -CONH₂, -CSNH₂, nitro, alkyl, alkylcarbonyl, alkoxy, halogenoalkyl, halogenoalkoxy, halogenoalkylsulphonyloxy, trialkylsilyl, alkoxycarbonyl, -CONR⁷R⁸, -OSO₂NR⁷R⁸, -S(O)ₒR⁶, -NR⁷R⁸, -NHCO₂R⁶, halogenoalkylaminosulphonyl, bisalkoxyborane or -B(OH)₂,
X represents a direct bond, oxygen, -S(O)ₒ, -NR⁶, carbonyl, carbonyloxy, oxycarbonyl, oxysulphonyl (OSO₂), alkylene, alkenylene, alkinylene, alkyleneoxy, oxyalkylene, oxyalkyleneoxy, thioalkylene, cyclopropylene or oxiranylene,
A represents phenyl, naphthyl or tetrahydronaphthyl, each of which is optionally mono- or polysubstituted by radicals from the list W¹ or represents 5- to 10-membered saturated or unsaturated heterocyclyl which contains one or more heteroatoms from the group consisting of nitrogen, oxygen and sulphur and which is optionally mono- or polysubstituted by radicals from the list W²,
B represents p-phenylene which is optionally mono- or disubstituted by radicals from the list W¹,
Z represents -(CH₂)ₙ-, oxygen or -S(O)ₒ-,
D represents hydrogen, alkyl, alkenyl, alkinyl, halogenoalkyl, halogenoalkenyl, halogenoalkylsulphonyl or dialkylaminosulphonyl,
Y repreents a direct bond, oxygen, sulphur, -SO₂-, carbonyl, carbonyloxy, oxycarbonyl, alkylene, alkenylene, alkinylene, alkyleneoxy, oxyalkylene, oxyalkyleneoxy, thioalkylene, halogenoalkylene or halogenoalkenylene,
E represents hydrogen, alkyl, alkenyl, alkinyl, halogenoalkyl, halogenoalkenyl, halogenoalkylsulphonyl or dialkylaminosulphonyl,
W¹ represents cyano, halogen, formyl, nitro, alkyl, trialkylsilyl, alkoxy, halogenoalkyl, halogenoalkoxy, halogenoalkenyloxy, halogenoalkylsulphonyloxy, alkylcarbonyl, alkoxycarbonyl, pentafluorothio, -S(O)ₒR⁶, -SO₂NR⁷R⁸, -OSO₂NR⁷R⁸, -OSO₂N(R⁶)CO₂R⁶, -OSO₂R¹² or -C(R⁶)=N-O(R⁶),
W² represents cyano, halogen, formyl, nitro, alkyl, trialkylsilyl, alkoxy, halogenoalkyl, halogenoalkoxy, halogenoalkenyloxy, halogenoalkylsulphonyloxy, alkylcarbonyl, alkoxycarbonyl, -S(O)ₒR⁶, -SO₂NR⁷R⁸, -OSO₂NR⁷R⁸ or -C(R⁶)=N-O(R⁶),
n represents 0, 1, 2, 3 or 4,
Q represents -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, a 5- to 7-membered saturated or unsaturated heterocycle which contains 2 to 4 heteroatoms from the group consisting of nitrogen, oxygen and sulphur,
Q furthermore represents -CO₂R¹³ or -CONR¹⁴R¹⁵,
o represents 0, 1 or 2,
R⁶ represents hydrogen, alkyl or halogenoalkyl,
R⁷ and R⁸ independently of one another each represent hydrogen, alkyl, cycloalkyl, halogenoalkyl, or together represent alkylene,
R⁹ represents hydrogen, alkyl, cycloalkyl, halogenoalkyl, aralkyl or phenyl,
R¹⁰ represents alkyl, halogenoalkyl or aralkyl,
R¹¹ represents alkyl or aryl,
R¹² represents alkyl, halogenoalkyl, aralkyl or aryl,
R¹³ represents hydrogen; represents mono- or polysubstituted alkyl; represents optionally substituted aminocarbonylalkyl; represents alkenyl or phenylalkenyl; represents in each case optionally substituted phenylalkyl or phenoxyalkyl; represents in each case optionally substituted cycloalkyl or cycloalkylalkyl; represents saturated or unsaturated heterocyclyl or heterocyclylalkyl, each of which contains 1 to 4 heteroatoms selected from the group consisting of nitrogen, oxygen and sulphur; or represents tetrahydronaphthyl or indanyl,
R¹⁴ and R¹⁵ independently of one another each represent hydrogen, halogenoalkyl or alkoxyalkyl; represent in each case optionally substituted phenyl or phenylalkyl; represent in each case optionally substituted cycloalkyl or cycloalkylalkyl; or represent in each case optionally substituted, in each case saturated or unsaturated heterocyclyl or heterocyclylalkyl, each of which contains 1 to 4 heteroatoms from the group consisting of nitrogen, oxygen and sulphur,
R¹⁴ and R¹⁵ furthermore together represent alkyleneoxyalkylene or alkylenethioalkylene.

2. Compounds of the formula (I) according to Claim 1 in which
Ar¹ represents the radical 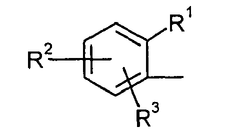 and
Ar² represents the radical 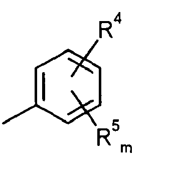 in which
m represents 0, 1, 2 or 3,
R¹ represents halogen, C₁-C₆-alkyl, C₁-C₆-alkoxy, C₁-C₆-halogenoalkyl, C₁-C₆-halogenoalkoxy, C₁-C₆-alkoxy-C₁-C₆-alkyl, -S(O)ₒR⁶ or -NR⁷R⁸,
R² and R³ independently of one another each represent hydrogen, halogen, cyano, nitro, C₁-C₆-alkyl, C₁-C₆-alkoxy, C₁-C₆-halogenoalkyl, C₁-C₆-halogenoalkoxy, C₁-C₆-alkoxy-C₁-C₆-alkyl, -S(O)ₒR⁶ or -NR⁷R⁸,
R⁴ represents fluorine, chlorine, bromine, iodine or one of the groups below, which are located in the ortho- or para-position of the aryl ring
(l) -X-A
(m) -B-Z-D
(n) -Y-E,
R⁵ represents halogen, hydroxyl, cyano, -CONH₂, -CSNH₂, nitro, C₁-C₆-alkyl, C₁-C₆-alkylcarbonyl, C₁-C₆-alkoxy, C₁-C₆-halogenoalkyl, C₁-C₆-halogenoalkoxy, (C₁-C₆-halogenoalkyl)sulphonyloxy, tri(C₁-C₆-alkyl)-silyl, C₁-C₆-alkoxycarbonyl,-CONR⁷R⁸, -OSO₂NR⁷R⁸, -S(O)ₒR⁶, -NR⁷R⁸, -NHCO₂R⁶, (C₁-C₆-halogenoalkyl)aminosulphonyl, bis(C₁-C₆-alkoxy)borane or -B(OH)₂,
X represents a direct bond, oxygen, -S(O)ₒ, -NR⁶, carbonyl, carbonyloxy, oxycarbonyl, oxysulphonyl (OSO₂), C₁-C₄-alkylene, C₂-C₄-alkenylene, C₂-C₄-alkinylene, C₁-C₄-alkyleneoxy, C₁-C₄-oxyalkylene, C₁-C₄-oxyalkyleneoxy, C₁-C₄-thioalkylene, cyclopropylene or oxiranylene,
A represents phenyl, naphthyl or tetrahydronaphthyl, each of which is optionally mono- to tetrasubstituted by radicals from the list W¹, or represents 5- to 10-membered heterocyclyl which contains 1 or 2 aromatic rings and 1 to 4 heteroatoms selected from 0 to 4 nitrogen atoms, 0 to 2 oxygen atoms and 0 to 2 sulphur atoms (in particular tetrazolyl, furyl, benzofuryl, thienyl, benzothienyl, pyrrolyl, indolyl, oxazolyl, benzoxazolyl, isoxazyl, imidazyl, pyrazyl, thiazolyl, benzothiazolyl, pyridyl, pyrimidinyl, pyridazyl, triazinyl, triazyl, quinolinyl or isoquinolinyl) and which is optionally mono- to tetrasubstituted by radicals from the list W²,
B represents p-phenylene which is optionally mono- or disubstituted by radicals from the list W¹,
Z represents -(CH₂)ₙ-, oxygen or -S(O)ₒ-,
D represents hydrogen, C₁-C₆-alkyl, C₂-C₆-alkenyl, C₂-C₆-alkinyl, C₁-C₆-halogenoalkyl, C₂-C₆-halogenoalkenyl, (C₁-C₆-halogenoalkyl)sulphonyl or di(C₁-C₆-alkyl)aminosulphonyl,
Y represents a direct bond, oxygen, sulphur, -SO₂-, carbonyl, carbonyloxy, oxycarbonyl, C₁-C₆-alkylene, C₂-C₆-alkenylene, C₂-C₆-alkinylene, C₁-C₆-halogenoalkylene, C₂-C₆-halogenoalkenylene, C₁-C₄-alkyleneoxy, C₁-C₄-oxyalkylene, C₁-C₄-oxyalkyleneoxy or C₁-C₄-thioalkylene,
E represents hydrogen, C₁-C₆-alkyl, C₂-C₆-alkenyl, C₂-C₆-alkinyl, C₁-C₆-halogenoalkyl, C₂-C₆-halogenoalkenyl, (C₁-C₆-halogenoalkyl)sulphonyl or di(C₁-C₆-alkyl)aminosulphonyl,
W¹ represents cyano, halogen, formyl, nitro, C₁-C₆-alkyl, tri(C₁-C₄-alkyl)silyl, C₁-C₆-alkoxy, C₁-C₆-halogenoalkyl, C₁-C₆-halogenoalkoxy, C₂-C₆-halogenoalkenyloxy, (C₁-C₆-halogenalkyl)sulphonyloxy, C₁-C₆-alkylcarbonyl, C₁-C₆-alkoxycarbonyl, pentafluorothio, -S(O)ₒR⁶, -SO₂NR⁷R⁸, -OSO₂NR⁷R⁸, -OSO₂N(R⁶)CO₂R⁶, -OSO₂R¹² or -C(R⁶)=N-O(R⁶),
W² represents cyano, halogen, formyl, nitro, C₁-C₆-alkyl, tri(C₁-C₄-alkyl)silyl, C₁-C₆-alkoxy, C₁-C₆-halogenoalkyl, C₁-C₆-halogenoalkoxy, C₂-C₆-halogenoalkenyloxy, (C₁-C₆-halogenoalkyl)sulphonyloxy, C₁-C₆-alkylcarbonyl, C₁-C₆-alkoxycarbonyl, -S(O)ₒR⁶, -SO₂NR⁷R⁸, -OSO₂NR⁷R⁸ or -C(R⁶)=N-O(R⁶),
n represents 0, 1, 2, 3 or 4,
Q represents -CO₂R⁹, -COR¹⁰, -CONR⁷R⁸, -CN, -CONH₂, -CSNH₂, -S(O)₀R⁶, -SO₂NR⁷R⁸, -PO(OR¹¹)₂, a 5- to 7-membered saturated or unsaturated heterocycle having 2 to 4 heteroatoms from the group consisting of nitrogen, oxygen and sulphur,
Q furthermore represents -CO₂R¹³ or -CONR¹⁴R¹⁵,
o represents 0, 1 or 2,
R⁶ represents hydrogen, C₁-C₆-alkyl or C₁-C₆-halogenoalkyl,
R⁷ and R⁸ independently of one another each represent hydrogen, C₁-C₆-alkyl, C₃-C₆-cycloalkyl or C₁-C₆-halogenoalkyl, or together represent alkylene,
R⁹ represents hydrogen, C₁-C₆-alkyl, C₃-C₆-cycloalkyl, C₁-C₆-halogenoalkyl, benzyl or phenyl,
R¹⁰ represents C₁-C₆-alkyl, C₁-C₆-halogenoalkyl or benzyl,
R¹¹ represents C₁-C₆-alkyl or phenyl,
R¹² represents C₁-C₆-alkyl, C₁-C₆-halogenoalkyl, benzyl or phenyl,
R¹³ represents hydrogen; represents C₁-C₆-alkyl which is mono- to trisubstituted by identical or different substituents from the group consisting of cyano, nitro, C₁-C₄-alkoxy, C₁-C₄-alkoxycarbonyl and 2-pyrrolidinone; represents aminocarbonyl C₁-C₆-alkyl which may be substituted on the amino group by identical or different substituents from the group consisting of C₁-C₄-alkyl, phenyl and halogenophenyl; represents C₂-C₈-alkenyl or phenyl-C₂-C₆-alkenyl; represents phenyl-C₁-C₄-alkyl or phenoxy-C₁-C₄-alkyl which may in each case be mono- to tetrasubstituted on the phenyl ring by identical or different substituents from the group consisting of halogen, C₁-C₄-alkyl, C₁-C₄-alkoxy and C₁-C₄-halogenoalkoxy; represents C₃-C₆-cycloalkyl or C₃-C₆-cycloalkyl-C₁-C₄-alkyl, which may in each case be mono- to trisubstituted on the cycloalkyl ring by C₁-C₄-alkyl; represents 5- or 6-membered saturated or unsaturated heterocyclyl or heterocyclyl-C₁-C₄-alkyl, each of which contains 1 to 4 heteroatoms selected from 0 to 4 nitrogen atoms, 0 to 2 oxygen atoms and 0 to 2 sulphur atoms (in particular furyl, thienyl, pyridinyl, furfuryl, thenyl or pyridinylmethyl), where the heterocycle may in each case be substituted by halogen; or represents tetrahydronaphthyl or indanyl,
R¹⁴ and R¹⁵ independently of one another each represent hydrogen, C₁-C₆-halogenoalkyl or C₁-C₆-alkoxy-C₁-C₆-alkyl; represent phenyl or phenyl-C₁-C₄-alkyl which may in each case be mono- to tetrasubstituted on the phenyl ring by identical or different substituents from the group consisting of halogen, C₁-C₄-alkyl, C₁-C4-alkoxy, C₁-C₄-halogenoalkyl and C₁-C₄-halogenoalkoxy; represent C₃-C₆-cycloalkyl or C₃-C₆-cycloalkyl-C₁-C₄-alkyl, which may in each case be mono- to trisubstituted on the cycloalkyl ring by identical or different substituents from the group consisting of halogen, C₁-C₄-alkyl, C₁-C₄-alkoxy, C₁-C₄-halogenoalkyl and C₁-C₄-halogenoalkoxy; represent 5- or 6-membered saturated or unsaturated heterocyclyl or heterocyclyl-C₁-C₄-alkyl having in each case 1 to 4 heteroatoms selected from 0 to 4 nitrogen atoms, 0 to 2 oxygen atoms and 0 to 2 sulphur atoms (in particular furyl, thienyl, tetrahydrofuryl, furfuryl, thenyl, tetrahydrofurylmethyl, thiazolyl), where the heterocycle may in each case be substituted by halogen or C₁-C₄-alkoxycarbonyl,
R¹⁴ and R¹⁵ furthermore together represent C₁-C₄-alkyleneoxy-C₁-C₄-alkylene or C₁-C₄-alkylenethio-C₁-C₄-alkylene.

3. Compounds of the formula (I) according to Claim 1, in which
Ar¹ represents the radical 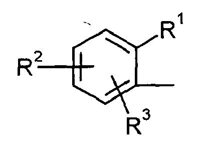 and
Ar² represents the radical 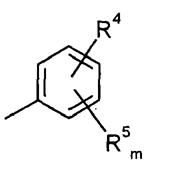 in which
m represents 0, 1 or 2,
R¹ represents fluorine, chlorine, bromine, C₁-C₆-alkyl, C₁-C₆-alkoxy, in each case fluorine- or chlorine-substituted C₁-C₆-alkyl or C₁-C₆-alkoxy,
R² and R³ independently of one another each represent hydrogen, fluorine, chlorine, bromine, iodine, C₁-C₆-alkyl, C₁-C₆-alkoxy, in each case fluorine- or chlorine-substituted C₁-C₆-alkyl or C₁-C₆-alkoxy,
R⁴ represents chlorine, bromine, iodine or one of the groupings below, located in the ortho- or para-position of the aryl ring
(l) -X-A
(m) -B-Z-D
(n) -Y-E,
R⁵ represents fluorine, chlorine, bromine, iodine, hydroxyl, cyano, -CONH₂, -CSNH₂, nitro, C₁-C₆-alkyl, C₁-C₆-alkylcarbonyl, C₁-C₆-alkoxy, in each case fluorine- or chlorine-substituted C₁-C₆-alkyl or C₁-C₆-alkoxy, -OSO₂CF₃, -CONR⁷R⁸, -OSO₂NR⁷R⁸, -S(O)ₒR⁶, -NR⁷R⁸, -NHCO₂R⁶, -B(OH)₂ or 2-(4,4,5,5-tetramethyl-1,3,2-dioxoborolane),
X represents a direct bond, oxygen, sulphur, -SO₂-, carbonyl, carbonyloxy, oxycarbonyl, oxysulphonyl (OSO₂), C₁-C₄-alkylene, C₂-C₄-alkenylene, C₂-C₄-alkinylene, C₁-C₄-alkyleneoxy, C₁-C₄-oxyalkylene, C₁-C₄-oxyalkyleneoxy, C₁-C₄-thioalkylene, cyclopropylene or oxiranylene,
A represents phenyl, naphthyl or tetrahydronaphthyl, each of which is optionally mono- to trisubstituted by radicals from the list W¹ or represents 5- to 10-membered heterocyclyl which contains 1 or 2 aromatic rings and 1 to 4 heteroatoms selected from 0 to 4 nitrogen atoms, 0 to 2 oxygen atoms and 0 to 2 sulphur atoms (in particular tetrazoyl, furyl, benzofuryl, thienyl, benzothienyl, pyrrolyl, indolyl, oxazolyl, benzoxazolyl, isoxazyl, imidazyl, pyrazyl, thiazolyl, benzothiazolyl, pyridyl, pyrimidinyl, pyridazyl, triazinyl, triazyl, quinolinyl or isoquinolinyl) and is optionally mono- to trisubstituted by radicals from the list W²,
B represents p-phenylene which is optionally mono- or disubstituted by radicals from the list W¹.
Z represents -(CH₂)ₙ-, oxygen or -S(O)ₒ-,
D represents hydrogen, C₁-C₆-alkyl, C₂-C₆-alkenyl, C₂-C₆-alkinyl, di(C₁-C₄-alkyl)aminosulphonyl, in each case fluorine- or chlorine-substituted C₁-C₆-alkyl, C₂-C₆-alkenyl or C₁-C₄-alkylsulphonyl,
Y represents a direct bond, oxygen, sulphur, -SO₂-, carbonyl, carbonyloxy, oxycarbonyl, C₁-C₆-alkylene, C₂-C₆-alkenylene, C₂-C₆-alkinylene; in each case fluorine- or chlorine-substituted C₁-C₆-alk-ylene or C₂-C₆-alkenylene; C₁-C₄-alkyleneoxy, C₁-C₄-oxyalkylene, C₁-C₄-oxyalkyleneoxy or C₁-C₄-thioalkylene,
E represents hydrogen, C₁-C₆-alkyl, C₂-C₆-alkenyl, C₂-C₆-alkinyl, di(C₁-C₆-alkyl)aminosulphonyl, in each case fluorine- or chlorine-substituted C₁-C₆-alkyl, C₂-C₆-alkenyl or C₁-C₆-alkylsulphonyl,
W¹ represents cyano, fluorine, chlorine, bromine, iodine, formyl, nitro, C₁-C₄-alkyl, C₁-C₄-alkoxy, in each case fluorine- or chlorine-substituted C₁-C₄-alkyl or C₁-C₄-alkoxy, C₂-C₆-halogenoalkenyloxy, (C₁-C₄-halogenoalkyl)sulphonyloxy, C₁-C₄-alkylcarbonyl, C₁-C₄-alkoxycarbonyl, -S(O)ₒR⁶, -SO₂NR⁷R⁸, -OSO₂NR⁷R⁸, -OSO₂R¹² or -C(R⁶)=N-O(R⁶),
W² represents cyano, fluorine, chlorine, bromine, formyl, nitro, C₁-C₄-alkyl, C₁-C₄-alkoxy, in each case fluorine- or chlorine-substituted C₁-C₄-alkyl or C₁-C₄-alkoxy, C₂-C₆-halogenoalkenyloxy, -OSO₂CF₃, C₁-C₄-alkylcarbonyl, C₁-C₄-alkoxycarbonyl, -S(O)ₒR⁶, -SO₂NR⁷R⁸, -OSO₂NR⁷R⁸ or -C(R⁶)=N-O(R⁶),
n represents 0, 1, 2 or 3,
Q represents -CO₂R⁹, -COR¹⁰, -CONR⁷R⁸, -CN, -PO(OR¹¹)₂, a 5- to 7-membered saturated or unsaturated heterocycle having 2 to 4 heteroatoms from the group consisting of nitrogen, oxygen and sulphur (in particular dihydrodioxazine, oxazoline, thiazoline, imidazoline, tetrazole),
Q furthermore represents -CO₂R¹³ or -CONR¹⁴R¹⁵,
o represents 0, 1 or 2,
R⁶ represents C₁-C₆-alkyl or in each case fluorine- or chlorine-substituted methyl or ethyl,
R⁷ and R⁸ independently of one another each represent hydrogen, C₁-C₆-alkyl, C₃-C₆-cycloalkyl, in each case fluorine- or chlorine-substituted C₁-C₆-alkyl, or together represent C₄-C₅-alkylene,
R⁹ represents hydrogen, C₁-C₆-alkyl, C₃-C₆-cycloalkyl, fluorine- or chlorine-substituted C₁-C₆-alkyl, benzyl or phenyl,
R¹⁰ represents C₁-C₆-alkyl or fluorine- or chlorine-substituted C₁-C₆-alkyl,
R¹¹ represents C₁-C₄-alkyl or phenyl,
R¹² represents C₁-C₄-alkyl or represents fluorine- or chlorine-substituted C₁-C₄-alkyl,
R¹³ represents hydrogen; represents C₁-C₄-alkyl which is mono- or disubstituted by identical or different substituents from the group consisting of cyano, nitro, methoxy, methoxycarbonyl and 2-pyrrolidinone; represents aminocarbonyl-C₁-C₄-alkyl which may be substituted on the amino group by identical or different substituents from the group consisting of C₁-C₄-alkyl, phenyl and halogenophenyl; represents C₂-C₆-alkenyl or phenyl-C₂-C₅-alkenyl; represents phenyl-C₁-C₄-alkyl or phenoxy-C₁-C₄-alkyl which may in each case be mono- to trisubstituted on the phenyl ring by identical or different substituents from the group consisting of fluorine, chlorine, methyl, methoxy and trifluoromethoxy; represents C₃-C₆-cycloalkyl or C₃-C₆-cycloalkyl-C₁-C₄-alkyl which may in each case be mono- to trisubstituted on the cycloalkyl ring by methyl; represents furyl, thienyl, pyridinyl, furfuryl, thenyl or pyridinylmethyl which may in each case be substituted on the heterocycle by chlorine; or represents tetrahydronaphthyl or indanyl,
R¹⁴ represents hydrogen,
R¹⁵ represents C₁-C₄-halogenoalkyl or C₁-C₄-alkoxy-C₁-C₄-alkyl; represents phenyl or phenyl-C₁-C₄-alkyl which may in each case be mono- to tetrasubstituted on the phenyl ring by identical or different substituents from the group consisting of fluorine, chlorine, methyl, methoxy, trifluoromethyl and trifluoromethoxy; represents C₃-C₆-cycloalkyl or C₃-C₆-cycloalkyl-C₁-C₄-alkyl which may in each case be mono- to trisubstituted on the cycloalkyl ring by identical or different substituents from the group consisting of fluorine, chlorine, methyl, trifluoromethyl and trifluoromethoxy; or represents furyl, thienyl, tetrahydrofuryl, furfuryl, thenyl, tetrahydrofurylmethyl, thiazolyl or C₁-C₄-alkoxy-carbonyl-substituted thiazolyl,
R¹⁴ and R¹⁵ furthermore together represent morpholino or thiomorpholino.

4. Compounds of the formula (I) according to Claim 1 in which
Ar¹ represents the radical 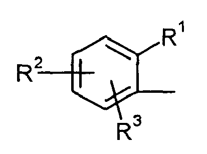 and
Ar² represents the radical 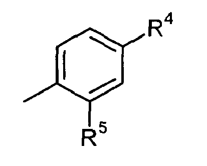 in which
R¹ represents fluorine, chlorine, bromine, methyl, ethyl, n-propyl, isopropyl, n-butyl, isobutyl, sec-butyl, tert-butyl, methoxy, ethoxy, n-propoxy, n-butoxy, isobutoxy, sec-butoxy or tert-butoxy,
R² and R³ independently of one another each represent hydrogen, fluorine, chlorine, bromine, methyl, ethyl, n-propyl, isopropyl, n-butyl, isobutyl, sec-butyl, tert-butyl, methoxy, ethoxy, n-propoxy, n-butoxy, isobutoxy, sec-butoxy or tert-butoxy,
R⁴ represents chlorine, bromine or one of the groupings below
(l) -X-A
(m) -B-Z-D
(n) -Y-E,
R⁵ represents fluorine, chlorine, bromine, hydroxyl, methyl, ethyl, methoxy, ethoxy, methylthio, ethylthio, trifluoromethyl, difluoromethoxy, trifluoromethoxy, trifluoromethylthio, -OSO₂CF₃, -OSO₂NMe₂ or -SO₂CF₃,
R⁵ also represents hydrogen,
X represents a direct bond, oxygen, sulphur, -SO₂-, carbonyl, -CH₂-, -(CH₂)₂-, -CH=CH- (E or Z), -C≡C-, -CH₂O-, -(CH₂)₂O-, -OCH₂-, -SCH₂-, -S(CH₂)₂-, -OCH₂O- or -O(CH₂)₂O-,
A represents phenyl which is optionally mono- or disubstituted by radicals from the list W¹ or represents tetrazolyl, furyl, benzofuryl, thienyl, benzothienyl, pyrrolyl, indolyl, oxazolyl, benzoxazolyl, isoxazyl, imidazyl, pyrazyl, thiazolyl, benzothiazolyl, pyridyl, pyrimidinyl, pyridazyl, triazinyl or triazyl, each of which is optionally mono- or disubstituted by radicals from the list W²,
B represents p-phenylene which is optionally monosubstituted by radicals from the list W¹,
Z represents oxygen, sulphur or -SO₂-,
D represents hydrogen, methyl, ethyl, n-propyl, isopropyl, n-butyl, isobutyl, sec-butyl, tert-butyl, 2-propenyl, butenyl, propargyl, butinyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H,-CF₂CHFCF₃, -SO₂CF₃, -SO₂(CF₂)₃CF₃, or -SO₂NMe₂,
Y represents a direct bond, oxygen, sulphur, -SO₂-, carbonyl, -CH₂-, -(CH₂)₂-, -CH=CH- (E or Z), -C≡C-, -CH₂O-, -(CH₂)₂O-, -OCH₂-, -SCH₂-, -S(CH₂)₂-, -OCH₂O- or -O(CH₂)₂O-,
E represents hydrogen, methyl, ethyl, n-propyl, isopropyl, n-butyl, isobutyl, sec-butyl, tert-butyl, 2-propenyl, butenyl, propargyl, butinyl, -CF₃, -CHF₂, -CClF₂, -CF₂CBFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, -SO₂CF₃, -SO₂(CF₂)₃CF₃ or -SO₂NMe₂,
W¹ represents cyano, fluorine, chlorine, bromine, formyl, nitro, methyl, ethyl, n-propyl, isopropyl, n-butyl, isobutyl, sec-butyl, tert-butyl, methoxy, ethoxy, n-propoxy, n-butoxy, isobutoxy, sec-butoxy, tert-butoxy, trifluoromethoxy, difluoromethoxy, -CF₃, -CHF₂, -CClF₂, -CF₂CHFCl, -CF₂CH₂F, -CF₂CCl₃, -CH₂CF₃, -CF₂CHFCF₃, -CH₂CF₂H, -OCH₂CF₃, -CH₂CF₂CF₃, -CF₂CF₂H, -CF₂CHFCF₃, -SCF₃, -SCHF₂, -SO₂CHF₂, -SO₂CF₃, -SOCHF₂,-SOCF₃, -SO₂NMe₂, -OSO₂CH₃, -OSO₂CF₃, -OSO₂(CF₂)₃CF₃, -COCH₃, -CO₂CH₃, -CO₂Et, -SO₂Me, -OSO₂NMe₂, -C(Me)=N-O(Et) or -C(Et)=N-OMe,
W² represents cyano, fluorine, chlorine, bromine, methyl, ethyl, n-propyl, isopropyl, tert-butyl, trifluoromethyl, trifluoromethoxy, difluoromethoxy, trifluoromethylthio, -OSO₂CF₃, -COCH₃, -CO₂CH₃, -OCH₂CF₃, -SO₂CF₃, -SO₂NMe₂, -OSO₂NMe₂, -C(Me)=N-O(Et) or -C(Et)=N-OMe,
Q represents -CO₂CH₃, -CO₂CH₂CH₃, -CO₂-n-propyl, -CO₂-isopropyl, -CO₂-n-butyl, -CO₂-isobutyl, -CO₂-sec-butyl, -CO₂-tert-butyl, -CO₂-n-pentyl, -CO₂-neopentyl, -CO₂-sec-isoamyl, -CO₂-pentan-3-yl, -CO₂-cyclopentyl, -CO₂-n-hexyl, -CO₂-cyclohexyl, -CO₂-trifluoroethyl, -CO₂CH₂Ph, -CO₂Ph, -COCH₃, -COCH₂CH₃, -CO-n-propyl, -CONHMe, -CONHEt, -CONH(n-propyl), -CONH(isopropyl), -CONH(n-butyl), -CONH(tert-butyl), -CONH(n-pentyl), -CONH(n-hexyl), -CONH(cyclohexyl), -CONMe₂, -CONEt₂, -CON(n-propyl)₂, -CON(isopropyl)₂, -CON(n-butyl)₂, -CON(n-pentyl)₂, -CON(Me)Et, -CON(Me)n-propyl, -CON(Me)n-butyl, -CON(Me)n-pentyl, -CN, -PO(OMe)₂, pyrrolidinocarbonyl, piperidinocarbonyl, -PO(OEt)₂, -PO(OPh)₂, dihydrodioxazinyl, oxazolyl, thiazolyl, imidazolyl or tetrazolyl,
Q furthermore represents -CO₂CH₂CN, -CO₂(CH₂)₂CN, -CO₂(CH₂)₃CN, -CO₂CH₂C(CH₃)₂NO₂, -CO₂(CH₂)₂OCH₃, -CO₂H, 2-methoxycarbonyl-propyloxycarbonyl, 3-(2-oxo-1-pyrrolidinyl)propyloxycarbonyl, N-4-fluorophenyl-N-isopropylamino-2-oxo-ethyloxycarbonyl, -CO₂(CH₂)₃CH=CH₂, -CO₂(CH₂)₂C(CH₃)=CH₂, -CO₂CH₂CH=CH-Ph, 2-chlorobenzyloxycarbonyl, 3-chlorobenzyloxycarbonyl, 4-chlorobenzyloxycarbonyl, 1-phenylethyloxycarbonyl, 3-phenylpropyloxycarbonyl, 2-phenoxyethyloxycarbonyl, 4-methylcyclohexyloxycarbonyl, cyclohexylmethyloxycarbonyl, 1-cyclohexylethyloxycarbonyl, 3-furfuryloxycarbonyl, 2-thenyloxycarbonyl, 3-thenyloxycarbonyl, 2-pyridinylmethyloxycarbonyl, 3-pyridinylmethyloxycarbonyl, 6-chloro3-pyridinylmethyloxycarbonyl, 1-tetrahydronaphthyloxycarbonyl, 1-indanyloxycarbonyl, -CON(H)CH₂CF₃, methoxyethylaminocarbonyl, 4-trifluoromethoxyphenylaminocarbonyl, benzylaminocarbonyl, 2-chlorobenzyl-aminocarbonyl, 3-chlorobenzylaminocarbonyl, 4-chlorobenzyl-aminocarbonyl, 3-methylbenzylaminocarbonyl, 4-methylbenzyl-aminocarbonyl, 2,4-dichlorobenzylaminocarbonyl, 2-methoxybenzyl-aminocarbonyl, 2,3-dimethoxybenzyl-aminocarbonyl, 3,5-dimethylbenzyl-aminocarbonyl, 2,4-difluorobenzyl-aminocarbonyl, 4-trifluoromethylcyclohexylaminocarbonyl, cyclohexylmethyl-aminocarbonyl, 2-thenylaminocarbonyl, 2-tetrahydrofurylmethyl-aminocarbonyl, 2-thiazolylaminocarbonyl, 5-methoxycarbonyl-2-thiazolyl-aminocarbonyl or morpholinocarbonyl.

5. Compounds of the formula (I) according to Claim 1, in which
Ar¹ represents the radical 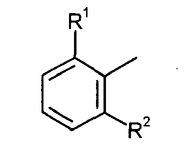
Ar² represents the radical 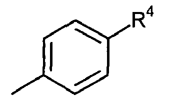
R¹ represents fluorine or chlorine,
R² represents hydrogen, chlorine or fluorine,
R⁴ represents B-Z-D,
B represents p-phenylene,
Z represents oxygen or sulphur,
D represents -CF₃,
Q represents -CO₂CH₃, -CO₂CH₂CH₃, -CO₂-n-propyl, -CO₂-isopropyl, -CO₂-n-butyl, -CO₂-isobutyl, -CO₂-sec-butyl, -CO₂-tert-butyl, -CO₂-n-pentyl, -CO₂-neopentyl, -CO₂-sec-isoamyl, -CO₂-pentan-3-yl, -CO₂-cyclopentyl, -CO₂-n-hexyl, -CO₂-cyclohexyl, -CONHMe, -CONHEt, -CONH(n-propyl), -CONH(isopropyl), -CONH(n-butyl), -CONH(tert-butyl), -CONH(n-pentyl), -CONH(n-hexyl), -CONH(cyclohexyl), -CONMe₂, -CONEt₂, -CON(n-propyl)₂, -CON(isopropyl)₂, -CON(n-butyl)₂, -CON(n-pentyl)₂, -CON(Me)Et, -CON(Me)n-propyl, -CON(Me)n-butyl, -CON(Me)n-pentyl, -CN, pyrrolidinocarbonyl or piperidinocarbonyl,
Q furthermore represents -CO₂H, -CO₂CH₂CN, -CO₂(CH₂)₂CN, -CO₂(CH₂)₃CN, -CO₂CH₂C(CH₃)₂NO₂, -CO₂(CH₂)₂OCH₃, 2-methoxycarbonyl-propyloxycarbonyl, 3-(2-oxo-1-pyrrolidinyl)propyloxycarbonyl, N-4-fluorophenyl-N-isopropyl-amino-2-oxoethyloxycarbonyl, -CO₂(CH₂)₃CH=CH₂, -CO₂(CH₂)₂C(CH₃)=CH₂, -CO₂CH₂CH=CH-Ph, 2-chlorobenzyloxycarbonyl, 3-chlorobenzyloxycarbonyl, 4-chlorobenzyloxycarbonyl, 1-phenylethyloxycarbonyl, 3-phenylpropyloxycarbonyl, 2-phenoxy-ethyloxycarbonyl, 4-methylcyclohexyloxycarbonyl, cyclohexylmethyloxycarbonyl, 1-cyclohexylethyloxycarbonyl, 3-furfuryloxycarbonyl, 2-thenyloxycarbonyl, 3-thenyloxycarbonyl, 2-pyridinylmethyloxycarbonyl, 3-pyridinylmethyloxycarbonyl, 6-chloro-3-pyridinylmethyloxycarbonyl, 1-tetrahydronaphthyloxycarbonyl, 1-indanyloxycarbonyl, -CON(H)CH₂CF₃, methoxyethylaminocarbonyl, 4-trifluoromethoxyphenyl-aminocarbonyl, benzyl-aminocarbonyl, 2-chlorobenzyl-aminocarbonyl, 3-chlorobenzyl-aminocarbonyl, 4-chlorobenzyl-aminocarbonyl, 3-methylbenzyl-aminocarbonyl, 4-methylbenzyl-aminocarbonyl, 2,4-dichlorobenzyl-aminocarbonyl, 2-methoxybenzyl-aminocarbonyl, 2,3-dimethoxybenzyl-aminocarbonyl, 3,5-dimethylbenzylaminocarbonyl, 2,4-difluorobenzyl-aminocarbonyl, 4-trifluoromethylcyclohexyl-aminocarbonyl, cyclohexylmethylaminocarbonyl, 2-thenyl-aminocarbonyl, 2-tetrahydrofurylmethylaminocarbonyl, 2-thiazolyl-aminocarbonyl, 5-methoxycarbonyl-2-thiazolyl-aminocarbonyl or represents morpholinocarbonyl.

6. Compounds of the formula (I) according to Claim 1, in which
Ar¹, Q and m are each as defined in Claims 1 to 5, and
Ar² represents the radical 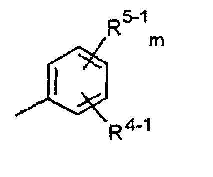 where
R⁴⁻¹ represents A or B-Z-D
where
A, B, Z and D are each as defined in Claims 1 to 5
and
R⁵⁻¹ represents fluorine, hydroxyl, cyano, nitro, alkyl, alkoxy, halogenoalkyl, halogenoalkoxy, trialkylsilyl, alkoxycarbonyl, -CONR⁷R⁸, -OSO₂NR⁷R⁸, -S(O)ₒR⁶, -NR⁷R⁸, -NHCO₂R⁶ or halogenoalkylaminosulphonyl,
where
R⁶, R⁷, R⁸ and o are each as defined in Claims 1 to 5.

7. Process for preparing compounds of the formula (I) according to any of Claims 1 to 6, **characterized in that**
A-1.) imino chlorides of the formula (II-a) 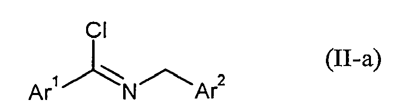 are reacted with dipolarophilic compounds of the formula (III) 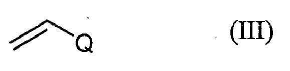 in which
Ar¹, Ar² and Q are each as defined in Claims 1 to 5
in the presence of an acid binder and, if appropriate, in the presence of a diluent, or
A-2.) imino chlorides of the formula (II-b) 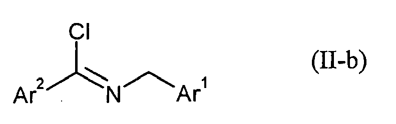 are reacted with dipolarophilic compounds of the formula (III) 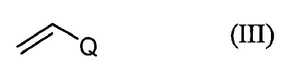 in which
Ar¹, Ar², and Q are each as defined in Claims 1 to 5
in the presence of an acid binder and, if appropriate, in the presence of a diluent, or
B.) oxazolinones of the formula (IV) 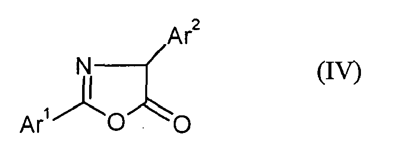 are reacted with dipolarophilic compounds of the formula (III) 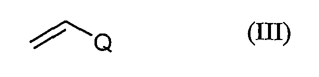 in which
Ar¹, Ar² and Q are each as defined in Claims 1 to 5,
if appropriate in the presence of a diluent, or
C.) carboxylic acids of the formula (V) 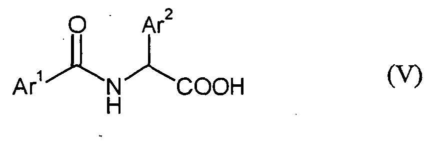 are reacted with dipolarophilic compounds of the formula (III) 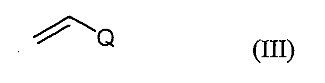 in which
Ar¹, Ar² and Q are each as defined in Claims 1 to 5
in the presence of acetic anhydride and, if appropriate, in the presence of a diluent, or
D.) Δ¹-pyrrolines of the formula (I-a) 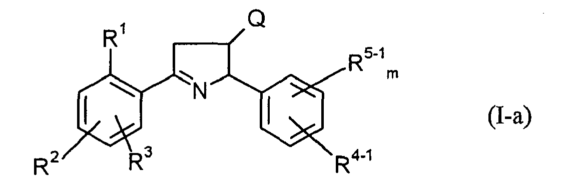 in which
R¹, R², R³, Q and m are each as defined in Claims 1 to 5,
R⁴⁻¹ represents A or B-Z-D
where
A, B, Z and D are each as defined in Claims 1 to 5
and
R⁵⁻¹ represents fluorine, hydroxyl, cyano, nitro, alkyl, alkoxy, halogenoalkyl, halogenoalkoxy, trialkylsilyl, alkoxycarbonyl, -CONR⁷R⁸, -OSO₂NR⁷R⁸, -S(O)ₒR⁶, -NR⁷R⁸, -NHCO₂R⁶, or halogenoalkylaminosulphonyl,
where
R⁶, R⁷, R⁸ and o are as defined in Claims 1 to 5
are obtained by reacting compounds of the formula (I-b) 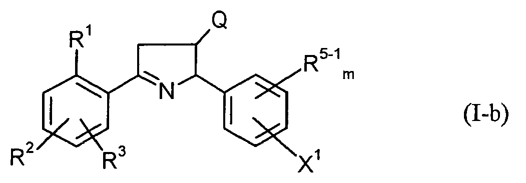 in which
R¹, R², R³, R⁵⁻¹, Q and m are each as defined in Claims 1 to 5 and
X¹ represents Cl, Br, I or -OSO₂CF₃
with boronic acids of the formula (VI) 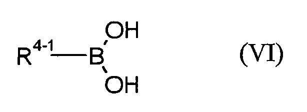 in which
R⁴⁻¹ is as defined in this claim
in the presence of a catalyst, if appropriate in the presence of an acid binder and if appropriate in the presence of a diluent,
or
compounds of the formula (I-a) are obtained by reacting compounds of the formula (I-b)
in which
X¹ represents 2-(4,4,5,5-tetramethyl-1,3,2-dioxoborolane)
with (hetero)cycles of the formula (VII)
T-A (VII)
in which A is as defined in Claims 1 to 5 and
T represents Cl, Br, I or -OSO₂CF₃,
in the presence of a catalyst, if appropriate in the presence of an acid binder and if appropriate in the presence of a diluent.

8. Pesticides, **characterized in that** they comprise at least one compound of the formula (I) according to any of Claims 1 to 6, in addition to extenders and/or surfactants.

9. Use of compounds of the formula (I) according to any of Claims 1 to 6 for controlling pests, except for the therapeutic treatment of the human or animal body.

10. Method for controlling pests, **characterized in that** compounds of the formula (I) according to any of Claims 1 to 6 are allowed to act on pests and/or their habitat, except for the therapeutic treatment of the human or animal body.

11. Use of compounds of the formula (I) according to any of Claims 1 to 6 for preparing a veterinary pharmaceutical for animal parasites, especially ectoparasites.

12. Process for preparing pesticides, **characterized in that** compounds of the formula (I) according to any of Claims 1 to 6 are mixed with extenders and/or surfactants.

13. Use of compounds of the formula (I) according to any of Claims 1 to 6 for preparing pesticides.

## Revendications

1. Δ¹-pyrrolines de formule (I) 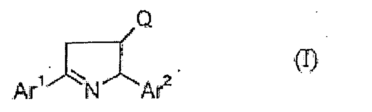 dans laquelle
Ar¹ représente le reste 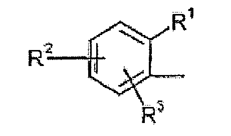 et
Ar² représente le reste 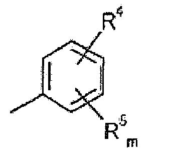 dans lesquels
m a la valeur 0, 1, 2, 3 ou 4,
R¹ représente un halogène, un reste alkyle, alkoxy, halogénalkyle, halogénalkoxy, alkoxyalkyle, -S(O)ₒR⁶, -NR⁷R⁸,
R² et R³ représentent, indépendamment l'un de l'autre, l'hydrogène, un halogène, un groupe cyano, nitro, un reste alkyle, alkoxy, halogénalkyle, halogénalkoxy, alkoxyalkyle, -S(O)ₒR⁶, -NR⁷R⁸,
R⁴ représente un halogène ou l'un des groupements suivants :
(l) -X-A
(m) -B-Z-D
(n) -Y-E
R⁵ représente un halogène, un groupe hydroxy, cyano, -CONH₂, -CSNH₂, nitro, un reste alkyle, alkylcarbonyle, alkoxy, halogénalkyle, halogénalkoxy, halogénalkylsulfonyloxy, trialkylsilyle, alkoxycarbonyle, -CONR⁷R⁸, -OSO₂NR⁷R⁸, -S(O)ₒR⁶, -NR⁷R⁸, -NHCO₂R⁶, halogénalkylaminosulfonyle, bisalkoxyborane, -B(OH)₂,
X représente une liaison directe, l'oxygène, un groupe -S(O)ₒ, -NR⁶, carbonyle, carbonyloxy, oxycarbonyle, oxysulfonyle(OSO₂), alkylène, alcénylène, alcynylène, alkylénoxy, oxyalkylène, oxyalkylénoxy, thioalkylène, cyclopropylène, oxyranylène,
A représente un reste phényle, naphtyle ou tétrahydronaphtyle chacun éventuellement substitué une ou plusieurs fois par des restes de la liste W¹, ou bien un reste hétérocyclyle saturé ou non saturé pentagonal à décagonal éventuellement substitué une ou plusieurs fois par des restes de la série W² et comportant un ou plusieurs hétéroatomes de la série azote, oxygène et soufre,
B représente un reste p-phénylène éventuellement substitué une ou deux fois par des restes de la liste W¹,
Z représente un groupe -(CH₂)ₙ-, l'oxygène ou un groupe -S(O)ₒ-,
D représente l'hydrogène, un reste alkyle, alcényle, alcynyle, halogénalkyle, halogénalcényle, halogénalkylsulfonyle, dialkylaminosulfonyle,
Y représente une liaison directe, l'oxygène, le soufre, un groupe -SO₂, carbonyle, carbonyloxy, oxycarbonyle, un reste alkylène, alcénylène, alcynylène, alkylénoxy, oxyalkylène, oxyalkylénoxy, thioalkylène, halogénalkylène, halogénalcénylène,
E représente l'hydrogène, un reste alkyle, alcényle, alcynyle, halogénalkyle, halogénalcényle, halogénalkylsulfonyle, dialkylaminosulfonyle,
W¹ représente un groupe cyano, un halogène, un groupe formyle, nitro, un reste alkyle, trialkylsilyle, alkoxy, halogénalkyle, halogénalkoxy, halogénalcényloxy, halogénalkylsulfonyloxy, alkylcarbonyle, alkoxycarbonyle, pentafluorothio, un groupe -S(O)ₒR⁶, -SO₂NR⁷R⁸, -OSO₂NR⁷R⁸, -OSO₂N(R⁶)CO₂R⁶, -OSO₂R¹², -C(R⁶)=N-O(R⁶),
W² représente un groupe cyano, un halogène, un groupe formyle, nitro, un reste alkyle, trialkylsilyle, alkoxy, halogénalkyle, halogénalkoxy, halogénalcényloxy, halogénalkylsulfonyloxy, alkylcarbonyle, alkoxycarbonyle, un groupe -S(O)ₒR⁶, -SO₂NR⁷R⁸, -OSO₂NR⁷R⁸, -C(R⁶)=N-O(R⁶),
n représente le nombre 0, 1, 2, 3 ou 4,
Q représente un groupe -CO₂R⁹, -COR¹⁰, -CONR⁷R⁸, -CN, -CONH₂, -CSNH₂, -S(O)ₒR⁶, -SO₂NR⁷R⁸, -PO(OR¹¹)₂, un hétérocycle pentagonal à heptagonal saturé ou non saturé ayant 2 à 4 hétéroatomes de la série azote, oxygène et soufre,
Q représente en outre un groupe -CO₂R¹³ ou -CONR¹⁴R¹⁵,
o a la valeur 0, 1 ou 2,
R⁶ représente l'hydrogène, un reste alkyle ou halogénalkyle,
R⁷ et R⁸ représentent, indépendamment l'un de l'autre, l'hydrogène, un reste alkyle, cycloalkyle, halogénalkyle ou forment ensemble un groupe alkylène,
R⁹ représente l'hydrogène, un reste alkyle, cycloalkyle, halogénalkyle, aralkyle, phényle,
R¹⁰ représente un reste alkyle, halogénalkyle, aralkyle,
R¹¹ représente un reste alkyle, aryle,
R¹² représente un reste alkyle, halogénalkyle, aralkyle ou aryle,
R¹³ représente l'hydrogène, un reste alkyle substitué une ou plusieurs fois ; un reste aminocarbonylalkyle éventuellement substitué ; un reste alcényle, phénylalcényle ; un reste phénylalkyle ou phénoxyalkyle chacun éventuellement substitué ; un reste cycloalkyle ou cycloalkylalkyle, chacun éventuellement substitué ; un reste hétérocyclyle ou hétérocyclylalkyle saturé ou non saturé, ayant chacun 1 à 4 hétéroatomes combinés de la série azote, oxygène et soufre ; un reste tétrahydronaphtyle ou indanyle,
R¹⁴ et R¹⁵ représentent indépendamment l'un de l'autre l'hydrogène, un reste halogénalkyle, alkoxyalkyle ; un reste phényle ou phénylalkyle, chacun éventuellement substitué ; un reste cycloalkyle ou cycloalkylalkyle, chacun éventuellement substitué ; un reste hétérocyclyle ou hétérocyclylalkyle, chacun saturé ou non saturé, chacun éventuellement substitué, ayant chacun 1 à 4 hétéroatomes de la série azote, oxygène et soufre,
R¹⁴ et R¹⁵ représentent en outre, ensemble, un reste alkylénoxyalkylène ou alkylènethioalkylène.

2. Composés de formule (I) suivant la revendication 1,
dans lesquels
Ar¹ représente le reste 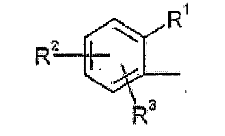
et
Ar² représente le reste 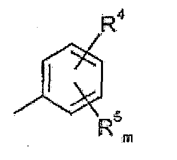 où
m a la valeur 0, 1, 2 ou 3,
R¹ représente un halogène, un reste alkyle en C₁ à C₆, alkoxy en C₁ à C₆, halogénalkyle en C₁ à C₆, halogénalkoxy en C₁ à C₆, (alkoxy en C₁ à C₆) - (alkyle en C₁ à C₆), -S(O)ₒR⁶, -NR⁷R⁸,
R² et R³ représentent, indépendamment l'un de l'autre, l'hydrogène, un halogène, un groupe cyano, nitro, un reste alkyle en C₁ à C₆, alkoxy en C₁ à C₆, halogénalkyle en C₁ à C₆, halogénalkoxy en C₁ à C₆, (alkoxy en C₁ à C₆) - (alkyle en C₁ à C₆), -S(O)ₒR⁶, -NR⁷R⁸,
R⁴ représente le fluor, le chlore, le brome, l'iode ou l'un des groupements suivants se trouvant en position ortho ou en position para du noyau aryle :
(l) -X-A
(m) -B-Z-D
(n) -Y-E
R⁵ représente un halogène, un groupe hydroxy, cyano, -CONH₂, -CSNH₂, nitro, un reste alkyle en C₁ à C₆, (alkyle en C₁ à C₆) carbonyle, alkoxy en C₁ à C₆, halogénalkyle en C₁ à C₆, halogénalkoxy en C₁ à C₆, (halogénalkyle en C₁ à C₆)sulfonyloxy, tri(alkyle en C₁ à C₆)silyle, (alkoxy en C₁ à C₆)carbonyle, -CONR⁷R⁸, -OSO₂NR⁷R⁸, -S(O)ₒR⁶, -NR⁷R⁸, -NHCO₂R⁶, (halogénalkyle en C₁ à C₆)aminosulfonyle, bis (alkoxy en C₁ à C₆) borane, -B(OH)₂,
X représente une liaison directe, l'oxygène, un groupe -S(O)ₒ, -NR⁶, carbonyle, carbonyloxy, oxycarbonyle, oxysulfonyle(OSO₂), alkylène en C₁ à C₄, alcénylène en C₂ à C₄, alcynylène en C₂ à C₄, alkylénoxy en C₁ à C₄, oxyalkylène en C₁ à C₄, oxyalkylénoxy en C₁ à C₄, thioalkylène en C₁ à C₄, cyclopropylène, oxyranylène,
A représente un reste phényle, naphtyle ou tétrahydronaphtyle, chacun éventuellement substitué une à quatre fois par des restes de la liste W¹, ou un reste hétérocyclyle pentagonal à décagonal, contenant 1 ou 2 noyaux aromatiques, éventuellement substitué une à quatre fois par des restes de la liste W² et ayant 1 à 4 hétéroatomes comprenant en combinaison 0 à 4 atomes d'azote, 0 à 2 atomes d'oxygène et 0 à 2 atomes de soufre (en particulier tétrazolyle, furyle, benzofuryle, thiényle, benzothiényle, pyrrolyle, indolyle, oxazolyle, benzoxazolyle, isoxazolyle, imidazyle, pyrazyle, thiazolyle, benzothiazolyle, pyridyle, pyrimidinyle, pyridazyle, triazinyle, triazyle, quinolinyle ou isoquinolinyle),
B représente un reste p-phénylène éventuellement substitué une ou deux fois par des restes de la liste W¹,
Z représente un groupe -(CH₂)ₙ-, l'oxygène ou un groupe -S(O)ₒ-,
D représente l'hydrogène, un reste alkyle en C₁ à C₆, alcényle en C₂ à C₆, alcynyle en C₂ à C₆, halogénalkyle en C₁ à C₆, halogénalcényle en C₂ à C₆, (halogénalkyle en C₁ à C₆)sulfonyle, di(alkyle en C₁ à C₆)aminosulfonyle,
Y représente une liaison directe, l'oxygène, le soufre, un groupe -SO₂-, carbonyle, carbonyloxy, oxycarbonyle, un reste alkylène en C₁ à C₆, alcénylène en C₂ à C₆, alcynylène en C₂ à C₆, halogénalkylène en C₁ à C₆, halogénalcénylène en C₂ à C₆, alkylénoxy en C₁ à C₄, oxyalkylène en C₁ à C₄, oxyalkylénoxy en C₁ à C₄, thioalkylène en C₁ à C₄,
E représente l'hydrogène, un reste alkyle en C₁ à C₆, alcényle en C₂ à C₆, alcynyle en C₂ à C₆, halogénalkyle en C₁ à C₆, halogénalcényle en C₂ à C₆, (halogénalkyle en C₁ à C₆)sulfonyle, di (alkyle en C₁ à C₆)aminosulfonyle,
W¹ représente un groupe cyano, un halogène, un groupe formyle, nitro, un reste alkyle en C₁ à C₆, tri(alkyle en C₁ à C₄)silyle, alkoxy en C₁ à C₆, halogénalkyle en C₁ à C₆, halogénalkoxy en C₁ à C₆, halogénalcényloxy en C₂ à C₆, (halogénalkyle en C₁ à C₆)sulfonyloxy, (alkyle en C₁ à C₆)carbonyle, (alkoxy en C₁ à C₆) carbonyle, pentafluorothio, un groupe -S(O)ₒR⁶, -SO₂NR⁷R⁸, -OSO₂NR⁷R⁸, -OSO₂N(R⁶)CO₂R⁶, -OSO₂R¹², -C(R⁶)=N-O(R⁶),
W² représente un groupe cyano, un halogène, un groupe formyle, nitro, un reste alkyle en C₁ à C₆, tri(alkyle en C₁ à C₄)silyle, alkoxy en C₁ à C₆, halogénalkyle en C₁ à C₆, halogénalkoxy en C₁ à C₆, halogénalcényloxy en C₂ à C₆, (halogénalkyle en C₁ à C₆)sulfonyloxy, (alkyle en C₁ à C₆)carbonyle, (alkoxy en C₁ à C₆)carbonyle, un groupe -S (O)ₒR⁶, -SO₂NR⁷R⁸, -OSO₂NR⁷R⁸, -C (R⁶)=N-O(R⁶),
n représente le nombre 0, 1, 2, 3 ou 4,
Q représente un groupe -CO₂R⁹, COR¹⁰, -CONR⁷R⁸, -CN, -CONH₂, -CSNH₂, -S(O)ₒR⁶, -SO₂NR⁷R⁸, -PO(OR¹¹)₂, un hétérocycle pentagonal à heptagonal saturé ou non saturé ayant 2 à 4 hétéroatomes de la série azote, oxygène et soufre,
Q représente en outre un groupe -CO₂R¹³ ou -CONR¹⁴R¹⁵,
o a la valeur 0, 1 ou 2,
R⁶ représente l'hydrogène, un reste alkyle en C₁ à C₆, halogénalkyle en C₁ à C₆,
R⁷ et R⁸ représentent, indépendamment l'un de l'autre, l'hydrogène, un reste alkyle en C₁ à C₆, cycloalkyle en C₃ à C₆, halogénalkyle en C₁ à C₆ ou forment ensemble un groupe alkylène,
R⁹ représente l'hydrogène, un reste alkyle en C₁ à C₆, cycloalkyle en C₃ à C₆, halogénalkyle en C₁ à C₆, benzyle, phényle,
R¹⁰ représente un reste alkyle en C₁ à C₆, halogénalkyle en C₁ à C₆, benzyle,
R¹¹ représente un reste alkyle en C₁ à C₆, phényle,
R¹² représente un reste alkyle en C₁ à C₆, halogénalkyle en C₁ à C₆, benzyle ou phényle,
R¹³ représente l'hydrogène, un reste alkyle en C₁ à C₆ substitué une à trois fois identiques ou différentes par un radical cyano, nitro, alkoxy en C₁ à C₄, (alkoxy en C₁ à C₄)carbonyle ou 2-pyrrolidinone ; un reste aminocarbonyl(alkyle en C₁ à C₆) qui peut être substitué sur le groupe amino identiquement ou différemment par un radical alkyle en C₁ à C₄, phényle ou halogénophényle ; un reste alcényle en C₂ à C₈, phényl(alcényle en C₂ à C₆) ; un reste phényl(alkyle en C₁ à C₄) ou un reste phénoxy(alkyle en C₁ à C₄) qui peuvent chacun être substitués dans le noyau phényle une à quatre fois identiques ou différentes par un radical halogéno, alkyle en C₁ à C₄, alkoxy en C₁ à C₄ ou halogénalkoxy en C₁ à C₄ ; un reste cycloalkyle en C₃ à C₆ ou un reste (cycloalkyle en C₃ à C₆) (alkyle en C₁ à C₄) qui peuvent chacun être substitués dans le noyau cycloalkyle une à trois fois par un radical alkyle en C₁ à C₄ ; un reste hétérocyclyle ou hétérocyclyl(alkyle en C₁ à C₄) pentagonal ou hexagonal saturé ou non saturé, ayant chacun 1 à 4 hétéroatomes comprenant en combinaison 0 à 4 atomes d'azote, 0 à 2 atomes d'oxygène et 0 à 2 atomes de soufre (en particulier furyle, thiényle, pyridinyle, furfuryle, thényle ou pyridinylméthyle), l'hétérocycle pouvant être substitué par un halogène ; un reste tétrahydronaphtyle ou indanyle,
R¹⁴ et R¹⁵ représentent indépendamment l'un de l'autre l'hydrogène, un reste halogénalkyle en C₁ à C₆, (alkoxy en C₁ à C₆) (alkyle en C₁ à C₆) ; un reste phényle ou un reste phényl(alkyle en C₁ à C₄) qui peuvent être substitués chacun dans le noyau phényle, une à quatre fois identiques ou différentes, par un radical halogéno, alkyle en C₁ à C₄, alkoxy en C₁ à C₄, halogénalkyle en C₁ à C₄ ou halogénalkoxy en C₁ à C₄ ; un reste cycloalkyle en C₃ à C₆ ou (cycloalkyle en C₃ à C₆) (alkyle en C₁ à C₄), qui peuvent chacun être substitués dans le noyau cycloalkyle une à trois fois identiques ou différentes par un radical halogéno, alkyle en C₁ à C₄, alkoxy en C₁ à C₄, halogénalkyle en C₁ à C₄ ou halogénalkoxy en C₁ à C₄ ; un reste hétérocyclyle ou hétérocyclyl(alkyle en C₁ à C₄) pentagonal ou hexagonal saturé ou non saturé, ayant chacun 1 à 4 hétéroatomes comprenant en combinaison 0 à 4 atomes d'azote, 0 à 2 atomes d'oxygène et 0 à 2 atomes de soufre (en particulier furyle, thiényle, tétrahydrofuryle, furfuryle, thényle, tétrahydrofurylméthyle, thiazolyle), l'hétérocycle pouvant dans chaque cas être substitué par un radical halogéno ou (alkoxy en C₁ à C₄) carbonyle,
R¹⁴ et R¹⁵ forment en outre, ensemble, un reste (alkylénoxy en C₁ à C₄)(alkylène en C₁ à C₄) ou (alkylènethio en C₁ à C₄) (alkylène en C₁ à C₄).

3. Composés de formule (I) suivant la revendication 1, dans lesquels
Ar¹ représente le reste 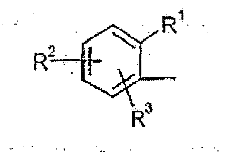
et
Ar² représente le reste 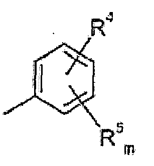 dans lesquels
m a la valeur 0, 1, ou 2,
R¹ représente le fluor, le chlore, le brome, un reste alkyle en C₁ à C₆, alkoxy en C₁ à C₆, alkyle en C₁ à C₆ ou alkoxy en C₁ à C₆, chacun substitué par du fluor ou du chlore,
R² et R³ représentent, indépendamment l'un de l'autre, l'hydrogène, le fluor, le chlore, le brome, l'iode, un reste alkyle en C₁ à C₆, alkoxy en C₁ à C₆, un reste alkyle en C₁ à C₆ ou alkoxy en C₁ à C₆, chacun substitué par du fluor ou du chlore,
R⁴ représente le chlore, le brome, l'iode ou l'un des groupements suivants se trouvant en position ortho ou en position para du noyau aryle :
(l) -X-A
(m) -B-Z-D
(n) -Y-E
R⁵ représente le fluor, le chlore, le brome, l'iode, un groupe hydroxy, cyano, -CONH₂, -CSNH₂, nitro, un reste alkyle en C₁ à C₆, (alkyle en C₁ à C₆)carbonyle, alkoxy en C₁ à C₆, un reste alkyle en C₁ à C₆ ou alkoxy en C₁ à C₆, chacun substitué par du fluor ou du chlore, un groupe -OSO₂CF₃, -CONR⁷R⁸, -OSO₂NR⁷R⁸, -S(O)ₒR⁶, -NR⁷R⁸, -NHCO₂R⁶, -B(OH)₂, 2-(4,4,5,5-tétraméthyl-1,3,2-dioxoborolane),
X représente une liaison directe, l'oxygène, le soufre un groupe -S(O)₂, carbonyle, carbonyloxy, oxycarbonyle, oxysulfonyle(OSO₂), un reste alkylène en C₁ à C₄, alcénylène en C₂ à C₄, alcynylène en C₂ à C₄, alkylénoxy en C₁ à C₄, oxyalkylène en C₁ à C₄, oxyalkylénoxy en C₁ à C₄, thioalkylène en C₁ à C₄, cyclopropylène, oxyranylène,
A représente un reste phényle, naphtyle ou tétrahydronaphtyle chacun éventuellement substitué une à trois fois par des restes de la liste W¹, ou un reste hétérocyclyle contenant 1 ou 2 noyaux aromatiques, pentagonal à décagonal, éventuellement substitué une à trois fois par des restes de la liste W² et ayant un à quatre hétéroatomes, comprenant en combinaison 0 à 4 atomes d'azote, 0 à 2 atomes d'oxygène et 0 à 2 atomes de soufre (en particulier tétrazolyle, furyle, benzofuryle, thiényle, benzothiényle, pyrrolyle, indolyle, oxazolyle, benzoxazolyle, isoxazolyle, imidazyle, pyrazyle, thiazolyle, benzothiazolyle, pyridyle, pyrimidinyle, pyridazyle, triazinyle, triazyle, quinolinyle ou isoquinolinyle),
B représente un reste p-phénylène éventuellement substitué une ou deux fois par des restes de la liste W¹,
Z représente un groupe -(CH₂)ₙ-, l'oxygène ou un groupe -S(O)ₒ-,
D représente l'hydrogène, un reste alkyle en C₁ à C₆, alcényle en C₂ à C₆, alcynyle en C₂ à C₆, di (alkyle en C₁ à C₄)aminosulfonyle, un reste alkyle en C₁ à C₆, alcényle en C₂ à C₆ ou alkylsulfonyle en C₁ à C₄, chacun substitué par du fluor ou du chlore,
Y représente une liaison directe, l'oxygène, le soufre, un groupe -SO₂-, carbonyle, carbonyloxy, oxycarbonyle, un reste alkylène en C₁ à C₆, alcénylène en C₂ à C₆, alcynylène en C₂ à C₆ ; un reste alkylène en C₁ à C₆ ou alcénylène en C₂ à C₆, chacun substitué par du fluor ou du chlore ; un reste alkylénoxy en C₁ à C₄, oxyalkylène en C₁ à C₄, oxyalkylénoxy en C₁ à C₄, thioalkylène en C₁ à C₄,
E représente l'hydrogène, un reste alkyle en C₁ à C₆, alcényle en C₂ à C₆, alcynyle en C₂ à C₆, di(alkyle en C₁ à C₆)aminosulfonyle, un reste alkyle en C₁ à C₆, alcényle en C₂ à C₆ ou alkylsulfonyle en C₁ à C₆, chacun substitué par du fluor ou du chlore,
W¹ représente un groupe cyano, le fluor, le chlore, le brome, l'iode, un groupe formyle, nitro, un reste alkyle en C₁ à C₄, alkoxy en C₁ à C₄, un reste alkyle en C₁ à C₄ ou alkoxy en C₁ à C₄ chacun substitué par du fluor ou du chlore, halogénalcényloxy en C₂ à C₆, (halogénalkyle en C₁ à C₄)sulfonyloxy, (alkyle en C₁ à C₄)carbonyle, (alkoxy en C₁ à C₄)carbonyle, -S(O)ₒR⁶, -SO₂NR⁷R⁸, -OSO₂NR⁷R⁸, -OSO₂R¹², -C(R⁶)=N-O(R⁶),
W² représente un groupe cyano, le fluor, le chlore, le brome, un groupe formyle, nitro, un reste alkyle en C₁ à C₄, alkoxy en C₁ à C₄, un reste alkyle en C₁ à C₄ ou alkoxy en C₁ à C₄, chacun substitué par du fluor ou du chlore, un reste halogénalcényloxy en C₂ à C₆, un groupe -OSO₂CF₃, un reste (alkyle en C₁ à C₄) carbonyle, (alkoxy en C₁ à C₄)carbonyle, un groupe -S(O)ₒR⁶, -SO₂NR⁷R⁸, -OSO₂NR⁷R⁸, -C(R⁶)=N-O(R⁶),
n a la valeur 0, 1, 2, ou 3,
Q représente un groupe -CO₂R⁹, -COR¹⁰, -CONR⁷R⁸, -CN, -PO(OR¹¹)₂, un hétérocycle pentagonal à heptagonal saturé ou non saturé ayant 2 à 4 hétéroatomes de la série azote, oxygène et soufre (en particulier dihydrodioxazine, oxazoline, thiazoline, imidazoline, tétrazole),
Q représente en outre un groupe -CO₂R¹³ ou -CONR¹⁴R¹⁵,
o a la valeur 0, 1 ou 2,
R⁶ représente un groupe alkyle en C₁ à C₆ ou un groupe méthyle ou éthyle, chacun substitué par du fluor ou du chlore,
R⁷ et R⁸ représentent, indépendamment l'un de l'autre, l'hydrogène, un reste alkyle en C₁ à C₆, cycloalkyle en C₃ à C₆, un reste alkyle en C₁ à C₆ substitué par du fluor ou du chlore, ou forment ensemble un reste alkylène en C₄ ou C₅,
R⁹ représente l'hydrogène, un reste alkyle en C₁ à C₆, cycloalkyle en C₃ à C₆, un reste alkyle en C₁ à C₆ substitué par du fluor ou du chlore, un reste benzyle, phényle,
R¹⁰ représente un reste alkyle en C₁ à C₆, un reste alkyle en C₁ à C₆ substitué par du fluor ou du chlore,
R¹¹ représente un reste alkyle en C₁ à C₄, phényle,
R¹² représente un reste alkyle en C₁ à C₄ ou un reste alkyle en C₁ à C₄ substitué par du fluor ou du chlore,
R¹³ représente l'hydrogène, un reste alkyle en C₁ à C₄ substitué une ou deux fois identiques ou différentes par un radical cyano, nitro, méthoxy, méthoxycarbonyle ou 2-pyrrolidinone ; un reste aminocarbonyl(alkyle en C₁ à C₄) qui peut être substitué sur le groupe amino identiquement ou différemment par un radical alkyle en C₁ à C₄, phényle ou halogénophényle ; un reste alcényle en C₂ à C₆, phényl(alcényle en C₂ à C₅) ; un reste phényl(alkyle en C₁ à C₄) ou un reste phénoxy(alkyle en C₁ à C₄), qui peuvent chacun être substitués dans le noyau phényle une à trois fois identiques ou différentes par un radical fluoro, chloro, méthyle, méthoxy ou trifluorométhoxy ; un reste cycloalkyle en C₃ à C₆ ou un reste (cycloalkyle en C₃ à C₆) (alkyle en C₁ à C₄) qui peuvent chacun être substitués dans le noyau cycloalkyle une à trois fois par un radical méthyle ; un reste furyle, thiényle, pyridinyle, furfuryle, thényle ou pyridinylméthyle dont chacun peut être substitué dans l'hétérocycle par du chlore ; un reste tétrahydronaphtyle ou indanyle,
R¹⁴ représente l'hydrogène,
R¹⁵ représente un reste halogénalkyle en C₁ à C₄, (alkoxy en C₁ à C₄) (alkyle en C₁ à C₄) ; un reste phényle ou un reste phényl(alkyle en C₁ à C₄) dont chacun peut être substitué dans la partie phényle, une à quatre fois identiques ou différentes, par un radical fluoro, chloro, méthyle, méthoxy, trifluorométhyle ou trifluorométhoxy ; un reste cycloalkyle en C₃ à C₆ ou (cycloalkyle en C₃ à C₆) (alkyle en C₁ à C₄), dont chacun peut être substitué dans le noyau cycloalkyle une à trois fois identiques ou différentes par un radical fluoro, chloro, méthyle, trifluorométhyle ou trifluorométhoxy ; un reste furyle, thiényle, tétrahydrofuryle, furfuryle, thényle, tétrahydrofurylméthyle, thiazolyle ou un reste thiazolyle substitué par un radical (alkoxy en C₁ à C₄)carbonyle,
R¹⁴ et R¹⁵ représentent en outre, conjointement, un groupe morpholino ou thiomorphilino.

4. Composés de formule (I) suivant la revendication 1, dans lesquels
Ar¹ représente le reste 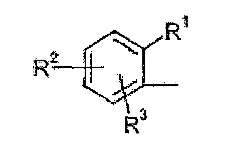
et
Ar² représente le reste 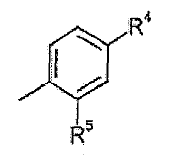
dans lesquels
R¹ représente le fluor, le chlore, le brome, un reste méthyle, éthyle, n-propyle, isopropyle, n-butyle, isobutyle, sec.-butyle, tertiobutyle, méthoxy, éthoxy, n-propoxy, n-butoxy, isobutoxy, sec.-butoxy, tertiobutoxy,
R² et R³ représentent, indépendamment l'un de l'autre, l'hydrogène, le fluor, le chlore, le brome, un reste méthyle, éthyle, n-propyle, isopropyle, n-butyle, isobutyle, sec.-butyle, tertiobutyle, méthoxy, éthoxy, n-propoxy, n-butoxy, isobutoxy, sec.-butoxy, tertiobutoxy,
R⁴ représente le chlore, le brome, ou l'un des groupements suivants :
(l) -X-A
(m) -B-Z-D
(n) -Y-E
R⁵ représente le fluor, le chlore, le brome, un groupe hydroxy, un reste méthyle, éthyle, méthoxy, éthoxy, méthylthio, éthylthio, trifluorométhyle, difluorométhoxy, trifluorométhoxy, trifluorométhylthio, -OSO₂CF₃, -OSO₂NMe₂,
ou -SO₂CF₃,
R⁵ représente en outre l'hydrogène,
X représente une liaison directe, l'oxygène, le soufre un groupe -SO₂-, carbonyle, -CH₂-, -(CH₂)₂-, -CH=CH- (E ou Z), -C≡C, -CH₂O-, -(CH₂)₂O-, -OCH₂-, -SCH₂-, -S(CH₂)₂-, -OCH₂O-, -O(CH₂)₂O-,
A représente un reste phényle éventuellement substitué une ou deux fois par des restes de la liste W¹, ou bien un reste tétrazolyle, furyle, benzofuryle, thiényle, benzothiényle, pyrrolyle, indolyle, oxazolyle, benzoxazolyle, isoxazolyle, imidazyle, pyrazyle, thiazolyle, benzothiazolyle, pyridyle, pyrimidinyle, pyridazyle, triazinyle, triazyle, chacun éventuellement substitué une ou deux fois par des restes de la liste W²,
B représente un reste p-phénylène éventuellement substitué une fois par des restes de la liste W¹,
Z représente l'oxygène, le soufre ou un groupe -SO₂-,
D représente l'hydrogène, un reste méthyle, éthyle, n-propyle, isopropyle, n-butyle, isobutyle, sec.-butyle, tertiobutyle, 2-propényle, butényle, propargyle, butynyle, un groupe -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, -SO₂CF₃, -SO₂(CF₂)₂CF₃, -SO₂NMe₂,
Y représente une liaison directe, l'oxygène, le soufre, un groupe -SO₂-, carbonyle, -CH₂-, -(CH₂)₂-, -CH=CH- (E ou Z), -C≡C-, -CH₂O-, -(CH₂)₂O-, -OCH₂-, -SCH₂-, -S(CH₂)₂-, -OCH₂O-, -O(CH₂)₂O-,
E représente l'hydrogène, un reste méthyle, éthyle, n-propyle, isopropyle, n-butyle, isobutyle, sec.-butyle, tertiobutyle, 2-propényle, butényle, propargyle, butynyle, un groupe -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, -SO₂CF₃, -SO₂(CF₂)₃CF₃, -SO₂NMe₂,
W¹ représente un groupe cyano, le fluor, le chlore, le brome, un groupe formyle, nitro, un reste méthyle, éthyle, n-propyle, isopropyle, n-butyle, isobutyle, sec.-butyle, tertiobutyle, méthoxy, éthoxy, n-propoxy, n-butoxy, isobutoxy, sec.-butoxy, tertiobutoxy, trifluorométhoxy, difluorométhoxy, un groupe -CF₃, -CHF₂, -CClF₂, -CF₂CHFCl, -CF₂CH₂F, -CF₂CCl₃, -CH₂CF₃, -CF₂CHFCF₃, -CH₂CF₂H, -OCH₂CF₃, -CH₂CF₂CF₃, -CF₂CF₂H, -CF₂CHFCF₃, -SCF₃, -SCHF₂, -SO₂CHF₂, -SO₂CF₃, -SOCHF₂, -SOCF₃, -SO₂NMe₂, -OSO₂CH₃, -OSO₂CF₃, -OSO₂(CF₂)₃CF₃, -COCH₃, -CO₂CH₃, -CO₂Et, -SO₂Me, -OSO₂NMe₂, C(Me)=N-O(Et), -C(Et)=N-OMe,
W² représente un groupe cyano, le fluor, le chlore, le brome, un reste méthyle, éthyle, n-propyle, isopropyle, tertiobutyle, trifluorométhyle, trifluorométhoxy, difluorométhoxy, trifluorométhylthio, -OSO₂CF₃, -COCH₃, -CO₂CH₃, -OCH₂CF₃, -SO₂CF₃, -SO₂NMe₂, -OSO₂NMe₂, -C(Me)=N-O(Et), -C(Et)=N-OMe,
Q représente un groupe -CO₂CH₃, -CO₂CH₂CH₃, -CO₂-n-propyle, -CO₂-isopropyle, -CO₂-n-butyle, -CO₂-isobutyle, -CO₂-sec.-butyle, -CO₂-tertiobutyle, -CO₂-n-pentyle, -CO₂-néopentyle, -CO₂-sec.-isoamyle, -CO₂-pentane-3-yle, -CO₂-cyclopentyle, -CO₂-n-hexyle, -CO₂-cyclohexyle, -CO₂-trifluoréthyle, -CO₂CH₂Ph, -CO₂Ph, -COCH₃, -COCH₂CH₃, -CO-n-propyle, -CONHMe, -CONHEt, -CONH(n-propyle), -CONH(isopropyle), -CONH(n-butyle), -CONH(tertiobutyle), -CONH(n-pentyle), -CONH(n-hexyle), -CONH (cyclohexyle), -CONMe₂, -CONEt₂, -CON(n-propyle)₂, -CON(isopropyle)₂, -CON(n-butyle)₂, -CON(n-pentyle)₂, -CON(Me)Et, -CON(Me)n-propyle, -CON(Me)n-butyle, -CON(Me)n-pentyle, -CN, -PO(OMe)₂, pyrrolidinocarbonyle, pipéridinocarbonyle, -PO(OEt)₂, -PO(OPh)₂, dihydrodioxacinyle, oxazolyle, thiazolyle, imidazolyle, tétrazolyle,
Q représente en outre un groupe -CO₂CH₂CN, -CO₂(CH₂)₂CN, -CO₂(CH₂)₃CN, -CO₂CH₂C(CH₃)₂NO₂, -CO₂(CH₂)₂OC_{H}3, -CO₂H, 2-méthoxycarbonyl-propyloxycarbonyle, 3-(2-oxo-1-pyrrolidinyl)propyloxycarbonyle, N-4-fluorophényl-N-isopropyl-amino-2-oxo-éthyloxycarbonyle, -CO₂(CH₂)₃CH=CH₂, -CO₂(CH₂)₂C(CH₃) =CH₂, -CO₂CH₂CH=CH-Ph, 2-chlorobenzyloxycarbonyle, 3-chlorobenzyloxycarbonyle, 4-chlorobenzyloxycarbonyle, 1-phényléthyloxycarbonyle, 3-phénylpropyloxycarbonyle, 2-phénoxyéthyloxycarbonyle, 4-méthyl-cyclohexyloxycarbonyle, cyclohexylméthyloxycarbonyle, 1-cyclohexyléthyloxycarbonyle, 3-furfuryloxycarbonyle, 2-thényloxycarbonyle, 3-thényloxycarbonyle, 2-pyridinylméthyloxycarbonyle, 3-pyridinylméthyloxycarbonyle, 6-chloro-3-pyridinylméthyloxycarbonyle, 1-tétrahydronaphtyloxycarbonyle, 1-indanyloxycarbonyle, -CON(H)CH₂CF₃, méthoxyéthylaminocarbonyle, 4-trifluorométhoxyphényl-aminocarbonyle, benzyl-aminocarbonyle, 2-chlorobenzyl-aminocarbonyle, 3-chlorobenzyl-aminocarbonyle, 4-chlorobenzyl-aminocarbonyle, 3-méthylbenzyl-aminocarbonyle, 4-méthylbenzyl-aminocarbonyle, 2,4-dichlorobenzyl-aminocarbonyle, 2-méthoxybenzyl-aminocarbonyle, 2,3-diméthoxybenzyl-aminocarbonyle, 3,5-diméthylbenzyl-aminocarbonyle, 2,4-difluorobenzyl-aminocarbonyle, 4-trifluorométhylcyclohexyl-aminocarbonyle, cyclohexylméthyl-aminocarbonyle, 2-thényl-aminocarbonyle, 2-tétrahydrofurylméthyl-aminocarbonyle, 2-thiazolyl-aminocarbonyle, 5-méthoxycarbonyl2-thiazolyl-aminocarbonyle ou morpholinocarbonyle.

5. Composés de formule (I) suivant la revendication 1, dans lesquels
Ar¹ représente le reste 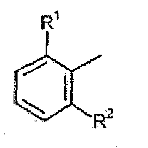
Ar² représente le reste 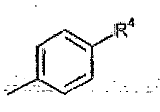
R¹ représente le fluor ou le chlore,
R² représente l'hydrogène, le chlore ou le fluor,
R⁴ représente un groupe B-Z-D,
B représente un groupe p-phénylène,
Z représente l'oxygène ou le soufre,
D représente un groupe -CF₃,
Q représente un groupe -CO₂CH₃, -CO₂CH₂CH₃, CO₂-n-propyle, -CO₂-isopropyle, -CO₂-n-butyle, -CO₂-isobutyle, -CO₂-sec.-butyle, -CO₂-tertiobutyle, -CO₂-n-pentyle, -CO₂-néopentyle, -CO₂-sec.-isoamyle, -CO₂-pentane-3-yle, -CO₂-cyclopentyle, -CO₂-n-hexyle, -CO₂-cyclohexyle, -CONHMe, -CONHEt, -CONH(n-propyle), -CONH(isopropyle), -CONH(n-butyle), -CONHtertiobutyle, -CONH(n-pentyle), -CONH(n-hexyle), -CONH(cyclohexyle), -CONMe₂, -CONEt₂, -CON(n-propyle)₂, -CON(isopropyle)₂, -CON(n-butyle)₂, -CON(n-pentyle)₂, -CON(Me)Et, -CON(Me)n-propyle, -CON(Me)n-butyle, -CON(Me)n-pentyle, -CN, pyrrolidinocarbonyle, pipéridinocarbonyle,
Q représente en outre un groupe -CO₂H, -CO₂CH₂CN, -CO₂(CH₂)₂CN, -CO₂(CH₂)₃CN, -CO₂CH₂C(CH₃)₂NO₂, -CO₂(CH₂)₂OCH₃, 2-méthoxycarbonyl-propyloxycarbonyle, 3-(2-oxo-1-pyrrolidinyl)propyloxy-carbonyle, N-4-fluorophényl-N-isopropyl-amino-2-oxo-éthyloxy-carbonyle, -CO₂(CH₂)₃CH=CH₂, -CO₂(CH₂)₂C(CH₃)=CH₂, -CO₂CH₂CH=CH-Ph, 2-chlorobenzyloxycarbonyle, 3-chlorobenzyloxycarbonyle, 4-chlorobenzyloxycarbonyle, 1-phényléthyloxycarbonyle, 3-phénylpropyloxycarbonyle, 2-phénoxyéthyloxycarbonyle, 4-méthylcyclohexyloxycarbonyle, cyclohexylméthyloxycarbonyle, 1-cyclohexyléthyloxycarbonyle, 3-furfuryloxycarbonyle, 2-thényloxycarbonyle, 3-thényloxycarbonyle, 2-pyridinylméthyloxycarbonyle, 3-pyridinylméthyloxycarbonyle, 6-chloro-3-pyridinylméthyloxycarbonyle, 1-tétrahydronaphtyloxycarbonyle, 1-indanyloxycarbonyle, -CON(H)CH₂CF₃, méthoxyéthylaminocarbonyle, 4-trifluorométhoxyphényl-aminocarbonyle, benzyl-aminocarbonyle, 2-chlorobenzyl-aminocarbonyle, 3-chlorobenzyl-aminocarbonyle, 4-chlorobenzyl-aminocarbonyle, 3-méthylbenzyl-aminocarbonyl, 4-méthylbenzyl-aminocarbonyle, 2,4-dichlorobenzyl-aminocarbonyle, 2-méthoxybenzyl-aminocarbonyle, 2,3-diméthoxybenzyl-aminocarbonyle, 3,5-diméthylbenzyl-aminocarbonyle, 2,4-difluorobenzyl-aminocarbonyle, 4-trifluorométhylcyclohexyl-aminocarbonyle, cyclohexylméthyl-aminocarbonyle, 2-thényl-aminocarbonyle, 2-tétrahydrofurylméthyl-aminocarbonyle, 2-thiazolyl-aminocarbonyle, 5-méthoxycarbonyl-2-thiazolyl-aminocarbonyle ou morpholinocarbonyle.

6. Composés de formule (I) suivant la revendication 1, dans lesquels Ar¹, Q et m ont les définitions indiquées dans les revendications 1 à 5, et
Ar² représente le reste 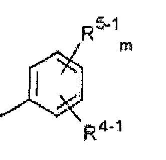 où
R⁴⁻¹ représente A ou un groupe B-Z-D,
dans lequel
A, B, Z et D ont les définitions indiquées dans les revendications 1 à 5,
et
R⁵⁻¹ représente le fluor, un groupe hydroxy, cyano, nitro, un reste alkyle, alkoxy, halogénalkyle, halogénalkoxy, trialkylsilyle, alkoxycarbonyle, -CONR⁷R⁸, -OSO₂NR⁷R⁸, -S(O)ₒR⁶, -NR⁷R⁸, -NHCO₂R⁶, halogénalkylaminosulfonyle,
R⁶, R⁷, R⁸ et o ayant les définitions indiquées dans les revendications 1 à 5.

7. Procédé de production de composés de formule (I) suivant l'une des revendications 1 à 6, **caractérisé en ce que**
A-1) on fait réagir des iminochlorures de formule (II-a) 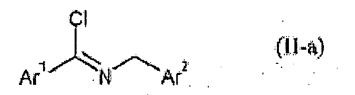 avec des dipolarophiles de formule (III) 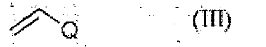 formules dans lesquelles
Ar¹, Ar² et Q ont les définitions indiquées dans les revendications 1 à 5,
en présence de l'accepteur d'acide et, le cas échéant, en présence d'un diluant, ou bien
A-2) on fait réagir des iminochlorures de formule (II-b) 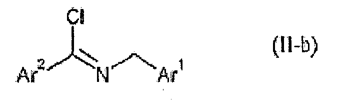 avec des dipolarophiles de formule (III) 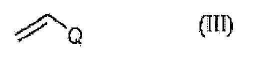 formules dans lesquelles
Ar¹, Ar² et Q ont les définitions indiquées dans les revendications 1 à 5,
en présence de l'accepteur d'acide et, le cas échéant, en présence d'un diluant, ou bien
B) on fait réagir des oxazolinones de formule (IV) 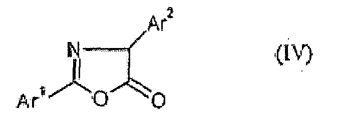 avec des dipolarophiles de formule (III) 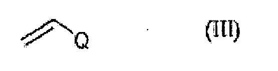 formules dans lesquelles
Ar¹, Ar² et Q ont les définitions indiquées dans les revendications 1 à 5,
le cas échéant, en présence d'un diluant, ou bien
C) on fait réagir des acides carboxyliques de formule (V) 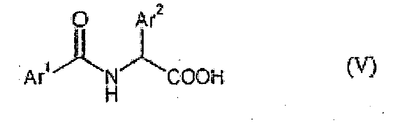 avec des dipolarophiles de formule (III) 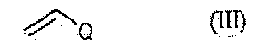 formules dans lesquelles
Ar¹, Ar² et Q ont les définitions indiquées dans les revendications 1 à 5,
en présence d'anhydride d'acide acétique et, le cas échéant, en présence d'un diluant, ou bien
D) on obtient des Δ¹-pyrrolines de formule (I-a) 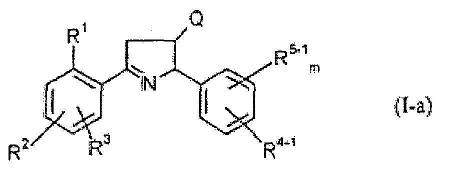 dans laquelle
R¹, R², R³, Q et m ont les définitions indiquées dans les revendications 1 à 5,
R⁴⁻¹ représente A ou B-Z-D,
A, B, Z et D ayant les définitions indiquées dans les revendications 1 à 5,
et
R⁵⁻¹ représente le fluor, un groupe hydroxy, cyano, nitro, un reste alkyle, alkoxy, halogénalkyle, halogénalkoxy, trialkylsilyle, alkoxycarbonyle, -CONR⁷R^{B}, -OSO₂NR⁷R⁸, -S(O)ₒR⁶, -NR⁷R⁸, -NHCO₂R⁶, halogénalkylaminosulfonyle,
où
R⁶, R⁷, R⁸ et o ayant les définitions indiquées dans les revendications 1 à 5,
en faisant réagir des composés de formule (I-b) 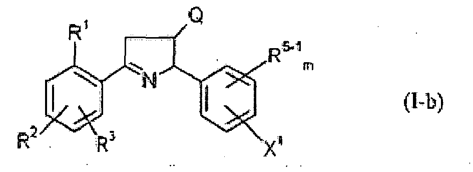 dans laquelle
R¹, R², R³, R⁵⁻¹, Q et m ont les définitions indiquées dans les revendications 1 à 5, et
X¹ représente Cl, Br, I, un groupe -OSO₂CF₃
avec des acides boroniques de formule (VI) 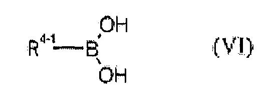 dans laquelle
R⁴⁻¹ a la définition indiquée dans cette revendication, en présence d'un catalyseur, éventuellement en présence d'un accepteur d'acide et, le cas échéant, en présence d'un diluant,
ou bien
on obtient des composés de formule (I-a) en faisant réagir des composés de formule (I-b)
dans laquelle
X¹ est un groupe 2-(4,4,5,5-tétraméthyl-1,3,2-dioxoborolane),
avec des composés (hétéro)cycliques de formule VII
T-A (VII)
dans laquelle A a la définition indiquée dans les revendications 1 à 5 et
T représente Cl, Br, I, un groupe -OSO₂CF₃,
en présence d'un catalyseur, éventuellement en présence d'un accepteur d'acide et, le cas échéant, en présence d'un diluant

8. Compositions pesticides, **caractérisées par** une teneur en au moins un composé de formule (I) suivant l'une des revendications 1 à 6, à côté de diluants et/ou d'agents tensioactifs.

9. Utilisation de composés de formule (I) suivant l'une des revendications 1 à 6, pour combattre des parasites, excepté pour le traitement thérapeutique du corps humain ou du corps animal.

10. Procédé pour combattre des parasites, **caractérisé en ce qu'**on fait agir des composés de formule (I) suivant l'une des revendications 1 à 6, sur les parasites et/ou sur leur milieu, excepté pour le traitement thérapeutique du corps humain ou du corps animal.

11. Utilisation de composés de formule (I) suivant l'une des revendications 1 à 6, pour la préparation d'un médicament à usage vétérinaire contre des parasites animaux, en particulier contre des ectoparasites.

12. Procédé de préparation de compositions pesticides, **caractérisé en ce qu'**on mélange des composés de formule (I) suivant l'une des revendications 1 à 6 avec des diluants et/ou des agents tensioactifs.

13. Utilisation de composés de formule (I) suivant l'une des revendications 1 à 6, pour la préparation de compositions pesticides.
